# EUROPEAN PATENT APPLICATION

(11) **EP 4 582 425 A1**
(43) Date of publication of application: **09.07.2025**
(21) Application number: 23856674.9
(22) Date of filing: 24.08.2023
(51) Int. Cl.: C07D 471/14, A61K 31/437, A61P 35/00, A61P 37/00

(54) **PRODRUG OF JAK KINASE INHIBITOR**

(30) Priority: 25.08.2022 WO PCT/CN2022/114802
(71) Applicant: E-nitiate Biopharmaceuticals (Hangzhou) Co., Ltd, Hangzhou, Zhejiang 311100 (CN)
(72) Inventor: LIU, Pengfei, Hangzhou, Zhejiang 311100 (CN); SHEN, Wang, Hangzhou, Zhejiang 311100 (CN); BAI, Rujun, Hangzhou, Zhejiang 311100 (CN); DING, Yue, Hangzhou, Zhejiang 311100 (CN); DING, Shizhe, Hangzhou, Zhejiang 311100 (CN)
(74) Representative: Botti & Ferrari S.p.A.
(86) International application number: PCT/CN2023/114558
(87) International publication number: WO 2024/041586

(57) **Abstract**

The present invention relates to a prodrug containing a JAK inhibitor, a composition, and use thereof. In particular, the present invention relates to a compound represented by formula (II), a pharmaceutical composition containing the compound of the present invention, and use thereof as a JAK inhibitor prodrug in treating inflammatory diseases and tumor-related diseases.

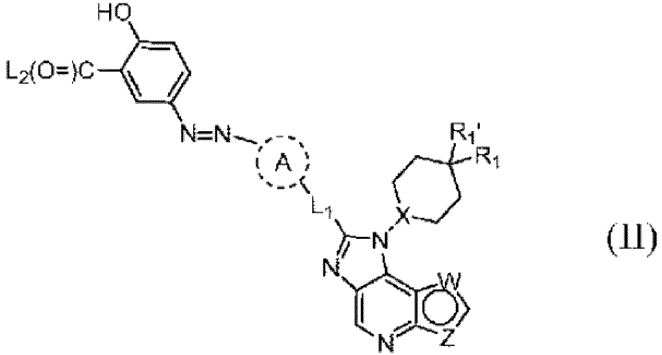

## Description

### TECHNICAL FIELD

The present application relates to the field of medicinal chemistry, in particular to a prodrug of a JAK enzyme inhibitor, a preparation method therefor and use thereof.

### BACKGROUND

Inflammatory bowel disease (IBD), such as ulcerative colitis (UC) and Crohn's disease (CD), has a significant adverse effect on the quality of life of patients. In an active stage of the disease, common symptoms of IBD include diarrhea, rectal bleeding, abdominal bleeding and pain, weight loss, fatigue, nausea, and vomiting. In 2018, there were 12.6 million cases of ulcerative colitis and 6.1 million cases of Crohn's disease worldwide. It is estimated that by 2027, the numbers will increase to 13.5 million and 6.5 million respectively.

Although there are many therapeutic schemes to induce and maintain disease remission in patients with IBD, they all have their limitations. Aminosalicylic acids are usually effective in mild diseases, but they are not effective in moderate and severe diseases. Further, long-term steroid maintenance therapy has safety problems (such as osteoporosis, muscular atrophy, neuropsychiatric disorders, etc.). Systemic immunosuppressants, such as azathioprine, mercaptopurine and methotrexate, and the like are mediocre effective in moderate and severe patients, and there is an increased risk of infection and lymphoma after long-term use. Anti-tumor necrosis factor (TNF) antibodies (such as, infliximab and adalimumab) need to be administrated by subcutaneous injection or intravenous injection, and up to a third of the patients who are first treated don't fully respond, while another third of patients who respond lose their response due to drug resistance.

At present, the clinical demand is still unsatisfied, and an effective oral therapy is needed to induce and maintain the remission of IBD and avoid the safety problems caused by chronic systemic immunosuppression. Direct action of medicaments on intestinal mucosa is the way to achieve this goal. A compound or formulation with appropriate physical and chemical properties can achieve targeted delivery to the intestine, and another option is an inactive prodrug that is cleaved by an enzyme during gastrointestinal transport to generate the active parent drug. Because the increase of many proinflammatory cytokines in ulcerative colitis (such as IL-6, IL-13, IL-15, IL-23 and IFNγ) and Crohn's disease (such as IL-13, IL-15, IL-22, IL-24 and IL-27) depends on the signal transduction of Janus kinase (JAK) tyrosine kinase family, inhibition of JAK may be beneficial to the treatment of ulcerative colitis and Crohn's disease. At present, FDA and EMA have approved tofacitinib for the treatment of moderate to severe active ulcerative colitis, and Upadacitinib was also approved for the treatment of moderate to severe active ulcerative colitis in March, 2022, and has shown a good therapeutic effect on Crohn's disease in clinical trials. However, because these drugs increase the risk of cancer, thrombosis, serious heart events and death, FDA issued a black box warning for these JAK inhibitors. If selective intestinal JAK inhibition is sufficient to achieve clinical efficacy, it may separate the beneficial anti-inflammatory activity of JAK inhibitors from potentially harmful systemic exposure.

Thus, it is urgent to develop a new JAK inhibitor in clinic to be used to treat local inflammation or local immune diseases, which can not only achieve good therapeutic effect, but also avoid the above toxicity caused by systemic exposure. Specifically, there is a strong need to develop JAK inhibitors for treating inflammatory enteritis (e.g., Crohn's disease, ulcerative colitis) with targeted oral administration and minimal systemic exposure.

### SUMMARY OF THE INVENTION

The present application provides a prodrug of JAK inhibitors that can be cleaved by enzymes produced by microorganisms in the gastrointestinal tract. The cleavage releases the JAK inhibitors in the gastrointestinal tract in a targeted or topical manner, thereby increasing the level of JAK inhibitors at the site of gastrointestinal inflammation to improve the curative effect and minimizing systemic exposure to other sites to reduce side effects.

In one aspect, the present application provides a compound of formula I, or a stereoisomer, a geometric isomer, a tautomer, a hydrate, a solvate, a metabolite, or a pharmaceutically acceptable salt thereof: 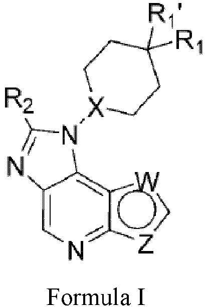 wherein,
X is selected from the group consisting of CRₐ and N;
W and Z are each independently selected from the group consisting of O, N, S, NR_{b}, and CR_{c}; W and Z cannot be CR_{c} at the same time;
R₁ is selected from the group consisting of H, halogen, CN, C₁₋₆-alkyl, C₁₋₆-alkoxy, and C₁₋₆-haloalkyl; the C₁₋₆-alkyl, C₁₋₆-alkoxy, and C₁₋₆-haloalkyl are optionally substituted by one or more R_{d};
R₁' is selected from the group consisting of H, halogen, amino, hydroxyl, C₁₋₆-alkyl, C₁₋₆-haloalkyl, C₁₋₆-alkoxy, and C₁₋₆-haloalkoxy;
R₂ is selected from the group consisting of -L₁-ONO₂, -L₁-NHC(=O)-L₂, -L₁-OC(=O)-L₂, -L₁-C(=O)O-L₂, and 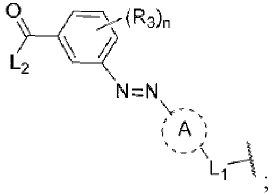 wherein,
L₁ is absent or a C₁₋₁₄- (preferably C₁₋₇-) divalent saturated or unsaturated, linear or branched hydrocarbon group; 1, 2, or 3 methylene groups in the hydrocarbon group are optionally and independently replaced by: -CH(Rₑ)-, -C(Rₑ)₂-, C₃₋₅-cycloalkylene, -N(Rₑ)-, -N(Rₑ)C(=O)-, -C(=O)N(Rₑ)-, -N(Rₑ)S(=O)₂-, -S(=O)₂N(Rₑ)-, -O-, -C(=O)-, -OC(=O)-, -C(=O)O-, -S-, -S(=O)-, or -S(=O)₂-;
ring A is absent or is selected from the group consisting of C₆₋₁₄-aryl, 5-14-membered heteroaryl, 3-14-membered heterocyclyl, and 3-14-membered carbocyclyl; the C₆₋₁₄-aryl, 5-14-membered heteroaryl, 3-14-membered heterocyclyl, and 3-14-membered carbocyclyl may be optionally substituted by one or more R_{f};
L₂ is selected from the group consisting of H, OH, C₁₋₆-alkyl, C₁₋₆-alkoxy, C₂₋₆-alkenyl, -C₀₋₆-alkylene-NH(C₁₋₆-alkyl), and -C₀₋₆-alkylene-N(C₁₋₆-alkyl)₂; the C₁₋₆-alkyl, C₁₋₆-alkoxy, C₂₋₆-alkenyl, -C₀₋₆-alkylene-NH(C₁₋₆-alkyl), and -C₀₋₆-alkylene-N(C₁₋₆-alkyl)₂ are optionally substituted with one or more substituents selected from the group consisting of hydrogen, halogen, -CN, C₁₋₆-alkyl, C₁₋₆-haloalkyl, C₂₋₆-alkenyl, C₂₋₆-alkynyl, C₁₋₆-alkoxy, C₁₋₆-haloalkoxy, -OR_{g}, -NR_{g}Rₕ, -ONO₂, -NO₂, -S(=O)₂OR_{g}, -N=CR_{g}Rₕ, -COOR_{g}, -CONHR_{g}, -C₀₋₆-alkylene-NHC(=O)R_{g}, -C₀₋₆-alkylene-C(=O)NHR_{g}, and -C(=O)NR_{g}Rₕ;
each R₃ is independently selected from the group consisting of H, OH, halogen, amino, -NR_{g}Rₕ, C₁₋₆-alkyl, C₁₋₆-haloalkyl, C₁₋₆-alkoxy, and C₁₋₆-haloalkoxy;
Rₐ, R_{b}, and R_{c} are each independently selected from the group consisting of H, C₁₋₆-alkyl, C₁₋₆-haloalkyl, C₁₋₆-alkoxy, and C₁₋₆-haloalkoxy;
each Rₐ is independently selected from the group consisting of H, halogen, CN, -NO₂, OH, NH₂, -NH(C₁₋₆-alkyl), -N(C₁₋₆-alkyl)₂, oxo, C₁₋₆-alkyl, C₁₋₆-alkoxy, C₂₋₆-alkenyl, C₂₋₆-alkynyl, -S-C₁₋₆-alkyl, -S(=O)-C₁₋₆-alkyl, -S(=O)₂-C₁₋₆-alkyl, -S(=O)NH₂, -S(=O)NHC₁₋₆-alkyl, -S(=O)N(C₁₋₆-alkyl)₂, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl; the -OH, NH₂, -NH(C₁₋₆-alkyl), -N(C₁₋₆ alkyl)₂, C₁₋₆-alkyl, C₁₋₆-alkoxy, C₂₋₆-alkenyl, C₂₋₆-alkynyl, -S-C₁₋₆-alkyl, -S(=O)-C₁₋₆-alkyl, -S(=O)₂-C₁₋₆-alkyl, -S(=O)NH₂, -S(=O)NHC₁₋₆-alkyl, -S(=O)N(C₁₋₆-alkyl)₂, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl are optionally substituted with one or more substituents selected from the group consisting of H, halogen, CN, -NO₂, OH, NH₂, -COOH, oxo, C₁₋₆-alkyl, C₁₋₆-haloalkyl, C₂₋₆-alkenyl, C₂₋₆-alkynyl, C₁₋₆-alkoxy, C₁₋₆-haloalkoxy, -NH(C₁₋₆-alkyl), -N(C₁₋₆-alkyl)₂, -COO(C₁₋₆-alkyl), -CONH(C₁₋₆-alkyl), -C₀₋₆-alkylene-NHC(=O)C₁₋₆-alkyl, -C₀₋₆-alkylene-NHC(=O)C₁₋₆-haloalkyl, -C₀₋₆-alkylene-NHC(=O)C₂₋₆-alkenyl, and -CON(C₁₋₆-alkyl)₂;
Rₑ, R_{f}, R_{g}, and Rₕ are each independently selected from the group consisting of H, C₁₋₆-alkyl, C₁₋₆-alkoxy, C₂₋₆-alkenyl, C₂₋₆-alkynyl, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl; wherein the C₁₋₆-alkyl, C₁₋₆-alkoxy, C₂₋₆-alkenyl, C₂₋₆-alkynyl, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl are optionally substituted with one or more substituents selected from the group consisting of H, halogen, CN, -NO₂, OH, NH₂, -COOH, oxo, C₁₋₆-alkyl, C₁₋₆-haloalkyl, C₂₋₆-alkenyl, C₂₋₆-alkynyl, C₁₋₆-alkoxy, C₁₋₆-haloalkoxy, -NH(C₁₋₆-alkyl), -N(C₁₋₆-alkyl)₂, -COO(C₁₋₆-alkyl), -CONH(C₁₋₆-alkyl), -C₀₋₆-alkylene-NHC(=O)C₁₋₆-alkyl, -C₀₋₆-alkylene-NHC(=O)C₁₋₆-haloalkyl, -C₀₋₆-alkylene-NHC(=O)C₂₋₆-alkenyl, and -CON(C₁₋₆-alkyl)₂; and
n is selected from the group consisting of 0, 1, 2, 3, and 4.

In some embodiments, X is selected from the group consisting of CH and N.

In some embodiments, W is selected from the group consisting of O, N, S, NH, and CH; preferably, W is CH.

In some embodiments, Z is selected from the group consisting of N, NR_{b}, and CR_{c}.

In some embodiments, Z is selected from the group consisting of N, NH, and CH; preferably, Z is NH.

In some embodiments, R_{d} is selected from the group consisting of H, OH, halogen, amino, CN, C₁₋₆-alkyl, C₁₋₆-haloalkyl, C₁₋₆-alkoxy, and -NHS(=O)₂-C₁₋₆-alkyl; wherein the C₁₋₆-alkyl, C₁₋₆-haloalkyl, C₁₋₆-alkoxy, -NHS(=O)₂-C₁₋₆-alkyl are optionally substituted with one or more substituents selected from the group consisting of halogen, CN, OH, NH₂, -NH(C₁₋₆-alkyl), and -N(C₁₋₆-alkyl)₂.

In some embodiments, R₁ is selected from the group consisting of H and C₁₋₄-alkyl; wherein the C₁₋₄-alkyl is optionally substituted with one or more substituents selected from the group consisting of CN, C₁₋₆-alkoxy, -S-C₁₋₆-alkyl, and -NHS(=O)₂C₁₋₆-alkyl-CN.

In some embodiments, R₁ is selected from the group consisting of H, -CH₂-CN, -CH₂-S-CH₃, and

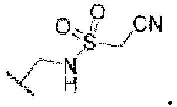

In some embodiments, R₁' is selected from the group consisting of H and C₁₋₃-alkyl.

In some embodiments, R₁' is selected from the group consisting of H and CH₃.

In some embodiments, Rₑ is selected from the group consisting of H, amino, C₁₋₄-alkyl, C₁₋₄-haloalkyl, and C₁₋₄-alkoxy.

In some embodiments, Rₑ is selected from the group consisting of H, methyl, ethyl, n-propyl, and isopropyl.

In some embodiments, L₁ is absent or a C₁₋₁₄ (preferably C₁₋₄) divalent saturated or unsaturated, liner or branched hydrocarbon group; wherein 1, 2, or 3 methylene of the hydrocarbon group are optionally and independently replaced by: -CH(Rₑ)-, -C(Rₑ)₂-, C₃₋₅-cycloalkylene, -N(Rₑ)-, -N(Rₑ)C(=O)-, -C(=O)N(Rₑ)-, -O-, -C(=O)-, -OC(=O)-, or -C(=O)O-.

In some embodiments, L₁ is absent or is selected from the group consisting of -C₁₋₆-alkylene-, -C₁₋₆-alkylene-O-C₁₋₆-alkylene-, -C₁₋₆-alkylene-OC(=O)C₁₋₆-alkylene-, -C₁₋₆-alkylene-OC(=O)OC₁₋₆-alkylene-, -C₁₋₆-alkylene-OC(=O)C₁₋₆-alkylene-O-, -C₁₋₆-alkylene-OC(=O)-NRₐC₁₋₆-alkylene-, and -C₁₋₆-alkylene-OC(=O)-NRₐ-; wherein each C₁₋₆ alkylene is optionally substituted with 1-6 Rₑ.

In some embodiments, L₁ is absent or is selected from the group consisting of -CH₂-, -CH₂CH₂-, 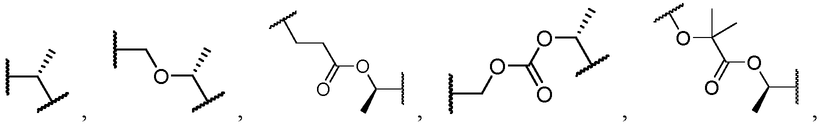 and

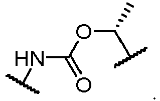

In some embodiments, R_{g} and Rₕ are each independently selected from the group consisting of H, C₁₋₆-alkyl, amino, and -COOH.

In some embodiments, L₂ is selected from the group consisting of hydrogen, OH, C₁₋₆-alkyl, C₂₋₆-alkenyl, -C₀₋₆-alkylene-NH(C₁₋₆-alkyl), and -C₀₋₆-alkylene-N(C₁₋₆-alkyl)₂; wherein the C₁₋₆-alkyl, C₂₋₆-alkenyl, -C₀₋₆-alkylene-NH(C₁₋₆-alkyl), and -C₀₋₆-alkylene-N(C₁₋₆-alkyl)₂ are optionally substituted with one or more substituents selected from the group consisting of hydrogen, halogen, CN, OH, NH₂, -ONO₂, -COOH, -S(=O)₂OH, -N=C(NH₂)₂, C₁₋₆-alkyl, and -COO(C₁₋₆-alkyl).

In some embodiments, L₂ is selected from the group consisting of OH, CH₃,

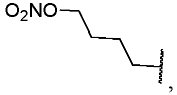

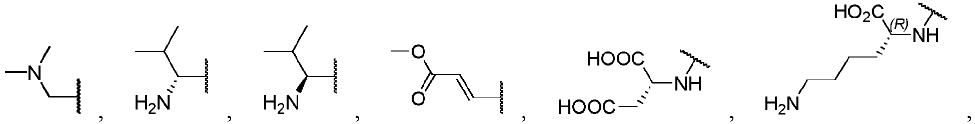

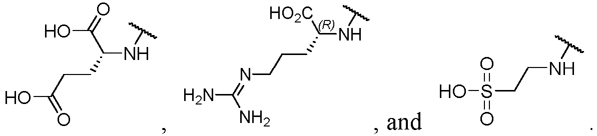

In some embodiments, L₂ is OH.

In some embodiments, L₂ is selected from the group consisting of CH₃,

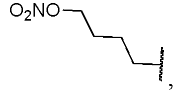

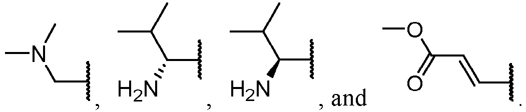

In some embodiments, L₂ is selected from the group consisting of

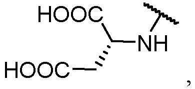

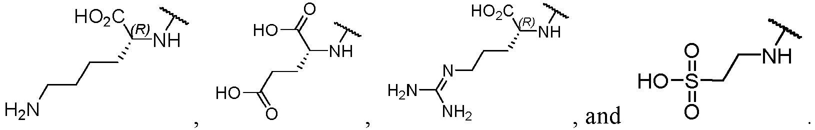

In some embodiments, R_{f} is selected from the group consisting of H and C₁₋₃-alkyl.

In some embodiments, ring A is absent or C₆₋₁₄-aryl; the C₆₋₁₄-aryl is optionally substituted by one or more R_{f}.

In some embodiments, R_{f} is selected from the group consisting of H and C₁₋₃-alkyl.

In some embodiments, ring A is absent or C₆₋₁₀-aryl.

In some embodiments, ring A is absent or phenyl.

In some embodiments, ring A is absent or is

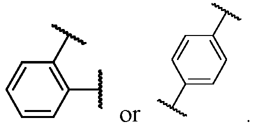

In some embodiments, R₃ is selected from the group consisting of H, hydroxyl, halogen, amino, C₁₋₃-alkyl, C₁₋₃-haloalkyl, C₁₋₃-haloalkoxy, and C₁₋₃-alkoxy.

In some embodiments, R₃ is selected from the group consisting of H and OH.

In some embodiments, n is 1 or 2.

In some embodiments, R₂ is selected from the group consisting of 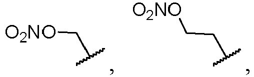 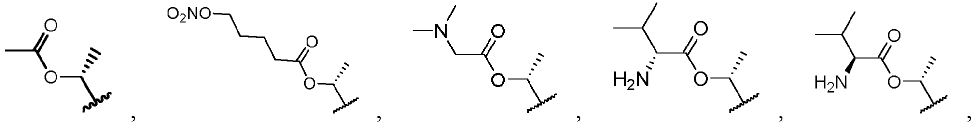 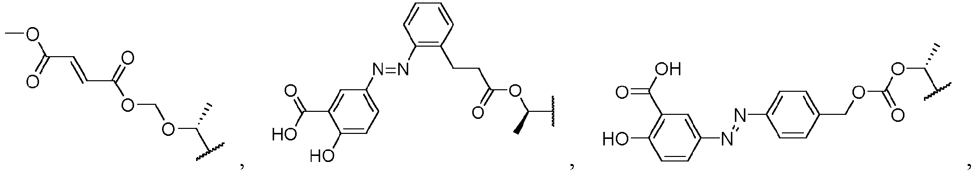 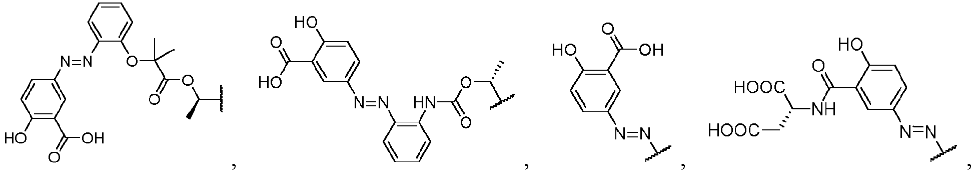 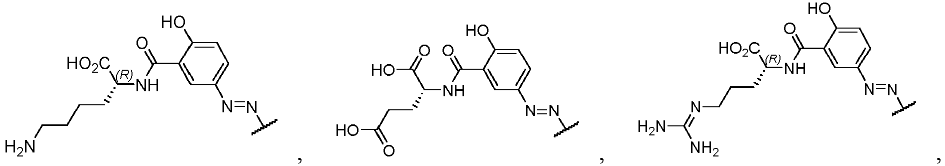 and

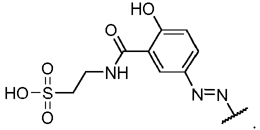

In some embodiments, the compound of formula I is a compound of formula II represented below:

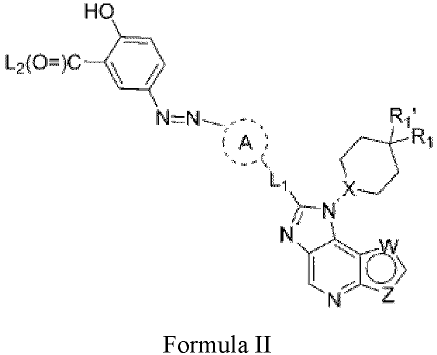

The definitions of X, W, Z, R₁, R₁', L₁, L₂, and ring A in the compound of formula II are as defined in formula I above.

In some embodiments, the compound of formula I is a compound of formula III represented below:

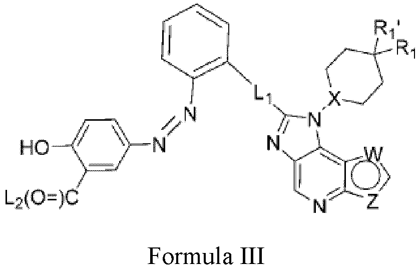

The definitions of X, W, Z, R₁, R₁', L₁, and L₂ in the compound of formula III are as defined in formula I above.

In some embodiments, the compound of formula I is a compound of formula IV represented below:

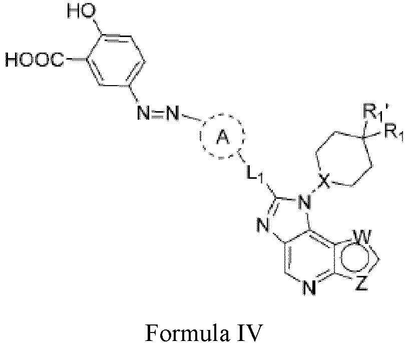

The definitions of X, W, Z, R₁, R₁', L₁, and ring A in the compound of formula IV are as defined in formula I above.

In some embodiments, the compound of formula I is a compound of formula V represented below:

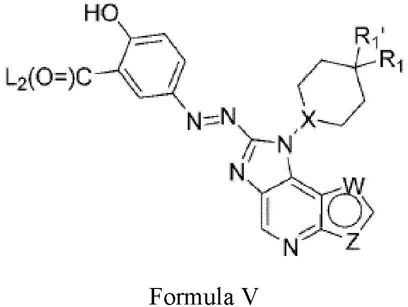

The definitions of X, W, Z, R₁, R₁', and L₂ in the compound of formula V are as defined in formula I above.

In some embodiments, the compound of formula I is a compound of formula VI represented below: 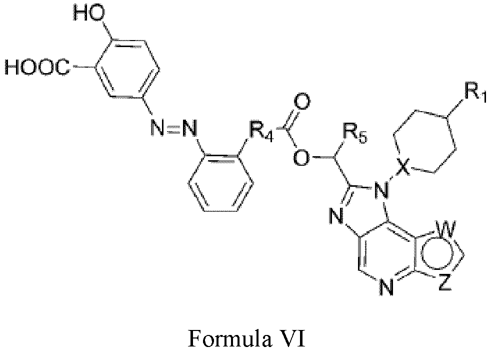
wherein, R₄ is C₁₋₃-alkylene or C₁₋₃-alkyleneoxy;
R₅ is hydrogen or C₁₋₃ alkyl;
wherein R₄ and R₅ are optionally substituted by one or more Rₑ, and Rₑ is defined in the formula I above; and
the definitions of X, W, Z, and R₁ in the compound of formula VI are as defined in formula I above.

In some embodiments, the compound of formula I is a compound of formula VII represented below:

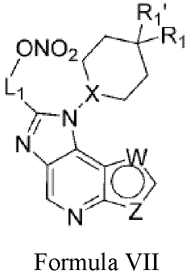

The definitions of X, W, Z, R₁, R₁', and L₁ in the compound of formula VII are as defined in formula I above.

In some embodiments, the compound of formula I is a compound of formula VIII represented below: 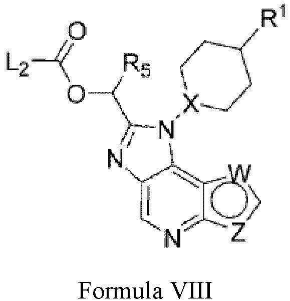
wherein R₅ is hydrogen or C₁₋₃-alkyl; the C₁₋₃-alkyl is optionally substituted by 1-6 Rₑ, and Rₑ is defined in the formula I above; and
the definitions of X, W, Z, R₁, and L₂ in the compound of formula VIII are as defined in formula I above.

In some embodiments, the compound of formula I is a compound of formula IX represented below:

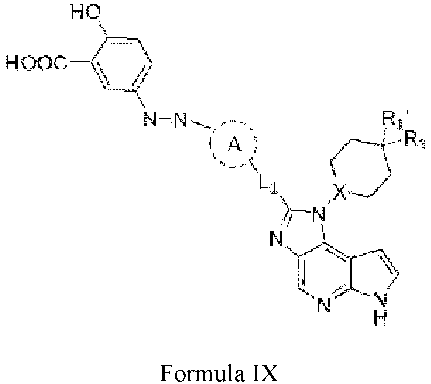

The definitions of X, R₁, R₁', L₁, and ring A in the compound of formula IX are as defined in formula I above.

In some embodiments, the compound of formula I is a compound of formula X represented below:

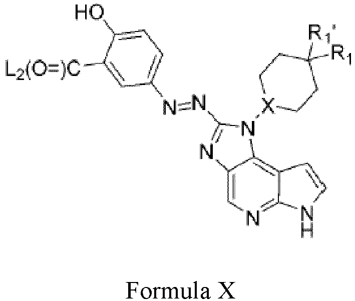

The definitions of X, R₁, R₁', and L₂ in the compound of formula X are as defined in formula I above.

In some embodiments, the compound of formula I is a compound of formula XI represented below: 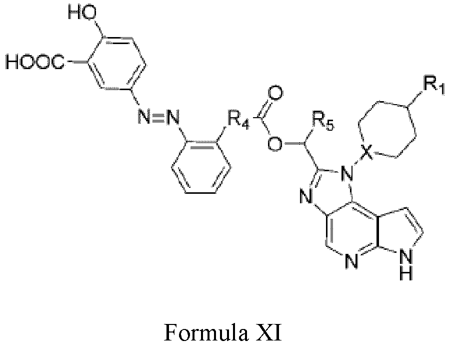
wherein R₄ is C₁₋₃-alkylene or C₁₋₃-alkyleneoxy;
R₅ is hydrogen or C₁₋₃-alkyl;
wherein R₄ and R₅ are optionally substituted by one or more Rₑ, and Rₑ is defined in the formula I above; and
the definitions of X and R₁ in the compound of formula XI are as defined in formula I above.

In some embodiments of the compound of formula I, or the stereoisomer, geometric isomer, tautomer, hydrate, solvate, metabolite or pharmaceutically acceptable salt thereof, the compound of formula I is selected from the group consisting of:
(*R*,*E*)-5-((2-(3-(1-(1-(4-(cyanomethyl)piperidin-1-yl)-1,6-dihydroimidazo[4,5-*d*]pyrrolo[2,3-*b*] pyridin-2-yl)ethoxy)-3-oxopropyl)phenyl)diazenyl)-2-hydroxybenzoic acid;
(*R*,*E*)-5-((4-((((1-(1-(4-cyanomethylpiperidin-1-yl)-1,6-dihydroimidazo[4,5-*d*]pyrrolo[2,3-*b*]py ridin-2-yl)ethoxy)carbonyl)oxy)methyl)phenyl)diazenyl)-2-hydroxybenzoic acid;
(*R*,*E*)-5-((2-((1-(1-(1-(4-cyanomethylpiperidin-1-yl)-1,6-dihydroimidazo[4,5-*d*]pyrrolo[2,3-*b*]p yridin-2-yl)ethoxy)-2-methyl-1-oxopropan-2-yl)oxy)phenyl)diazenyl)-2-hydroxybenzoic acid;
(*R*,*E*)-5-((2-((1-(1-(4-(cyanomethyl)piperidin-1-yl)-1,6-dihydroimidazo[4,5-*d*]pyrrolo[2,3-*b*]py ridin-2-yl)ethoxy)carbonyl)amino)phenyl)diazenyl)-2-hydroxybenzoic acid;
(*R*)-1-(1-(4-(cyanomethyl)piperidin-1-yl)-1,6-dihydroimidazo[4,5-*d*]pyrrolo[2,3-*b*]pyridin-2-yl )ethyl acetate;
(*R*)-1-(1-(4-(cyanomethyl)piperidin-1-yl)-1,6-dihydroimidazo[4,5-*d*]pyrrolo[2,3-*b*]pyridin-2-yl )ethyl 5-(nitrooxy)valerate;
(*R*)-1-(1-(4-(cyanomethyl)piperidin-1-yl)-1,6-dihydroimidazo[4,5-*d*]pyrrolo[2,3-*b*]pyridin-2-yl )ethyl dimethylglycinate;
(*R*)-1-(1-(4-(cyanomethyl)piperidin-1-yl)-1,6-dihydroimidazo[4,5-*d*]pyrrolo[2,3-*b*]pyridin-2-yl )ethyl D-valinate;
(*R*)-1-(1-(4-cyanomethylpiperidin-1-yl)-1,6-dihydroimidazo[4,5-*d*]pyrrolo[2,3-*b*]pyridin-2-yl)e thyl L-valinate;
(*R*)-(1-(1-(4-(cyanomethyl)piperidin-1-yl)-1,6-dihydroimidazo[4,5-*d*]pyrrolo[2,3-*b*]pyridin-2-y l)ethoxy)methyl monomethyl fumarate;
(1-(4-methyl-4-((methylthio)methyl)piperidin-1-yl)-1,6-dihydroimidazo[4,5-*d*]pyrrolo[2,3-*b*]p yridin-2-yl)methyl nitrate;
2-(1-(4-methyl-4-((methylthio)methyl)piperidin-1-yl)-1,6-dihydroimidazo[4,5-*d*]pyrrolo[2,3-*b*] pyridin-2-yl)ethyl nitrate;
5-((E)-(1-((1R,4R)-4-(cyanomethyl)cyclohexyl)-1,6-dihydroimidazo[4,5-d]pyrrolo[2,3-b]pyrid in-2-yl)diazenyl)-2-hydroxybenzoic acid;
(*E*)-5-((1-(4-cyanomethylpiperidin-1-yl)-1,6-dihydroimidazo[4,5-*d*]pyrrolo[2,3-*b*]pyridin-2-yl) diazenyl)-2-hydroxybenzoic acid;
(5-((*E*)-(1-((1*R*,4*R*)-4-(cyanomethyl)cyclohexyl)-1,6-dihydroimidazo[4,5-*d*]pyrrolo[2,3-*b*]pyri din-2-yl)diazenyl)-2-hydroxybenzoyl)-D-aspartic acid;
(5-((*E*)-(1-((1*R*,4*R*)-4-(cyanomethyl)cyclohexyl)-1,6-dihydroimidazo[4,5-*d*]pyrrolo[2,3-*b*]pyri din-2-yl)diazenyl)-2-hydroxybenzoyl)-D-lysine;
(5-((*E*)-(1-((1*R*,4*R*)-4-(cyanomethyl)cyclohexyl)-1,6-dihydroimidazo[4,5-*d*]pyrrolo[2,3-*b*]pyri din-2-yl)diazenyl)-2-hydroxybenzoyl)-D-glutamic acid;
(*R*)-2-(5-((*E*)-(1-((1*R*,4*R*)-4-(cyanomethyl)cyclohexyl)-1,6-dihydroimidazo[4,5-*d*]pyrrolo[2,3-*b*]pyridin-2-yl)diazenyl)-2-hydroxybenzamido)-5-((diaminomethylene)amino)valeric acid;
2-(5-((*E*)-(1-((1*R*,4*R*)-4-(cyanomethyl)cyclohexyl)-1,6-dihydroimidazo[4,5-*d*]pyrrolo[2,3-*b*]py ridin-2-yl)diazenyl)-2-hydroxybenzamido)ethan-1-sulfonic acid;
(*E*)-2-hydroxy-5-((1-(piperidin-1-yl)-1,6-dihydroimidazo[4,5-*d*]pyrrolo[2,3-*b*]pyridin-2-yl)dia zenyl)benzoic acid;
(*E*)-5-((1-(4-(((cyanomethyl)sulfonamido)methyl)piperidin-1-yl)-1,6-dihydroimidazo[4,5-*d*]py rrolo[2,3-*b*]pyridin-2-yl)diazenyl)-2-hydroxybenzoic acid;
5-((*E*)-(1-((1*R*,4*R*)-4-(cyanomethyl)cyclohexyl)-1*H*-imidazo[4,5-*d*]thieno[3,2*-b*]pyridin-2-yl)d iazenyl)-2-hydroxybenzoic acid;
5-((*E*)-(1-((1*R*,4*R*)-4-(cyanomethyl)cyclohexyl)-1*H*-furo[3,2-*b*]imidazo[4,5-*d*]pyridin-2-yl)dia zenyl)-2-hydroxybenzoic acid;
1-(piperidin-1-yl)-1,6-dihydroimidazo[4,5-*d*]pyrrolo[2,3-*b*]pyridin-2-amine;
*N*-((1-(2-aminoimidazo[4,5-d]pyrrolo[2,3-*b*]pyridin-1(6*H*)-yl)-piperidin-4-yl)methyl)-1-cyano methanesulfonamide;
2-((1*R*,4*R*)-4-(2-aminoimidazo[4,5-*d*]pyrrolo[2,3-*b*]pyridin-1(6*H*)-ethyl)cyclohexyl)acetonitril e;
2-(1-(2-amino-1*H*-imidazo[4,5-*d*]thieno[3,2-*b*]pyridin-1-yl)piperidin-4-yl)acetonitrile; and
2-((1*R*,4*R*)-4-(2-amino-1*H*-furo[3,2-*b*]imidazo[4,5-*d*]pyridin-1-yl)cyclohexyl)acetonitrile.

The present application also provides a pharmaceutical composition comprising a therapeutically effective amount of at least one compound of formula I, or the stereoisomer, geometric isomer, tautomer, hydrate, solvate, metabolite, or pharmaceutically acceptable salt thereof, together with one or more pharmaceutically acceptable carriers, diluents, or excipients.

Further, the present application also provides a pharmaceutical composition further comprising an additional therapeutic agent. Preferably, the additional therapeutic agent is an anti-inflammatory medicament, an immunomodulator or immunosuppressant, a neurotrophic factor, an active agent for treating cardiovascular diseases, an active agent for treating diabetes, an active agent for treating diarrhea and/or an active agent for treating autoimmune diseases.

The present application also relates to use of the compound of formula I, or the stereoisomer, geometric isomer, tautomer, hydrate, solvate, metabolite or pharmaceutically acceptable salt thereof, or the pharmaceutical composition of the present application, in the manufacture of a medicament as JAK inhibitors. Specifically, the use involves a prodrug of JAK inhibitors. The prodrug releases JAK inhibitors, preferably JAK1 and/or JAK2 inhibitors, through the action of intestinal enzymes in the use.

The present application further relates to use of the compound of formula I, or the stereoisomer, geometric isomer, tautomer, hydrate, solvate, metabolite or pharmaceutically acceptable salt thereof, or the pharmaceutical composition of the present application, in the manufacture of a medicament for the treatment of inflammatory diseases and/or cancerous diseases.

The present application further relates to a method for treating inflammatory diseases and/or cancerous diseases comprising administering a therapeutically effective amount of the compound of formula I, or the stereoisomer, geometric isomer, tautomer, hydrate, solvate, metabolite or pharmaceutically acceptable salt thereof, or the pharmaceutical composition of the present application, to a patient.

In some embodiments, the inflammatory disease and/or cancerous disease is mediated by JAK.

In some embodiments, the inflammatory disease is selected from the group consisting of rheumatoid arthritis, dermatitis, psoriasis, and inflammatory bowel disease; the cancerous disease is selected from the group consisting of myelofibrosis, polycythemia vera and essential thrombocythemia, acute myeloid leukemia (AML), acute lymphocytic leukemia (ALL), ductal carcinoma of breast, and non-small cell lung carcinoma (NSCLC). Preferably, the inflammatory bowel disease is chronic intestinal inflammatory disease, and more preferably is ulcerative colitis or Crohn's disease.

### DEFINITION AND DESCRIPTION

General chemical terms used in the above structural formulae have their usual meanings.

For example, the terms "halo" and "halogen" used in the present application refer to fluorine, chlorine, bromine or iodine. Preferably, halogen comprises fluorine, chlorine, and bromine.

In the present application, the term "hydrocarbon chain" or "hydrocarbon group" refers to a saturated or unsaturated, linear or branched carbon chain, such as alkyl, alkenyl, and alkynyl. Preferably, the hydrocarbon chain consists of 1-14 carbon atoms, more preferably 1-7 carbon atoms, and most preferably 1-4 carbon atoms, such as methyl, ethyl, n-propyl, isopropyl, butyl, allyl and the like. Divalent "hydrocarbon chain" or "hydrocarbon group" includes alkylene; non-limiting examples includes, but are not limited to, methylene (-CH₂-), 1,1-ethylidene (-CH(CH₃)-), 1,2-ethylene (-CH₂CH₂-), 1,1-propylidene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), 1,7-heptylene (-CH₂CH₂CH₂CH₂CH₂CH₂CH₂-), and the like.

In the present application, the term "alkyl" refers to a liner or branched monovalent saturated hydrocarbon group, preferably "C₁₋₈-alkyl", and more preferably "C₁₋₆-alkyl". The alkyl used herein may optionally be substituted with one or more substituents. Non-limiting examples of alkyl include methyl, ethyl, propyl, isopropyl, n-butyl, isobutyl, sec-butyl, tert-butyl, n-pentyl, 3-(2-methyl)butyl, 2-pentyl, 2-methylbutyl, neopentyl, n-hexyl, 2-hexyl, 2-methylpentyl and the like. Similarly, "C₁₋₆" in "C₁₋₆-alkyl" refers to a group with 1, 2, 3, 4, 5, or 6 carbon atoms arranged in a linear, branched or cyclic form.

The terms "alkenyl" and "alkynyl" refer to liner or branched alkenyl and alkynyl groups, preferably "C₂₋₈-alkenyl" or "C₂₋₈-alkynyl", more preferably "C₂₋₆-alkenyl" or "C₂₋₆-alkynyl". Similarly, "C₂₋₆" in "C₂₋₆-alkenyl" and "C₂₋₆" in "C₂₋₆-alkynyl" refers to an alkenyl or an alkynyl with 2, 3, 4, 5, or 6 carbon atoms arranged in a linear, branched or cyclic form.

The term "alkoxy" refers to an oxy-ether form of the aforementioned linear or branched alkyl.

The term "haloalkyl" means that the aforementioned "alkyl" is substituted by one or more halogens.

In the present application, "a", "an", "the", "at least one" and "one or more" can be used interchangeably. Thus, for example, a composition containing "a" pharmaceutically acceptable excipient may be interpreted as a composition containing "one or more" pharmaceutically acceptable excipient(s).

In the present application, the presence of **○** in a ring of a general formula indicates that the ring is aromatic.

In the present application, unless otherwise specified, the term "aryl" refers to an unsubstituted or substituted monocyclic, fused, or bridged aromatic group comprising carbon atoms. Preferably, the aryl is a C₆₋₁₄-aryl, more preferably a C₆₋₁₀-aryl, and even more preferably a phenyl group.

The term "heteroaryl" refers to a monocyclic or polycyclic (e.g., fused bicyclic) aromatic heterocyclyl having at least one heteroatom ring member(s) (e.g., 1-4 heteroatoms, or preferably 1-3 heteroatoms) selected from the group consisting of N, O, and S. For the heteroatoms, the nitrogen or sulfur heteroatom may optionally be oxidized, and the nitrogen heteroatom may optionally be quaternized. The heteroaryl can be attached to other moieties through any heteroatom or carbon atom, resulting in a stable structure. Preferably, the heteroaryl is a 5-10-membered heteroaryl, and more preferably is a 5-6-membered heteroaryl. Examples of heteroaryl include, but are not limited to, thienyl, furyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuryl, benzothienyl, benzoisoxazolyl, benzoxazolyl, benzopyrazolyl, benzothiazolyl, benzothiadiazolyl, benzotriazolyl, adeninyl, quinolinyl, and isoquinolinyl.

The term "carbocyclyl" refers to a saturated or unsaturated cyclic group without aromaticity. According to the special level of saturation, the terms "cycloalkyl", "cycloalkenyl" or "cycloalkynyl" are used respectively. Monocyclic carbocyclyl includes, but is not limited to, cyclopropyl, cyclobutyl, cyclopentyl, cyclohexyl or cyclohexenyl and the like. Polycyclic carbocyclyl includes spirocyclic groups, fused cyclic groups and bridged cyclic groups. The carbocyclyl is preferably a C₃₋₁₄-carbocyclyl, more preferably a C₃₋₈-carbocyclyl, even more preferably a C₃₋₆-carbocyclyl, and most preferably a C₅₋₆-carbocyclyl.

The term "cycloalkyl" refers to a saturated monocyclic or polycyclic system consisting solely of carbon atoms in the ring, which may optionally be substituted with one or more substituents. Polycyclic "cycloalkyl" groups encompass bridged, fused, and spirocyclic ring systems. Non-limiting examples of cycloalkyl include, e.g., cyclopropyl, cyclobutyl, cyclopentyl, cyclohexyl, cyclohexenyl, spiro[3.4]octyl, bicyclo[2.2.1]heptyl, and the like. The cycloalkyl is preferably a C₃₋₁₄-cycloalkyl, more preferably a C₃₋₈-cycloalkyl, even more preferably a C₃₋₆-cycloalkyl, and most preferably a C₅₋₆-cycloalkyl.

In the present application, unless otherwise specified, the term "heterocyclyl" refers to an unsubstituted or substituted monocyclic or polycyclic system composed of carbon atoms and 1-3 heteroatoms selected from N, O, and S, and includes both saturated and unsaturated cyclic systems, as well as polycyclic systems with unsaturated and/or aromatic moieties. In the heterocyclyl, nitrogen or sulfur heteroatom can be selectively oxidized, and nitrogen heteroatom can be selectively quaternized. The heterocyclyl can be attached to other moieties through any heteroatom or carbon atom, resulting in a stable structure. It should be understood that polycyclic heterocyclyl encompasses fused, bridged and spirocyclic ring systems. The heterocyclyl is preferably a 3-14-membered heterocyclyl, more preferably a 3-8-membered heterocyclyl or a 5-10-membered heterocyclyl. The 3-8-membered heterocyclyl is further preferably a 3-6-membered heterocyclyl, and the 3-6-membered heterocyclyl is even further preferably a 5-6 membered heterocyclyl. Heterocyclyl used herein can optionally be substituted with one or more substituents. Examples of heterocyclyl include, but are not limited to, azetidinyl, pyrrolidinyl, piperidinyl, piperazinyl, oxopiperazinyl, oxopiperidinyl, tetrahydrofuryl, dioxolanyl, tetrahydroimidazolyl, tetrahydothiazolyl, tetrahydrooxazolyl, tetrahydropyranyl, morpholinyl, thiomorpholinyl, thiomorpholinosulfinyl, thiomorpholinosulfonyl, and tetrahydrooxadiazolyl.

However, under no circumstances will heterocyclyl and carbocyclyl overlap or contain each other. Therefore, based on the above definitions, if at least one all-carbon ring is fused with a heterocyclyl to form a bi-, multi-, or spiro-cyclic ring, it will still be defined as a heterocyclyl.

Additionally, if a heteroaryl is fused with a heterocyclyl to form a bi-, multi-, or spiro-cyclic ring, it will be defined as a heterocyclyl rather than a heteroaryl.

The term "substituted" means that one or more hydrogen atoms in a group are respectively replaced by the same or different substituents. Typical substituents include, but are not limited to, halogen (F, Cl, Br, or I), C₁₋₈-alkyl, C₃₋₁₂-cycloalkyl, -OR¹, -SR¹, =O, =S, -C(O)R¹, -C(S)R¹, =NR¹, -C(O)OR¹, -C(S)OR¹, -NR¹R², -C(O)NR¹R², CN, nitro, -S(O)₂R¹, -O-S(O)₂OR¹, -O-S(O)₂R¹, -OP(O)(OR¹)(OR²); where R¹ and R² are independently selected from -H, C₁₋₆-alkyl, and C₁₋₆-haloalkyl. In some embodiments, the substituents are independently selected from the group consisting of -F, -Cl, -Br, -I, -OH, trifluoromethoxy, ethoxy, propoxy, isopropoxy, n-butoxy, isobutoxy, tert-butoxy, -SCH₃, -SC₂H₅, formyl, -C(OCH₃), CN, nitro, -CF₃, -OCF₃, amino, dimethylamino, methylthio, sulfonyl, and acetyl.

The term "pharmaceutically acceptable salt" refers to a salt prepared from a pharmaceutically acceptable non-toxic base or acid. When the compound provided by the present application is an acid, the corresponding salt can be conveniently prepared from pharmaceutically acceptable non-toxic bases, including inorganic bases and organic bases.

Since the compound of formula I will be used as a medicament, the compound preferably has a certain purity, for example, a purity of at least 60%, more suitably a purity of at least 75%, and particularly suitably a purity of at least 98% (% refers to a mass percentage).

Obviously, a definition of a substituent or variable at a specific position in a molecule is independent of another position in the molecule. It is easy to understand that those skilled in the art can select the substituents or substituted forms of the compounds in the present application by means known in the prior art and the method described in the present application, so as to obtain the compounds which are chemically stable and easy to synthesize.

The compounds of the present application may contain one or more asymmetric center(s), which may result in diastereomers and optical isomers. The present application includes all possible diastereomers and their racemic mixtures, their substantially pure resolution enantiomers, all possible geometric isomers and their pharmaceutically acceptable salts.

The above-mentioned compound of formula I does not exactly define the three-dimensional structure of some positions of the compound. The present application includes all stereoisomers of the compound of formula I and pharmaceutically acceptable salts thereof. Further, the mixture of stereoisomers and the separated specific stereoisomers are also included in the present application. In the synthetic process of such compounds, or in the process of racemization or epimerization known to those skilled in the art, the product obtained can be a mixture of stereoisomers.

When the compound of formula I has a tautomer, unless otherwise stated, the present application includes any possible tautomer and pharmaceutically acceptable salts thereof, as well as mixtures thereof.

When the compound of formula I or the pharmaceutically acceptable salt thereof has a solvate or a polymorph, the present application includes any possible solvates and polymorphs. The type of solvent for forming the solvate is not particularly limited, as long as the solvent is pharmaceutically acceptable. The term "composition" in the present application refers to a product including each specified ingredient in a specified amount, and any product(s) directly or indirectly produced by the combination of each specified ingredient in a specified amount. Thus, a pharmaceutical composition containing the compound of the present application as an active ingredient and a method for preparing the compound of the present application are also part of the present application. In addition, several crystalline forms of the compound may exist in polymorph, and such polymorph is included in the present application. Moreover, some compounds can form a solvate with water (i.e. hydrate) or a common organic solvent, and such solvate also fall within the scope of the present application.

The pharmaceutical composition provided by the present application comprises a compound of formula I (or a pharmaceutically acceptable salt thereof) as an active component, a pharmaceutically acceptable excipient and other optional therapeutic components or excipients. Although in any given case, the most suitable mode of administration of an active ingredient depends on the specific subject, the nature of the subject and the severity of disease, the pharmaceutical composition of the present application includes pharmaceutical compositions suitable for oral, rectal, topical, and parenteral (including subcutaneous, intramuscular, and intravenous) administration. The pharmaceutical composition of the present application can be in a unit dosage form known in the art and conveniently prepared by any preparation method known in the pharmaceutical field.

The pharmaceutical composition of the present application can be prepared by any pharmaceutical method. Generally, such method includes a step of complexing an active component with a carrier constituting one or more essential components. Generally, the pharmaceutical composition is prepared by uniformly and closely mixing the active component with a liquid carrier or a finely divided solid carrier or a mixture of both. In addition, the product can be easily prepared into the required appearance.

Thus, the pharmaceutical composition of the present application includes a pharmaceutically acceptable carrier and the compound of formula I or the stereoisomer, geometric isomer, tautomer, hydrate, solvate, metabolite or pharmaceutically acceptable salt thereof. The combination of the compound of formula I or a pharmaceutically acceptable salt thereof with one or more other compounds with therapeutic activity is also included in the pharmaceutical composition of the present application.

The carrier of the medicament used in the present application can be, for example, a solid carrier, a liquid carrier or a gas carrier.

### BRIEF DESCRIPTION OF THE DRAWING

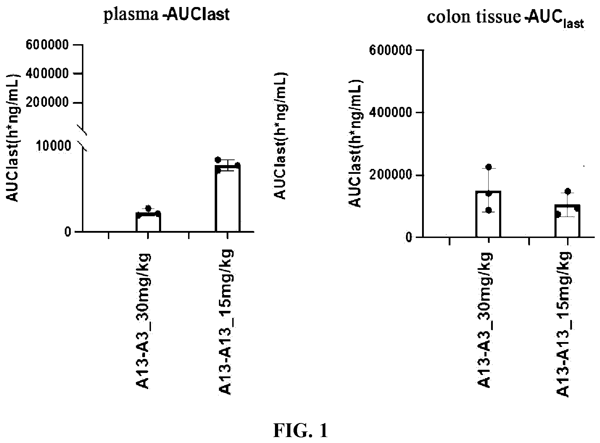

FIG.1: Comparison figure of AUC for the active ingredient A13 in plasma and colon tissue in rats after administration of compounds A3 and A13, respectively.

### Cleavage of Prodrug

When in contact with azoreductase in gastrointestinal tract, the azo bond in the compound of the present application will be cleaved by azoreductase and release a JAK inhibitor. Embodiments of cleavage are as follows:

### Embodiment 1

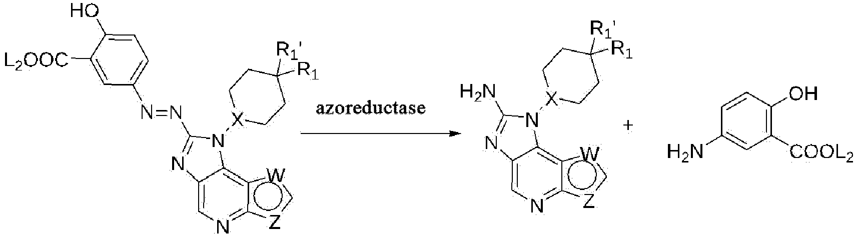

### Embodiment 2

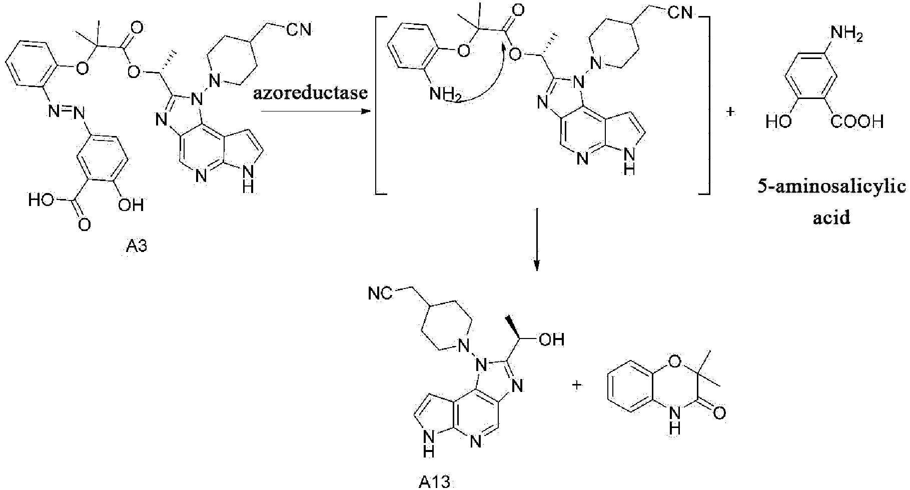

Similar to the cleavage of compound A3, compounds A1 and A2 (see Examples) can release compound A13 by utilizing enteral enzymes (e.g., azoreductase).

The compounds of the present application can be prepared according to many preparation routes that are disclosed in literatures. An example of the synthesis method for preparing the compounds of the present application is provided in the following schemes, and the following is an exemplary general synthesis scheme:

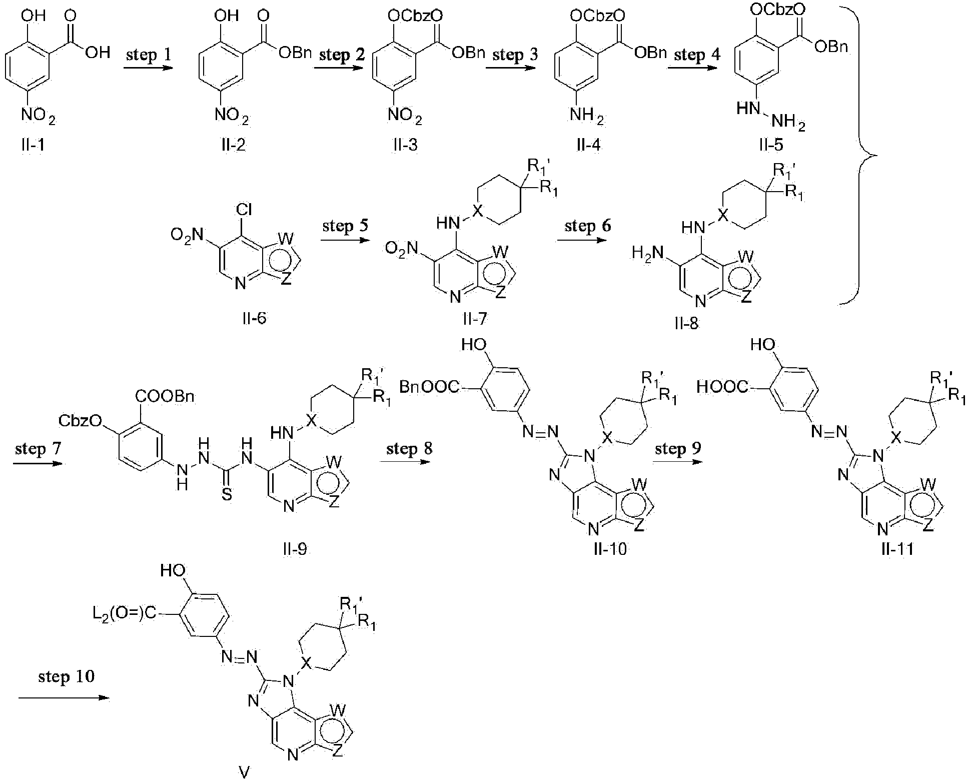

In a specific embodiment, the compound is manufactured by the process steps as follows:
S1. protecting the carboxyl group of starting compound II-1 with a benzyl group to obtain compound II-2;
S2. protecting the phenolic hydroxyl group in compound II-2 with Cbz to obtain compound II-3;
S3. reducing the nitro group in compound II-3 to an amine group to obtain compound II-4;
S4. nitrosating and then reducing the amine group in compound II-4 to obtain compound II-5;
S5. subjecting another starting compound II-6 to an addition reaction (e.g., with hydrazine) to obtain compound II-7;
S6. reducing the nitro group in compound II-7 to an amine group to obtain compound II-8;
S7. combining compounds II-5 and II-8 via a thiocarbonyl group to form compound II-9;
S8. subjecting compound II-9 to a ring closing reaction, and removing the protecting group of the phenolic hydroxyl group to obtain compound II-10;
S9. removing the protecting group of the carboxyl group in compound II-10 to obtain compound II-11, which can be used as a final product or as an intermediate for further reaction; and
S10. optionally, subjecting compound II-11 to a condensation reaction with a compound bearing amine or hydroxyl to obtain compound V.

Specific processes of the above reactions are as follows. Compound II-1 can be purchased directly or obtained through known common synthesis methods. The carboxyl group and phenolic hydroxyl group in compound II-1 can be protected with protecting groups to obtain compound II-3. The nitro group can be reduced by reducing agents such as iron powder, zinc powder, catalytic hydrogenation, stannous chloride and the like, to convert intermediate product II-3 to compound II-4. Compound II-4 can be nitrosated using HNO₂ (obtained by adding acid to NaNO₂) and then reduced under the action of stannous dichloride to obtain compound II-5. Compound II-6 can be purchased directly or obtained through known common synthesis methods. Compound II-6 can be subjected to an addition reaction with hydrazine to obtain compound II-7, on which the nitro group can be reduced by actions such as iron powder, zinc powder, catalytic hydrogenation, stannous chloride and the like to obtain compound II-8. Compounds II-5 and II-8 can be combined through the action of thiocarbonyl diimidazole or thiophosgene to obtain compound II-9 that is bonded by a thiocarbonyl. Compound II-9 can be subjected to a ring closed reaction under the action of a condensing agent (e.g., EDCI, DCC, etc.) while also removing the protecting group of phenolic hydroxyl group to obtain compound II-10. Then, the protecting group of carboxyl group in compound II-10 can be removed to obtain compound II-11. The carboxyl group in compound II-11 can be condensed with an amine or hydroxyl compound to obtain the compound of Formula V.

### DETAILED DESCRIPTION OF THE INVENTION

In order to make the above content clearer and more definite, the technical solutions of the present application will be further illustrated by the following examples. The following examples are provided solely to illustrate the specific embodiments of the present application, so that those skilled in the art may understand the present application, and are not intended to limit the scope of protection of the present application. Unless otherwise specified, the technical means or methods and the like in the specific embodiments of the present application are conventional technical means or methods and the like in the art.

Unless otherwise stated, all parts and percentages in the present application are calculated by weight, and all temperatures are in degree Celsius.

The raw materials and reagents used in the examples are commercially available or can be prepared through conventional methods in the art.

### Example 1: Synthesis of Compound A1

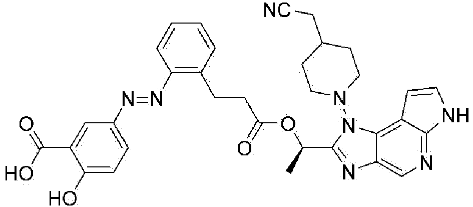

### (R,E)-5-((2-(3-(1-(1-(4-(cyanomethyl)piperidin-1-yl)-1,6-dihydroimidazo[4,5-d]pyrrolo[2,3-b] pyridin-2-yl)ethoxy)-3-oxopropyl)phenyl)diazenyl)-2-hydroxybenzoic acid

The specific synthesis steps are as follows:

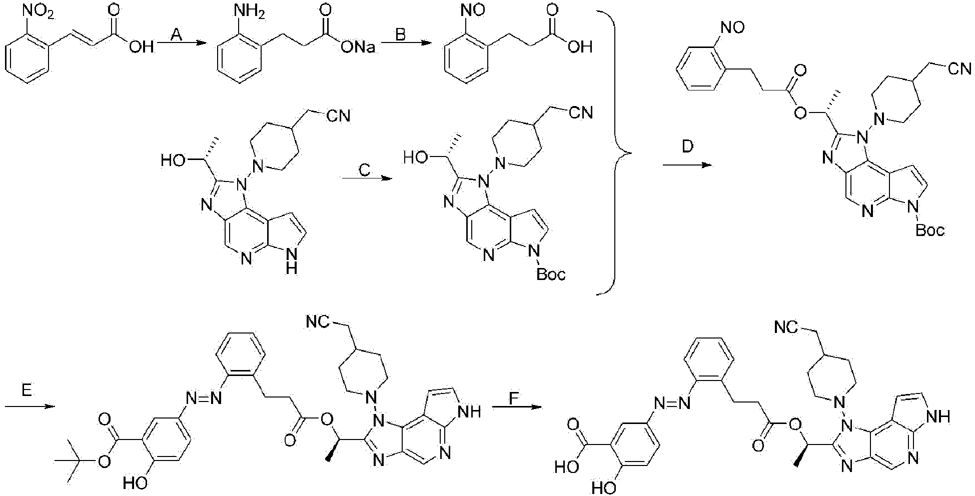

### Step A:

2-Nitrocinnamic acid (5.0 g) was dissolved in 100 mL water and 103 mL 2% NaOH, then 10% Pd/C (500 mg) was added. The reaction solution was stirred under hydrogen atmosphere at room temperature for 64 hours. After the reaction was completed, the resultant was filtered and rotary dried to obtain crude compound sodium o-aminophenylpropionate (6.8 g).

### Step B:

Compound sodium o-aminophenylpropionate (2.0 g) was dissolved in 20 mL water and 60 mL dichloromethane, and potassium monopersulfate (9.862 g) was added slowly at 0°C and stirred at room temperature for 2 hours. The reaction solution was extracted with dichloromethane (2×20 mL), and the organic phases were combined and dried over anhydrous sodium sulfate to obtain crude compound o-nitrosophenylpropionic acid (1.8 g).

### Step C:

(*R*)-2-(1-(2-(1-(hydroxyethyl))imidazo[4,5-*d*]pyrrolo[2,3-*b*]pyridin-1(6*H*)-yl)piperidin-4-yl)ac etonitrile (10.0 g), 4-dimethylaminopyridine (377 mg) and triethylamine (9.352 g) were dissolved in 100 mL dichloromethane, and di-tert-butyl dicarbonate (6.728 g) was added slowly under nitrogen protection at 0°C. After the reaction was completed, the resultant was rotary dried and purified by column chromatography to obtain compound tert-butyl (*R*)-1-(4-cyanomethylpiperidin-1-yl)-2-(1-hydroxyethyl)imidazo[4,5-*d*]pyrrolo[2,3-*b*]pyridin-1(6*H*) -carboxylate (11.6 g, yield 88%). LC-MS: m/z 425.0 [M + H]⁺.

### Step D:

Compound tert-butyl (*R*)-1-(4-cyanomethylpiperidin-1-yl)-2-(1-hydroxyethyl)imidazo[4,5-*d*]pyrrolo[2,3-*b*]pyridin-1(6*H*) -carboxylate (1.13 g), crude compound o-nitrosophenylpropionic acid (1.8 g), dicyclohexylcarbodiimide (1.104 g) and 4-dimethylaminopyridine (33 mg) were dissolved in 50 mL dichloromethane, and stirred under nitrogen protection at room temperature for 16 hours. After the reaction was completed, the resultant was filtered, rotary dried, and purified by column chromatography to obtain compound tert-butyl (*R*)-1-(4-cyanomethylpiperidin-1-yl)-2-(1-((3-(2-nitrosophenyl)propionyl)oxy)ethyl)imidazo[4,5-*d*] pyrrolo[2,3-*b*]pyridin-6(1*H*)-carboxylate (568 mg, 37% yield). LC-MS: m/z 586.4 [M + H]⁺.

### Step E:

Tert-butyl 5-aminosalicylate (568 mg) and compound tert-butyl (*R*)-1-(4-cyanomethylpiperidin-1-yl)-2-(1-((3-(2-nitrosophenyl)propionyl)oxy)ethyl)imidazo[4,5-*d*] pyrrolo[2,3-*b*]pyridin-6(1*H*)-carboxylate (305 mg) were dissolved in 10 mL acetic acid, and stirred under nitrogen protection at room temperature for 16 hours. After the reaction was completed, the resultant was rotary dried and purified by column chromatography to obtain compound tert-butyl (*R*,*E*)-5-((2-(3-(1-(1-(4-cyanomethylpiperidin-1-yl)-1,6-dihydroimidazo[4,5-d]pyrrolo[2,3-*b*]pyridin -2-yl)ethoxy)-2-propionyl)phenyl)diazenyl)-2-hydroxybenzoate (695 mg). LC-MS: m/z 677.4 [M + H]⁺.

### Step F:

Compound tert-butyl (*R*,*E*)-5-((2-(3-(1-(1-(4-cyanomethylpiperidin-1-yl)-1,6-dihydroimidazo[4,5-*d*]pyrrolo[2,3-*b*]pyridin -2-yl)ethoxy)-2-propionyl)phenyl)diazenyl)-2-hydroxybenzoate (695 mg) was dissolved in 20 mL dichloromethane, and 3 mL trifluoroacetic acid was added and stirred under nitrogen protection at room temperature for 3 hours. After the reaction was completed, the resultant was rotary dried and purified by column chromatography to obtain compound *(R,E)-5-((2-(3-(1-(1-(4-(cyanomethyl)piperidin-1-yl)-1,6-dihydroimidazo[4,5-d]pyrrolo[2,3-b]pyrid* in-2-yl)ethoxy)-3-oxopropyl)phenyl)diazenyl)-2-hydroxybenzoic acid (379 mg, yield 59%).
¹H NMR (400 MHz, DMSO-*d*₆) *δ* 12.01 (s, 1H), 8.59 (s, 1H), 8.32 (d, *J =* 2.5 Hz, 1H), 8.00 (dd, *J =* 8.9, 2.5 Hz, 1H), 7.59 - 7.56 (m, 1H), 7.52 (t, *J* = 3.0 Hz, 1H), 7.47 - 7.45 (m, 1H), 7.42 - 7.38 (m, 1H), 7.35 - 7.31 (m, 1H), 7.07 (d, *J* = 8.9 Hz, 1H), 6.73 (dd, *J* = 3.6, 1.9 Hz, 1H), 6.30 - 6.25 (m, 1H), 3.56 - 3.47 (m, 3H), 3.44 - 3.32 (m, 2H), 3.14 (d, *J =* 10.5 Hz, 1H), 3.01 (d, *J =* 10.4 Hz, 1H), 2.76 - 2.71 (m, 2H), 2.55 (d, *J =* 6.6 Hz, 2H), 2.13-2.00 (m, 1H), 1.97-1.81 (m, 2H), 1.60 (d, *J* = 6.7 Hz, 3H), 1.55 - 1.32 (m, 2H). LC-MS: m/z 621.4 [M + H]⁺.

### Example 2: Synthesis of Compound A2

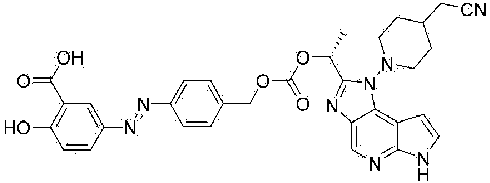

### (R,E)-5-((4-((((1-(1-(4-cyanomethylpiperidin-1-yl)-1,6-dihydroimidazo[4,5-d]pyrrolo[2,3-b]py ridin-2-yl)ethoxy)carbonyl)oxy)methyl)phenyl)diazenyl)-2-hydroxybenzoic acid

The specific synthesis steps are as follows:

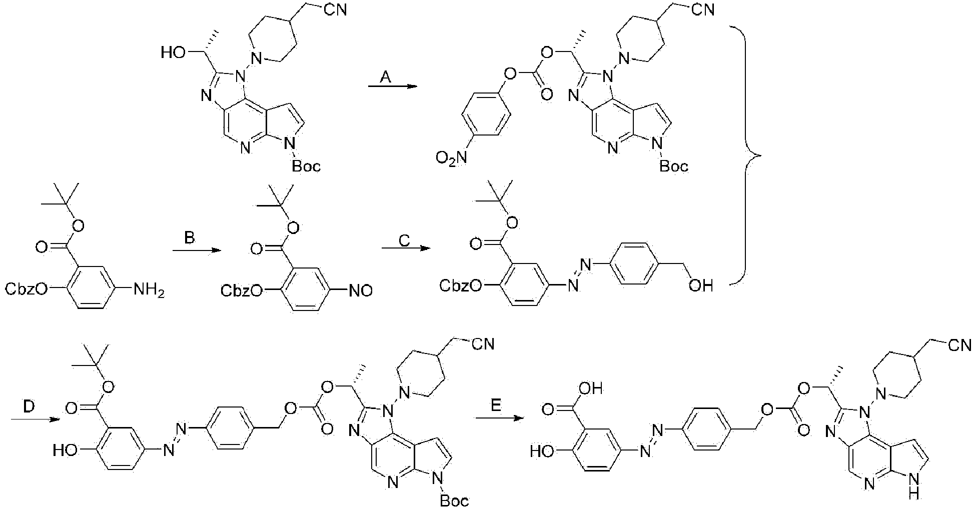

### Step A:

### Tert-butyl

(R)-1-(4-(cyanomethyl)piperidin-1-yl)-2-(1-hydroxyethyl)imidazo[4,5-d]pyrrolo[2,3-b]pyridin-6(1 *H*)-carboxylate (500 mg) was dissolved in 20 mL dichloromethane, and pyridine (279 mg) and phenyl 4-nitrochloroformate (594 mg) were added. The reaction solution was stirred at 20°C for 3 hours. After the reaction was completed, the resultant was washed with saturated brine, dried over anhydrous sodium sulfate, filtered, rotary dried, and purified by column chromatography to obtain compound tert-butyl (*R*)-1-(4-cyanomethylpiperidin-1-yl)-2-(1-(((4-nitrophenoxy)carbonyl)oxy)ethyl)imidazo[4,5-*d*]pyrr olo[2,3-*b*]pyridin-6(1*H*)-carboxylate (639 mg, 92% yield).

### Step B:

Compound tert-butyl 5-amino-2-((benzyloxycarbonyl)oxy)benzoate (1.7 g) was dissolved in 40 mL dichloromethane, and a solution of *m*-chloroperoxybenzoic acid (1.709 g) in dichloromethane (20 mL) was added slowly, and the mixture was stirred at room temperature for 16 hours. After the reaction was completed, the reaction solution was washed with saturated brine, dried over anhydrous sodium sulfate, filtered, rotary dried, and purified by column chromatography to obtain compound tert-butyl 5-nitroso-2-((benzyloxycarbonyl)oxy)benzoate (1.238 g, 70% yield).

### Step C:

Compound tert-butyl 5-amino-2-((benzyloxycarbonyl)oxy)benzoate (1.238 g) and *p*-aminobenzyl alcohol (950 mg) were dissolved in 10 mL acetic acid, and stirred at room temperature for 1 hour. After the reaction was completed, the reaction solution was rotary dried and purified by column chromatography to obtain compound tert-butyl (*E*)-2-((benzyloxycarbonyl)oxy)-5-((4-(hydroxymethyl)phenyl)diazenyl)benzoate (1.167 g, 72.8% yield). LC-MS: m/z 463.1 [M + H]⁺.

### Step D:

Compound tert-butyl (*E*)-2-((benzyloxycarbonyl)oxy)-5-((4-(hydroxymethyl)phenyl)diazenyl)benzoate (500 mg), 4-dimethylaminopyridine (311 mg), triethylamine (257 mg), and compound tert-butyl (*R*)-1-(4-cyanomethylpiperidin-1-yl)-2-(1-(((4-nitrophenoxy)carbonyl)oxy)ethyl)imidazo[4,5-*d*]pyrr olo[2,3-*b*]pyridin-6(1*H*)-carboxylate (784 mg) were dissolved in 10 mL dichloromethane, and the mixture was stirred under nitrogen protection at 30°C for 16 hours. After the reaction was completed, the reaction solution was rotary dried and purified by column chromatography to obtain compound tert-butyl (*R,E*)-2-(1-((((4-((3-(tert-butoxycarbonyl)-4-hydroxyphenyl)diazenyl)benzyl)oxy)carbonyl)oxy)eth yl)-1-(4-cyanomethylpiperidin-1-yl)-1,6-dihydroimidazo[4,5-*d*]pyrrolo[2,3-*b*]pyridin-6(1*H*)-carbox ylate (414 mg, 64% yield). LC-MS: m/z 779.4 [M + H]⁺.

### Step E:

Compound tert-butyl (*R,E*)-2-(1-((((4-((3-(tert-butoxycarbonyl)-4-hydroxyphenyl)diazenyl)benzyl)oxy)carbonyl)oxy)eth yl)-1-(4-cyanomethylpiperidin-1-yl)-1,6-dihydroimidazo[4,5-*d*]pyrrolo[2,3-*b*]pyridin-6(1*H*)-carbox ylate (410 mg) was dissolved in 10 mL dichloromethane, and trifluoroacetic acid (3 mL) was added and stirred at 35°C for 16 hours. After the reaction was completed, the resultant was rotary dried and purified by column chromatography to obtain compound (*R*,*E*)-5-((4-((((1-(1-(4-cyanomethylpiperidin-1-yl)-1,6-dihydroimidazo[4,5-*d*]pyrrolo[2,3-*b*]pyridin -2-yl)ethoxy)carbonyl)oxy)methyl)phenyl)diazenyl)-2-hydroxybenzoic acid (318 mg, 97% yield).
¹H NMR (400 MHz, DMSO-*d*₆) *δ* 12.05 (s, 1H), 8.64 (s, 1H), 8.34 (d, *J* = 2.6 Hz, 1H), 8.09 (dd, *J* = 8.9, 2.6 Hz, 1H), 7.88 (d, *J* = 8.4 Hz, 2H), 7.62-7.56 (m, 2H), 7.53 (t, *J* = 3.1 Hz, 1H), 7.16 (d, *J* = 8.9 Hz, 1H), 6.76 (dd, *J* = 3.6, 1.9 Hz, 1H), 6.22 (q, *J* = 6.6 Hz, 1H), 5.31 (d, *J* = 2.2 Hz, 2H), 3.61 - 3.50 (m, 3H), 3.25-3.18 (m, 1H), 3.08-3.01 (m, 1H), 2.57 (dd, *J* = 6.3, 2.8 Hz, 2H), 2.14 - 2.03 (m, 1H), 1.98-1.90 (m, 1H), 1.88-1.80 (m, 1H), 1.74 (d, *J* = 6.6 Hz, 3H), 1.60-1.49 (m, 1H), 1.45-1.32 (m, 1H). LC-MS: m/z 623.1 [M + H]⁺.

### Example 3: Synthesis of Compound A3

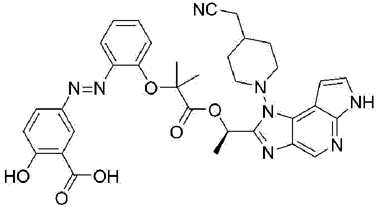

### (R,E)-5-((2-((1-(1-(1-(4-cyanomethylpiperidin-1-yl)-1,6-dihydroimidazo[4,5-d]pyrrolo[2,3-b]p yridin-2-yl)ethoxy)-2-methyl-1-oxopropan-2-yl)oxy)phenyl)diazenyl)-2-hydroxybenzoic acid

The specific synthesis steps are as follows:

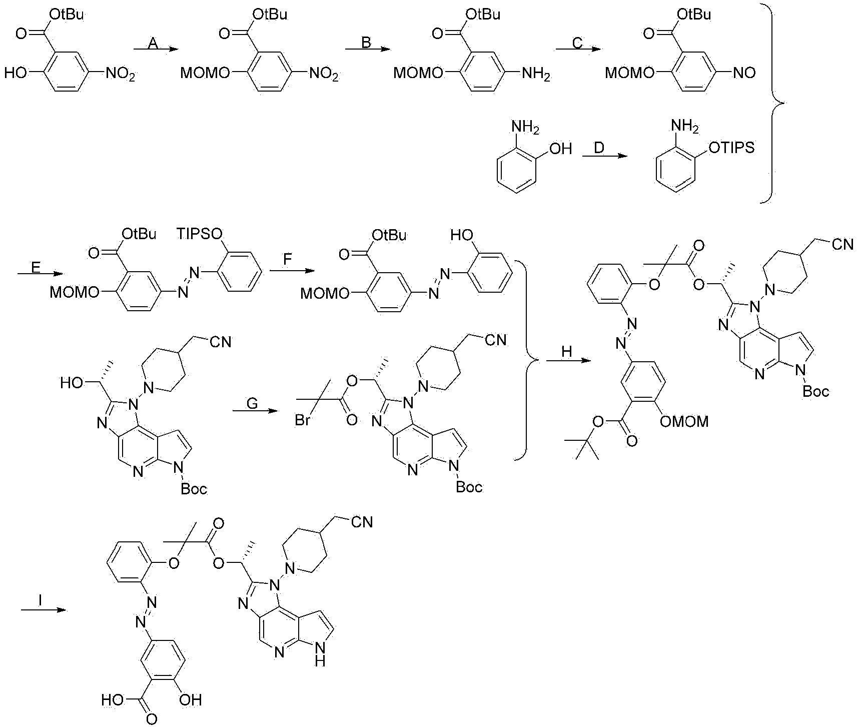

### Step A:

Compound tert-butyl 5-nitrosalicylate(5.6 g) was dissolved in 50 mL *N,N-*dimethylformamide, and sodium hydride (730 mg) was added slowly at 0°C. Then bromomethyl methyl ether (4.095 g) was added, and the reaction solution was stirred at room temperature for 2 hours after the addition. After the reaction was completed, 50 mL water was added, and the resultant was extracted with ethyl acetate (2×50 mL). The organic phases were combined and washed with saturated brine, and the organic phase was dried over anhydrous sodium sulfate, filtered, rotary dried, and purified by column chromatography to obtain compound tert-butyl 2-(methoxymethyl)oxy-5-nitrobenzoate (3.5 g, 52.8% yield).

### Step B:

Compound tert-butyl 2-(methoxymethyl)oxy-5-nitrobenzoate (3.5 g) was dissolved in 50 mL methanol, and 10% palladium/carbon (350 mg) was added and stirred under hydrogen atmosphere at room temperature for 16 hours. After the reaction was completed, the reaction solution was filtered, rotary dried, and purified by column chromatography to obtain compound tert-butyl 2-(methoxymethyl)oxy-5-aminobenzoate (2.753 g, 89.2% yield).

### Step C:

Compound tert-butyl 2-(methoxymethyl)oxy-5-aminobenzoate (900 mg) was dissolved in 30 mL dichloromethane, and a solution of m-chloroperoxybenzoic acid (1.165 g) in dichloromethane (30 mL) was added slowly at 0°C, and the reaction solution was stirred at 0°C for 1.5 hours. After the reaction was completed, the resultant was filtered, rotary dried, and purified by column chromatography to obtain compound tert-butyl 2-(methoxymethyl)oxy-5-nitrosobenzoate (439 mg, 33.6% yield).

### Step D:

Compound 2-hydroxyaniline (2.0 g) was dissolved in 30 mL *N,N*-dimethylformamide, imidazole (1.872 g) and triisopropylsilyl chloride (4.593 g) were added slowly at 0°C, and the reaction solution was stirred under nitrogen atmosphere at room temperature for 16 hours. After the reaction was completed, water (30 mL) was added at 0°C, and the mixture was extracted with ethyl acetate (3×50 mL). Organic phases were combined and washed with saturated brine, and the organic phase was dried over anhydrous sodium sulfate, filtered, rotary dried, and purified by column chromatography to obtain compound 2-((triisopropylsilyl)oxy)aniline (3.87 g, 79.5% yield).

### Step E:

Compound tert-butyl 2-(methoxymethyl)oxy-5-nitrosobenzoate (439 mg) and compound 2-((triisopropylsilyl)oxy)aniline (566 mg) were dissolved in acetic acid (10 mL), and the reaction solution was stirred at room temperature for 16 hours. After the reaction was completed, the resultant was rotary dried and purified by column chromatography to obtain compound tert-butyl (*E*)-2-((methoxymethyl)oxy)-5-((2-((triisopropylsilyl)oxy)phenyl)diazenyl)benzoate (341 mg, 40.3% yield).

### Step F:

Compound tert-butyl (*E*)-2-((methoxymethyl)oxy)-5-((2-((triisopropylsilyl)oxy)phenyl)diazenyl)benzoate (340 mg) and cesium fluoride (301 mg) were added to acetonitrile (6 mL), and the reaction solution was stirred at 60°C for 1 hour. After the reaction was completed, the resultant was filtered, rotary dried, and purified by column chromatography to obtain compound tert-butyl (*E*)-5-((2-hydroxyphenyl)diazenyl)-2-((methoxymethyl)oxy)benzoate (211 mg, 89.1% yield).

### Step G:

Compound tert-butyl (*R*)-1-(4-(cyanomethyl)piperidin-1-yl)-2-(1-hydroxyethyl)imidazo[4,5-*d*]pyrrolo[2,3-*b*]pyridin-6(1 *H*)carboxylate (1.0 g), 1,3-dicyclohexylcarbodiimide (2.634 g), 4-(dimethylamino)pyridine (78 mg) and 2-bromo-2-methylpropionic acid (1.18 g) were added to 50 mL dichloromethane, and the reaction solution was stirred under nitrogen atmosphere at room temperature for 16 hours. After the reaction was completed, the resultant was filtered, rotary dried, and purified by column chromatography to obtain compound tert-butyl (*R*)-2-(1-((2-bromo-2-methylpropionyl)oxy)ethyl)-1-(4-cyanomethylpiperidin-1-yl)imidazo[4,5-*d*]p yrrolo[2,3-*b*]pyridin-6(1H)-carboxylate (1.134 g, 83.9% yield).

### Step H:

Compound tert-butyl (*E*)-5-((2-hydroxyphenyl)diazenyl)-2-((methoxymethyl)oxy)-benzoate (200 mg), compound tert-butyl (*R*)-2-(1-((2-bromo-2-methylpropionyl)oxy)ethyl)-1-(4-cyanomethylpiperidin-1-yl)imidazo[4,5-*d*]p yrrolo[2,3-*b*]pyridin-6(1*H*)-carboxylate (959 mg), and potassium carbonate (231 mg) were added to 10 mL *N,N-*dimethylformamide, and the reaction solution was stirred under nitrogen atmosphere at 80°C for 4 hours. After the reaction was completed, the resultant was filtered, rotary dried, and purified by column chromatography to obtain compound tert-butyl (*R*,*E*)-2-(1-((2-(2-((3-(tert-butoxycarbonyl)-4-((methoxymethyl)oxy)phenyl)diazenyl)phenoxy)-2-m ethylpropionyl)oxy)ethyl)-1-(4-cyanomethylpiperidin-1-yl)imidazo[4,5-*d*]pyrrolo[2,3-*b*]pyridin-6(1 *H*)-carboxylate (446 mg, 93.9% yield). LC-MS: m/z 851.6 [M + H]⁺.

### Step I:

Compound tert-butyl (*R,E*)-2-(1-((2-(2-((3-(tert-butoxycarbonyl)-4-((methoxymethyl)oxy)phenyl)diazenyl)phenoxy)-2-m ethylpropionyl)oxy)ethyl)-1-(4-cyanomethylpiperidin-1-yl)imidazo[4,5-*d*]pyrrolo[2,3-*b*]pyridin-6(1 *H*)-carboxylate (200 mg) was dissolved in 10 mL dichloromethane, and trifluoroacetic acid (1.0 mL) was added and stirred at 60°C for 16 hours. After the reaction was completed, the resultant was rotary dried and purified by column chromatography to obtain compound (*R*,*E*)-5-((2-((1-(1-(1-(4-cyanomethylpiperidin-1-yl)-1,6-dihydroimidazo[4,5-*d*]pyrrolo[2,3-*b*]pyridi n-2-yl)ethoxy)-2-methyl-1-oxopropan-2-yl)oxy)phenyl)diazenyl)-2-hydroxybenzoic acid (55 mg, 36% yield).
¹H NMR (400 MHz, DMSO-*d*₆) *δ* 12.01 (s,1H), 8.61 (s, 1H), 8.32 (d, *J =* 2.5 Hz, 1H), 7.98 (dd, *J* = 8.9, 2.5 Hz, 1H), 7.57-7.50 (m, 2H), 7.41 - 7.37 (m, 1H), 7.21-7.06 (m, 3H), 6.75 (dd, *J* = 3.6, 1.9 Hz, 1H), 6.35 (q, *J =* 6.6 Hz, 1H), 3.81-3.54 (m, 2H), 3.20 - 3.12 (m, 2H), 2.61 (d, *J* = 6.5 Hz, 2H), 2.17-2.06 (m, 1H), 1.98-1.89 (m, 2H), 1.69 (d, *J =* 6.7 Hz, 3H), 1.61-1.52 (m, 8H). LC-MS: m/z 651.3 [M + H]⁺.

### Example 4: Synthesis of Compound A4

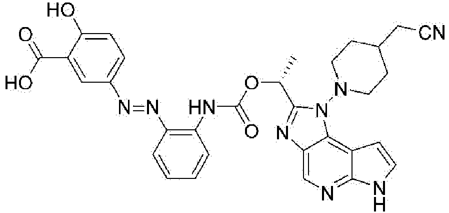

### (R,E)-5-((2-((1-(1-(4-(cyanomethyl)piperidin-1-yl)-1,6-dihydroimidazo[4,5-d]pyrrolo[2,3-b]py ridin-2-yl)ethoxy)carbonyl)amino)phenyl)diazenyl)-2-hydroxybenzoic acid

The specific synthesis steps are as follows:

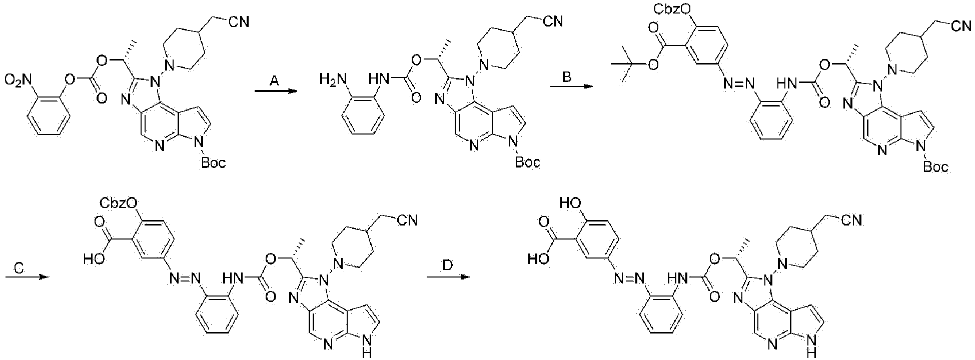

### Step A:

Compound tert-butyl (*R*)-1-(4-(cyanomethyl)piperidin-1-yl)-2-(1-(2-nitrophenoxy)carbonyl)oxy)ethyl)imidazo[4,5-*d*]pyrr olo[2,3-*b*]pyridin-6(1*H*)-carboxylate (368 mg) and phenylenediamine (337 mg) were dissolved in 5 mL *N,N*-dimethylformamide, and the reaction solution was stirred under hydrogen atmosphere at 80°C for 16 hours. After the reaction was completed, the resultant was rotary dried and purified by column chromatography to obtain compound tert-butyl (*R*)-2-(1-((2-aminobenzamide)oxy)ethyl)-1-(4-cyanomethylpiperidin-1-yl)imidazo[4,5-*d*]pyrrolo[2, 3-b]pyridin-6(1H)-carboxylate (300 mg, 86% yield).

### Step B:

Compound tert-butyl (*R*)-2-(1-((2-aminobenzamide)oxy)ethyl)-1-(4-cyanomethylpiperidin-1-yl)imidazo[4,5-*d*]pyrrolo[2, 3-*b*]pyridin-6(1*H*)-carboxylate (150 mg) and compound tert-butyl 5-nitroso-2-((benzyloxycarbonyl)oxy)benzoate (144 mg) were dissolved in 10 mL acetic acid and stirred at 20°C for 16 hours. After the reaction was completed, the resultant was rotary dried and purified by column chromatography to obtain compound tert-butyl (*R*,*E*)-2-(1-(((2-((4-((benzyloxycarbonyl)oxy)-3-(tert-butoxycarbonyl)phenyl)diazenyl)phenyl)form amyl)oxy)ethyl)-1-(4-(cyanomethylpiperidin-1-yl)imidazo[4,5-*d*]pyrrolo[2,3-*b*]pyridin-6(1*H*)-carbo xylate (84 mg, 34.8% yield). LC-MS: m/z 898.4 [M + H]⁺.

### Step C:

Compound tert-butyl (*R,E*)-2-(1-(((2-((4-((benzyloxycarbonyl)oxy)-3-(tert-butoxycarbonyl)phenyl)diazenyl)phenyl)form amyl)oxy)ethyl)-1-(4-(cyanomethylpiperidin-1-yl)imidazo[4,5-*d*]pyrrolo[2,3-*b*]pyridin-6(1*H*)-carbo xylate (75 mg) was dissolved in 10 mL dichloromethane, and trifluoroacetic acid (0.5 mL) was added and stirred at 45°C for 16 hours. After the reaction was completed, the resultant was rotary dried to obtain crude compound (*R,E*)-2-(((benzyloxycarbonyl)oxy)-5-((2-(((1-(1-(4-cyanomethylpiperidin-1-yl)-1,6-dihydroimidaz o[4,5-*d*]pyrrolo[2,3-*b*]pyridin-2-yl)ethoxy)carbonyl)amino)phenyl)diazenyl)benzoic acid (61 mg, 99% yield).

### Step D:

(*R,E*)-2-(((benzyloxycarbonyl)oxy)-5-((2-(((1-(1-(4-cyanomethylpiperidin-1-yl)-1,6-dihydroim idazo[4,5-*d*]pyrrolo[2,3-*b*]pyridin-2-yl)ethoxy)carbonyl)amino)phenyl)diazenyl)benzoic acid (61 mg) was dissolved in 6 mL dichloromethane, and triethylamine (3.0 mL) was added and stirred at 35°C for 72 hours. After the reaction was completed, the resultant was rotary dried to obtain (*R*,*E*)-5-((2-((1-(1-(4-(cyanomethyl)piperidin-1-yl)-1,6-dihydroimidazo[4,5-*d*]pyrrolo[2,3-*b*]pyridin -2-yl)ethoxy)carbonyl)amino)phenyl)diazenyl)-2-hydroxybenzoic acid (18 mg, 36% yield).
¹H NMR (400 MHz, DMSO-*d*₆) *δ* 12.06 (s, 1H), 9.76 (s, 1H), 8.65 (s, 1H), 8.36 (d, *J* = 2.5 Hz, 1H), 8.17 (dd, *J* = 8.9, 2.6 Hz, 1H), 8.07 (d, *J =* 8.3 Hz, 1H), 7.65 (dd, *J =* 8.1, 1.5 Hz, 1H), 7.55-7.47 (m, 2H), 7.21-7.17 (m, 1H), 7.08 (d, *J =* 9.0 Hz, 1H), 6.77 (dd, *J =* 3.6, 1.9 Hz, 1H), 6.37 (q, *J* = 6.6 Hz, 1H), 3.62-3.50 (m, 2H), 3.28 - 3.21 (m, 2H), 2.50-2.45 (m, 1H), 2.12-1.91 (m, 2H), 1.79-1.73 (m, 3H), 1.62 - 1.43 (m, 2H). LC-MS: m/z 608.3 [M + H]⁺.

### Example 5: Synthesis of Compound A5

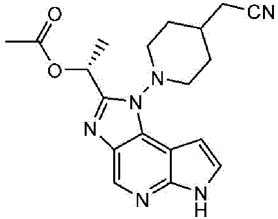

### (R)-1-(1-(4-(cyanomethyl)piperidin-1-yl)-1,6-dihydroimidazo[4,5-d]pyrrolo[2,3-b]pyridin-2-yl )ethyl acetate

The specific synthesis steps are as follows:

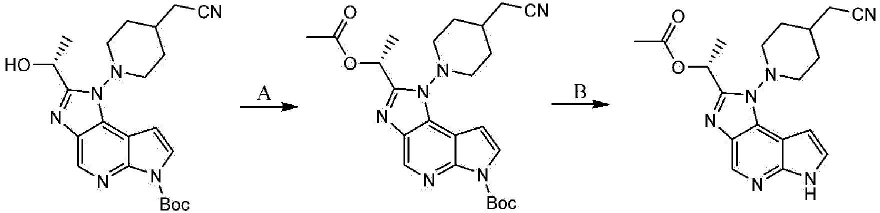

### Step A:

Compound tert-butyl (*R*)-1-(4-(cyanomethyl)piperidin-1-yl)-2-(1-hydroxyethyl)imidazo[4,5-*d*]pyrrolo[2,3-*b*]pyridin-6(1 *H*)carboxylate (50 mg), acetic anhydride (120 mg), triethylamine (238 mg), and 4-dimethylaminopyridine (1 mg) were dissolved in 5 mL dichloromethane, and the reaction solution was stirred at room temperature for 0.5 hour. After the reaction was completed, the resultant was rotary dried and purified by column chromatography to obtain compound tert-butyl (*R*)-2-(1-acetoxyethyl)-1-(4-(cyanomethyl)piperidin-1-yl)-imidazo[4,5-*d*]pyrrolo[2,3-*b*]pyridin-6(1 *H*)-carboxylate (55 mg, 100% yield).

### Step B:

Compound tert-butyl (*R*)-2-(1-acetoxyethyl)-1-(4-(cyanomethyl)piperidin-1-yl)-imidazo[4,5-*d*]pyrrolo[2,3-*b*]pyridin-6(1 *H*)-carboxylate (55 mg) was dissolved in 6 mL dichloromethane, and trifluoroacetic acid (0.3 mL) was added. The reaction solution was stirred at room temperature for 1 hour. After the reaction was completed, the reaction solution washed with saturated sodium bicarbonate solution, dried, and purified to obtain compound (*R*)-1-(1-(4-(cyanomethyl)piperidin-1-yl)-1,6-dihydroimidazo[4,5-*d*]pyrrolo[2,3-*b*]pyridin-2-yl)ethy l acetate (32 mg, 74.1% yield).
¹H NMR (400 MHz, CDCl₃) *δ* 9.80 (s, 1H), 8.81 (s, 1H), 7.42 (dd, *J =* 3.1, 2.0 Hz, 1H), 6.66 (d, *J* = 2.5 Hz, 1H), 6.41 (q, *J* = 6.7 Hz, 1H), 3.82-3.59 (m, 2H), 3.32 - 3.29 (m, 1H), 3.19-3.17 (m, 1H), 2.46 (d, *J =* 6.6 Hz, 2H), 2.15 - 1.67 (m, 11H). LC-MS: m/z 367.2 [M + H]⁺.

### Example 6: Synthesis of Compound A6

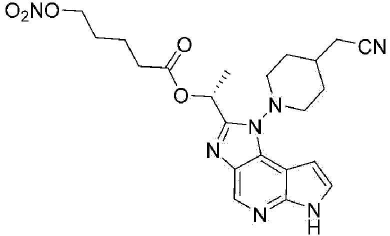

### (R)-1-(1-(4-(cyanomethyl)piperidin-1-yl)-1,6-dihydroimidazo[4,5-d]pyrrolo[2,3-b]pyridin-2-yl )ethyl 5-(nitrooxy)valerate

The specific synthesis steps are as follows:

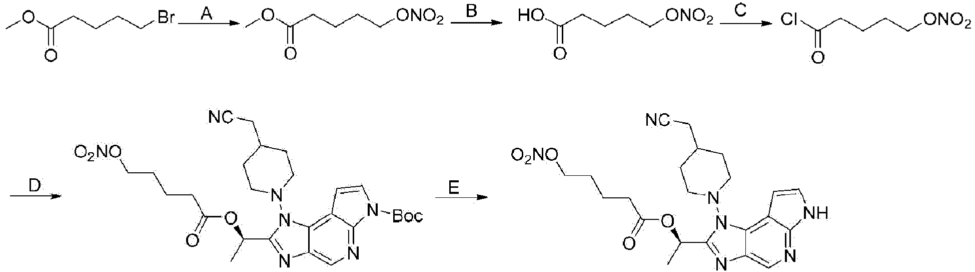

### Step A:

Methyl 5-bromovalerate (2.00 g) was dissolved in 10 mL acetonitrile, and silver nitrate (2.61 g) was added under nitrogen atmosphere and stirred at 70°C for 2 hours. The mixture was filtered under reduced pressure, the filtrate was collected, and solvent was removed under reduced pressure. The residue was purified by silica gel column chromatography (petroleum ether:ethyl acetate = 4:1) to obtain methyl 5-nitrooxy-valerate (1.8 g, 96% yield).
¹H NMR (400 MHz, DMSO-*d*₆) *δ* 4.52 (t, *J =* 6.3 Hz, 2H), 3.59 (s, 3H), 2.37 (t, *J =* 7.2 Hz, 2H), 1.73-1.54 (m, 4H).

### Step B:

Methyl 5-nitrooxy-valerate (1.90 g) was dissolved in 20 mL acetonitrile, and LiOH (10 mL) was added dropwise under nitrogen protection under an ice bath and stirred at room temperature for 2 hours. The mixture was neutralized with 1 N hydrochloric acid to pH = 3 and extracted three times with ethyl acetate. Organic phases were combined, dried over anhydrous sodium sulfate, and suction filtered, and solvent was removed under reduced pressure. The residue was purified by silica gel column chromatography (PE:EA = 4:1) to obtain 5-nitrooxyvaleric acid (1.7 g, 96% yield).
¹H NMR (400 MHz, DMSO-*d*₆) *δ* 12.05 (s, 1H), 4.52 (t, *J* = 6.4 Hz, 2H), 2.35-2.10 (m, 2H), 1.74-1.63 (m, 2H), 1.62- 1.51 (m, 2H).

### Step C:

5-Nitrooxyvaleric acid (64 mg) was dissolved in 6 mL dichloromethane, and thionyl chloride (0.2 mL) was added under nitrogen protection and stirred at room temperature for 3 hours. The solvent was removed under reduced pressure to obtain crude product of compound 5-nitrooxyvaleryl chloride (626 mg).

### Step D:

5-Nitrooxyvaleryl chloride (500 mg) was dissolved in 10 mL dichloromethane, and tert-butyl (*R*)-1-(4-(cyanomethyl)piperidin-1-yl)-2-(1-hydroxyethyl)imidazo[4,5-*d*]pyrrolo[2,3-*b*]pyridin-6(1 *H*)carbonate (50 mg), DMAP (2 mg), TEA (36 mg) were added under nitrogen protection and stirred at room temperature for 3 hours. The solvent was removed under reduced pressure, and the resultant was purified by silica gel column chromatography (methanol:dichloromethane = 1:10) to obtain tert-butyl (*R*)-1-(4-(cyanomethyl)piperidin-1-yl)-2-(1-((5-nitrooxyvaleryl)oxy)ethyl)imidazo[4,5-*d*]pyrrolo[2, 3-*b*]pyridin-6(1*H*)-carboxylate (70 mg, 34% yield).

### Step E:

Compound tert-butyl (*R*)-1-(4-(cyanomethyl)piperidin-1-yl)-2-(1-((5-nitrooxyvaleryl)oxy)ethyl)imidazo[4,5-*d*]pyrrolo[2, 3-*b*]pyridin-6(1*H*)-carboxylate (70 mg) was dissolved in 8 mL dichloromethane, and trifluoroacetic acid (0.2 mL) was added under nitrogen protection and stirred at room temperature for 3 hours. After the reaction was completed, sodium bisulfate solution was added. The mixture was extracted with dichloromethane, organic phases were combined, dried over anhydrous sodium sulfate, filtered, concentrated, and purified by silica gel column chromatography (methanol:dichloromethane = 1:10) to obtain compound (*R*)-1-(1-(4-(cyanomethyl)piperidin-1-yl)-1,6-dihydroimidazo[4,5-*d*]pyrrolo[2,3-*b*]pyridin-2-yl)ethy l 5-(nitrooxy)valerate (17 mg, 30% yield).
¹H NMR (400 MHz, DMSO-*d*₆) *δ* 11.99 (s, 1H), 8.57 (d, *J =* 11.0 Hz, 1H), 7.52 (t, *J =* 2.9 Hz, 1H), 6.75 (s, 1H), 6.30 (q, *J* = 6.6 Hz, 1H), 4.52 (t, *J =* 6.2 Hz, 2H), 3.58 (t, *J* = 11.4 Hz, 2H), 3.18 (d, *J =* 9.6 Hz, 1H), 3.10 (d, *J* = 9.9 Hz, 1H), 2.62 (d, *J =* 6.3 Hz, 2H), 2.41 (dd, *J =* 13.1, 7.1 Hz, 2H), 2.11 (s, 1H), 1.95 (d, *J =* 11.4 Hz, 2H), 1.74-1.56 (m, 8H). LCMS ESI(+)m/z: 470.3 (M+1) .

### Example 7: Synthesis of Compound A7

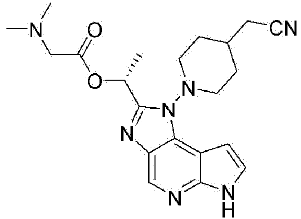

### (R)-1-(1-(4-(cyanomethyl)piperidin-1-yl)-1,6-dihydroimidazo[4,5-d]pyrrolo[2,3-b]pyridin-2-yl )ethyl dimethylglycinate

The specific synthesis steps are as follows:

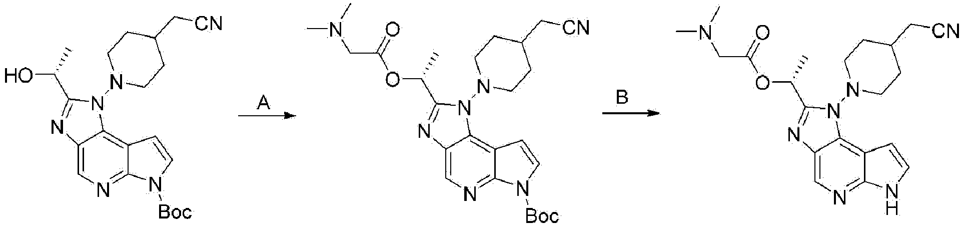

### Step A:

Dimethylglycine (compound 1.2, 1.093 g), 1,3-dicyclohexylcarbodiimide (2.187 g) and 4-(dimethylamino)pyridine (43 mg) were added to 10 mL dichloromethane. The reaction solution was stirred under nitrogen environment at room temperature for 1.5 hours, and then tert-butyl (*R*)-1-(4-cyanomethylpiperidin-1-yl)-2-(1-hydroxyethyl)imidazo[4,5-*d*]pyrrolo[2,3-*b*]pyridin-6(1*H*) -carboxylate (1.5 g) was added and stirred at room temperature for 16 hours. After the reaction was completed, the resultant was filtered, rotary dried, and purified by column chromatography to obtain compound tert-butyl (*R*)-1-(4-cyanomethylpiperidin-1-yl)-2-(1-(dimethylglycyloxyethyl)imidazo[4,5-*d*]pyrrolo[2,3-*b*]py ridin-6(1*H*)-carboxylate (1.2 g, 66.7% yield). LC-MS: m/z 510.1 [M + H]⁺.

### Step B:

Compound tert-butyl (*R*)-1-(4-cyanomethylpiperidin-1-yl)-2-(1-(dimethylglycyloxyethyl)imidazo[4,5-*d*]pyrrolo[2,3-*b*]py ridin-6(1*H*)-carboxylate (1.5 g) was added to 10 mL dichloromethane, and trifluoroacetic acid (2.5 mL) was added, and the reaction solution was stirred at room temperature for 16 hours. After the reaction was completed, the solution was washed with saturated sodium bicarbonate, the organic phase was dried over anhydrous sodium sulfate, filtered, and rotary dried to obtain compound (*R*)-1-(1-(4-(cyanomethyl)piperidin-1-yl)-1,6-dihydroimidazo[4,5-*d*]pyrrolo[2,3-*b*]pyridin-2-yl)ethy l dimethylglycinate (911 mg, 75.5% yield).
¹H NMR (400 MHz, CDCl₃) *δ* 14.17 (s, 1H), 8.65 (s, 1H), 7.62 (s, 1H), 6.81 (s, 1H), 6.38 (q, *J =* 6.6 Hz, 1H), 3.99 (s, 2H), 3.79 - 3.64 (m, 2H), 3.43 (d, *J* = 9.6 Hz, 1H), 3.22 (d, *J =* 10.3 Hz, 1H), 2.93 (s, 6H), 2.62-2.43 (m, 2H), 2.27-1.96 (m, 3H), 1.86 - 1.72 (m, 5H). LC-MS: m/z 410.2 [M + H]⁺.

### Example 8: Synthesis of Compound A8

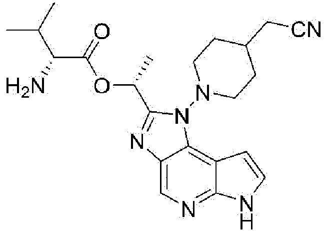

### (R)-1-(1-(4-(cyanomethyl)piperidin-1-yl)-1,6-dihydroimidazo[4,5-d]pyrrolo[2,3-b]pyridin-2-yl )ethyl D-valinate

The specific synthesis steps are as follows:

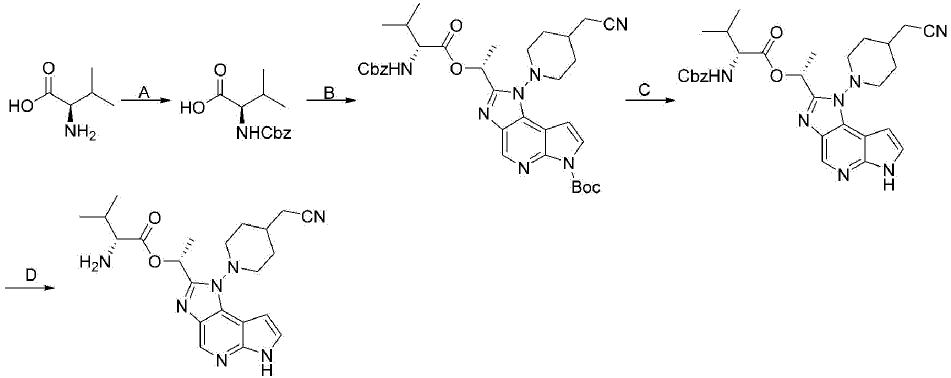

### Step A:

*D*-valine (compound 1, 5.0 g) was added to water (30 mL), and then sodium hydroxide (1.707 g) and benzyl chloroformate (8.01 g) were added, and the reaction solution was stirred under nitrogen atmosphere at room temperature for 16 hours. After the reaction was completed, the reaction solution was extracted with dichloromethane (30 mL×2), and organic phases were combined, dried over anhydrous sodium sulfate, filtered, and rotary dried to obtain compound Cbz-*D*-valine (9.6 g, 89.5% yield).
¹H NMR (400 MHz, CDCl₃) *δ* 7.42-7.26 (m, 6H), 5.37-5.22 (m, 1H), 5.20-5.02 (m, 2H), 4.47-4.25 (m, 1H), 2.33-2.07 (m, 1H), 1.12-0.79 (m, 6H).

### Step B:

Compound Cbz-*D*-valine (4.44 g), 1,3-dicyclohexylcarbodiimide (3.645 g) and 4-(dimethylamino)pyridine (86 mg) were added to 60 mL dichloromethane. The reaction solution was stirred under nitrogen atmosphere at room temperature for 1.5 hours, and then tert-butyl (*R*)-1-(4-cyanomethylpiperidin-1-yl)-2-(1-hydroxyethyl)imidazo[4,5-*d*]pyrrolo[2,3-*b*]pyridin-6(1*H*) -carboxylate (3.0 g) was added and stirred at room temperature for 16 hours. After the reaction was completed, the resultant was filtered, rotary dried, and purified by column chromatography to obtain compound tert-butyl 2-((*R*)-1-(Cbz-*D*-valyloxyethyl)-1-(4-cyanomethylpiperidin-1-yl)-imidazo[4,5-*d*]pyrrolo[2,3-*b*]pyri din-6(1*H*)-carboxylate (1.96 g, 42.2% yield). LC-MS: m/z 658.3 [M + H]⁺.

### Step C:

Compound tert-butyl 2-((*R*)-1-(Cbz-*D*-valyloxyethyl)-1-(4-cyanomethylpiperidin-1-yl)-imidazo[4,5-*d*]pyrrolo[2,3-*b*]pyri din-6(1*H*)-carboxylate (1.96 g) was added to 20 mL dichloromethane, and trifluoroacetic acid (4.0 mL) was added, and the reaction solution was stirred at room temperature for 3 hours. After the reaction was completed, the solution was washed with saturated sodium bicarbonate, and the organic phase was dried over anhydrous sodium sulfate, filtered, rotary dried, and purified by column chromatography to obtain (*R*)-1-(1-(4-cyanomethylpiperidin-1-yl)-1,6-dihydroimidazo[4,5-*d*]pyrrolo[2,3-*b*]pyridin-2-yl)ethyl Cbz-*D*-valinate (1.36 g, 81.8% yield). LC-MS: m/z 558.3 [M + H]⁺.

### Step D:

Compound (*R*)-1-(1-(4-cyanomethylpiperidin-1-yl)-1,6-dihydroimidazo[4,5-*d*]pyrrolo[2,3-*b*]pyridin-2-yl)ethyl Cbz-*D*-valinate (1.36 g) and palladium/carbon (200 mg, 10%) were added to 20 mL methanol, and the reaction solution was stirred under hydrogen atmosphere for 3 hours. After the reaction was completed, the resultant was filtered, rotary dried, and purified by column chromatography to obtain (*R*)-1-(1-(4-(cyanomethyl)piperidin-1-yl)-1,6-dihydroimidazo[4,5-*d*]pyrrolo[2,3-*b*]pyridin-2-yl)ethy 1 *D*-valinate (989 mg, 95.7% yield).
¹H NMR (400 MHz, CDCl₃) *δ* 10.20 -9.47 (m, 1H), 8.82 (d,*J* = 31.1 Hz, 1H), 7.42 (t, *J*= 3.3 Hz, 1H), 6.65 - 6.64 (m, 1H), 6.51-6.39 (m, 1H), 3.80 - 3.66 (m, 2H), 3.43-3.32 (m, 2H), 3.21 -3.15 (m, 1H), 2.46 (d, *J =* 6.3 Hz, 2H), 2.17-2.04 (m, 4H), 1.85-1.70 (m, 4H), 1.26 - 1.22 (m, 1H), 1.03 - 0.85 (m, 6H).LC-MS: m/z 424.2 [M + H]⁺.

### Example 9: Synthesis of Compound A9

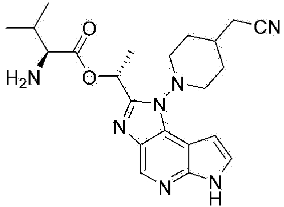

### (R)-1-(1-(4-cyanomethylpiperidin-1-yl)-1,6-dihydroimidazo[4,5-d]pyrrolo[2,3-b]pyridin-2-yl)e thyl L-valinate

The specific synthesis steps are as follows:

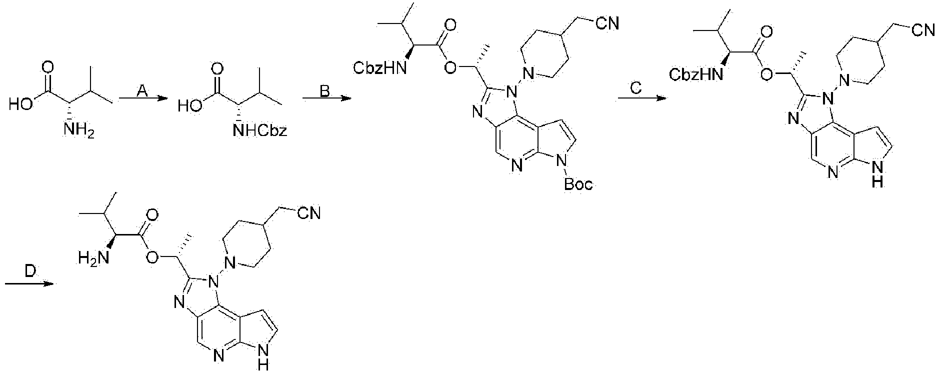

### Step A:

*L*-valine (3.0 g) was added to water (20 mL), and then sodium hydroxide (1.024 g) and benzyl chloroformate (4.81 g) were added, and the reaction solution was stirred under nitrogen atmosphere at room temperature for 16 hours. After the reaction was completed, the reaction solution was extracted with dichloromethane (30 mL×2), and organic phases were combined, dried over anhydrous sodium sulfate, filtered, and rotary dried to obtain compound Cbz-*L*-valine (5.232 g, 81.3% yield).
¹H NMR (400 MHz, CDCl₃) *δ* 7.40-7.27 (m, 6H), 5.11 (s, 2H), 4.44-4.31 (m, 1H), 2.31-2.13 (m, 1H), 1.06 - 0.83 (m, 6H).

### Step B:

Compound Cbz-*L*-valine (3.208 g), 1,3-dicyclohexylcarbodiimide (2.634 g) and 4-(dimethylamino)pyridine (78 mg) were added to 30 mL dichloromethane. The reaction solution was stirred under nitrogen atmosphere at room temperature for 1.5 hours, and then tert-butyl (*R*)-1-(4-(cyanomethyl)piperidin-1-yl)-2-(1-hydroxyethyl)imidazo[4,5-*d*]pyrrolo[2,3-*b*]pyridin-6(1 *H*)carboxylate (2.71 g) was added and stirred at room temperature for 16 hours. After the reaction was completed, the resultant was filtered, rotary dried, and purified by column chromatography to obtain compound tert-butyl 2-((*R*)-1-(Cbz-*L*-valyloxyethyl)-1-(4-(cyanomethyl)piperidin-1-yl)-imidazo[4,5-*d*]pyrrolo[2,3-*b*]pyr idin-6(1*H*)-carboxylate (2.1 g, 50% yield). LC-MS: m/z 658.3 [M + H]⁺.

### Step C:

Compound tert-butyl 2-((*R*)-1-(Cbz-*L*-valyloxyethyl)-1-(4-(cyanomethyl)piperidin-1-yl)-imidazo[4,5-*d*]pyrrolo[2,3-*b*]pyr idin-6(1*H*)-carboxylate (2.1 g) was added to 10 mL dichloromethane, and trifluoroacetic acid (4.0 mL) was added, and the reaction solution was stirred at room temperature for 3 hours. After the reaction was completed, the solution was washed with saturated sodium bicarbonate, and the organic phase was dried over anhydrous sodium sulfate, filtered, rotary dried, and purified by column chromatography to obtain compound (*R*)-1-(1-(4-(cyanomethyl)piperidin-1-yl)-1,6-dihydroimidazo[4,5-*d*]pyrrolo[2,3-*b*]pyridin-2-yl)ethy l Cbz-*L*-valinate (1.622 g, 91.1% yield). LC-MS: m/z 558.3 [M + H]⁺.

### Step D:

Compound (*R*)-1-(1-(4-cyanomethylpiperidin-1-yl)-1,6-dihydroimidazo[4,5-*d*]pyrrolo[2,3-*b*]pyridin-2-yl)ethyl Cbz-*L*-valinate (1.62 g) and palladium/carbon (200 mg, 10%) were added to 20 mL methanol, and the reaction solution was stirred under hydrogen atmosphere for 3 hours. After the reaction was completed, the resultant was filtered, rotary dried, and purified by column chromatography to obtain (*R*)-1-(1-(4-cyanomethylpiperidin-1-yl)-1,6-dihydroimidazo[4,5-*d*]pyrrolo[2,3-*b*]pyridin-2-yl)ethyl *L*-valinate (960 mg, 78.0% yield).
¹H NMR (400 MHz, CDCl₃) *δ* 10.55 (s, 1H), 8.82 (d, *J* = 15.0 Hz, 1H), 7.44 (d, *J =* 3.5 Hz, 1H), 6.65 (dd, *J =* 3.5, 1.7 Hz, 1H), 6.50 - 6.42 (m, 1H), 3.81 - 3.57 (m, 2H), 3.43 - 3.32 (m, 2H), 3.19-3.16 (m, 1H), 2.47 - 2.44 (m, 2H), 2.17 - 2.01 (m, 4H), 1.82-1.72 (m, 8H), 1.02 - 0.85 (m, 6H). LC-MS: m/z 424.2 [M + H]⁺.

### Example 10: Synthesis of Compound A10

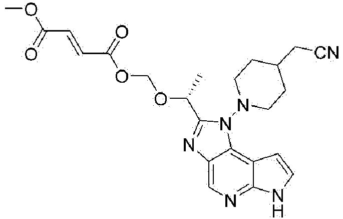

### (R)-(1-(1-(4-(cyanomethyl)piperidin-1-yl)-1,6-dihydroimidazo[4,5-d]pyrrolo[2,3-b]pyridin-2-y l)ethoxy)methyl monomethyl fumarate

The specific synthesis steps are as follows:

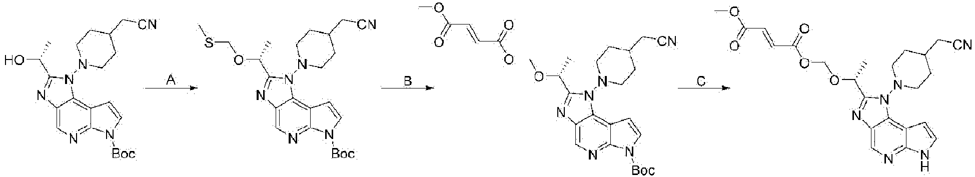

### Step A:

Compound tert-butyl (*R*)-1-(4-cyanomethylpiperidin-1-yl)-2-(1-hydroxyethyl)imidazo[4,5-*d*]pyrrolo[2,3-*b*]pyridin-6(1*H*) carboxylate (300 mg) and sodium hydroxide (85 mg) were added to N,N-dimethylformamide (10 mL), and then chloromethyl methyl sulfide (205 mg) was added. The reaction solution was stirred under a nitrogen environment at room temperature for 3 hours. After the reaction was completed, water (30 mL) was added to the reaction solution, extracted with ethyl acetate (30 mL), and the organic phase was dried over anhydrous sodium sulfate, filtered, rotary dried, and purified by column chromatography to obtain compound tert-butyl (*R*)-1-(4-cyanomethylpiperidin-1-yl)-2-(1-(methylthio)methoxy)ethyl)imidazo[4,5-*d*]pyrrolo[2,3-*b*] pyridin-6(1*H*)carboxylate (262 mg, 76.5% yield). LC-MS: m/z 485.1 [M + H]⁺.

### Step B:

Compound tert-butyl (*R*)-1-(4-cyanomethylpiperidin-1-yl)-2-(1-(methylthio)methoxy)ethyl)imidazo[4,5-*d*]pyrrolo[2,3-*b*] pyridin-6(1*H*)carboxylate (200 mg) and thionyl chloride (0.8 mL) were added to 10 mL dichloromethane. The reaction solution was stirred under nitrogen environment at 55°C for 5 hours, and then rotary-dried. The residue compound and N,N-diisopropylethylamine (213 mg) were dissolved in dichloromethane (10 mL), and then monomethyl fumarate (107 mg) was added and stirred at room temperature for 16 hours. After the reaction was completed, the resultant was washed with saturated brine, dried over anhydrous sodium sulfate, filtered, rotary dried, and purified by column chromatography to obtain compound (*R*)-(1-(6-Boc-1-(4-cyanomethylpiperidin-1-yl)-1,6-dihydroimidazo[4,5-*d*]pyrrolo[2,3-*b*]pyridin-2-y l)ethoxy)methyl monomethyl fumarate (89 mg, 38.1% yield). LC-MS: m/z 567.2 [M + H]⁺.

### Step C:

(*R*)-(1-(6-Boc-1-(4-cyanomethylpiperidin-1-yl)-1,6-dihydroimidazo[4,5-*d*]pyrrolo[2,3-*b*]pyridi n-2-yl)ethoxy)methyl monomethyl fumarate (84 mg) was added to 5 mL dichloromethane, and trifluoroacetic acid (0.3 mL) was added, and the reaction solution was stirred at room temperature for 3 hours. After the reaction was completed, the solution was washed with saturated sodium bicarbonate, and the organic phase was dried over anhydrous sodium sulfate, filtered, rotary dried, and purified by column chromatography to obtain compound (*R*)-(1-(1-(4-(cyanomethyl)piperidin-1-yl)-1,6-dihydroimidazo[4,5-*d*]pyrrolo[2,3-*b*]pyridin-2-yl)eth oxy)methyl monomethyl fumarate (8 mg, 11.6% yield).
¹H NMR (400 MHz, CDCl₃) *δ* 8.87 (s, 1H), 7.59 (d, *J =* 3.7 Hz, 1H), 6.86 (q, *J =* 15.8 Hz, 2H), 6.66 (d, *J =* 3.7 Hz, 1H), 6.51 (s, 2H), 5.36-5.26 (m, 2H), 3.82 (s,3H), 3.77 - 3.67 (m, 2H), 3.32 - 3.23 (m, 2H), 2.48 (d, *J =* 8.0 Hz, 2H), 2.18 - 1.97 (m, 2H), 1.73 (d, *J =* 6.8 Hz, 3H), 1.35 - 1.19 (m, 2H). LC-MS: m/z 467.2[M + H]⁺.

### Example 11: Synthesis of Compound A11

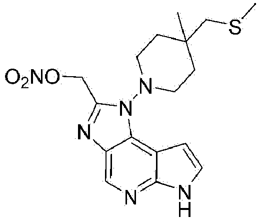

### (1-(4-Methyl-4-((methylthio)methyl)piperidin-1-yl)-1,6-dihydroimidazo[4,5-d]pyrrolo[2,3-b]p yridin-2-yl)methyl nitrate

The specific synthesis steps are as follows:

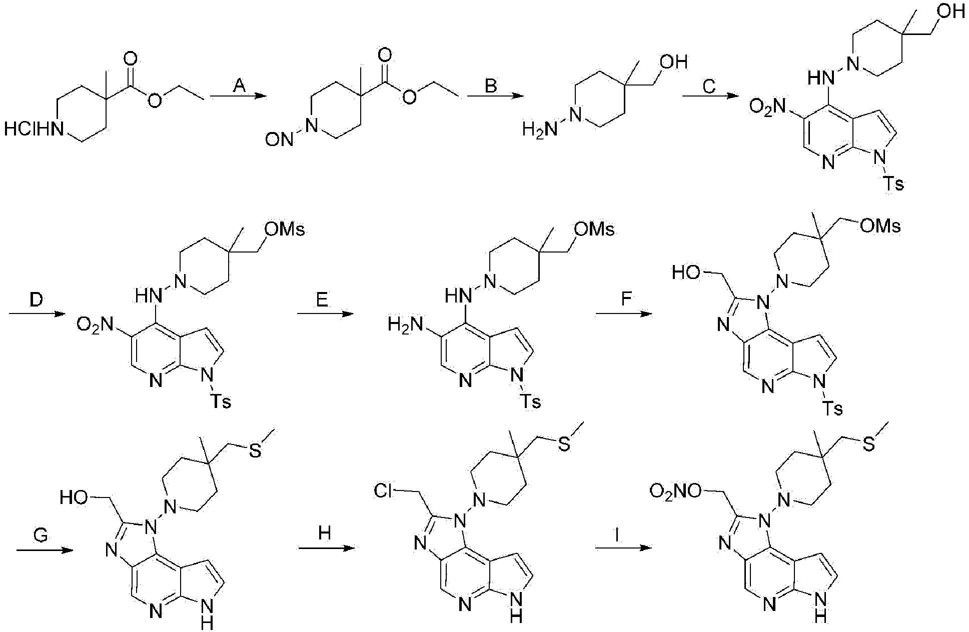

### Step A:

Ethyl 4-methylpiperidin-4-carboxylate hydrochloride (5.0 g) and sodium nitrite (4.153 g) were dissolved in 100 mL water, acetic acid (2.891 g) was added dropwise slowly at 0°C, and after the addition, the reaction solution was stirred at room temperature for 16 hours. After the reaction was completed, the resultant was extracted with ethyl acetate (3×100 mL). Organic phases were combined, washed with saturated brine, dried over anhydrous sodium sulfate, filtered, and rotary dried to obtain crude compound ethyl 4-methyl-1-nitrosopiperidin-4-carboxylate (5.944 g, 100% yield).

### Step B:

Compound ethyl 4-methyl-1-nitrosopiperidin-4-carboxylate (5.944 g) was dissolved in 200 mL tetrahydrofuran, and 2.5 N lithium aluminum hydride solution (39 mL) was added dropwise slowly at 0°C. After the addition, the mixture was stirred under nitrogen protection at room temperature for 3 hours. After the reaction was completed, the reaction solution was cooled to 0°C and quenched by adding water (3.9 mL) slowly, and then 2 N sodium hydroxide (3.9 mL) and water (11.7 mL) were added successively. The solution was stirred at room temperature for 0.5 hour, filtered, and rotary dried to obtain crude compound ethyl 4-methyl-1-aminopiperidin-4-carboxylate (6.6 g, 100% yield).

### Step C:

Compound 4-chloro-5-nitro-1*H*-pyrrolo[2,3-b]pyridine (6.96 g), crude compound ethyl 4-methyl-1-nitrosopiperidin-4-carboxylate (6.6 g) and N,N-diisopropylethylamine (15.346 g) were dissolved in 100 mL isopropanol and stirred under nitrogen protection at 85°C for 16 hours. After the reaction was completed, the reaction solution was cooled to room temperature, filtered, and rotary dried to obtain compound (4-methyl-1-((5-nitro-1-*p*-tosyl-1*H*-pyrrolo[2,3-*b*]pyridin-4-yl)amino)piperidin-4-yl)methanol (6.496 g, 71.5% yield).

### Step D:

(4-Methyl-1-((5-nitro-1-*p*-tosyl-1*H*-pyrrolo[2,3-*b*]pyridin-4-yl)amino)piperidin-4-yl)methanol (6.496 g) was dissolved in 200 mL dichloromethane, and triethylamine (3.239 g) and methanesulfonyl chloride (1.86 g) were added at 0°C dropwise. After the addition, the mixture was stirred at room temperature for 2 hours. After the reaction was completed, the reaction solution was washed with saturated brine (3×100 mL), dried over anhydrous sodium sulfate, filtered, and rotary dried to obtain crude compound (4-methyl-1-((5-nitro-1-*p*-tosyl-1*H*-pyrrolo[2,3-b]pyridin-4-yl)amino)piperidin-4-yl)methyl methanesulfonate (7.3 g, 99% yield).

### Step E:

(4-Methyl-1-((5-nitro-1-*p*-tosyl-1H-pyrrolo[2,3-b]pyridin-4-yl)amino)piperidin-4-yl)methyl methanesulfonate (2.7 g) was dissolved in 60 mL ethanol, and then iron powder (2.333 g) and a solution of ammonium chloride (1.117 g) in water (6 mL) were added and stirred at 80°C for 1 hour. After the reaction was completed, the reaction solution was filtered, rotary dried, and purified to obtain (4-methyl-1-((5-amino-1-*p*-tosyl-1*H*-pyrrolo[2,3-*b*]pyridin-4-yl)amino)piperidin-4-yl)methyl methanesulfonate (2.14 g, 84% yield).

### Step F:

Hydroxylacetamide (799 mg) and triethyloxonium tetrafluoroborate (2.021 g) were dissolved in 20 mL anhydrous tetrahydrofuran, stirred at 35°C for 0.5 hour, and rotary dried. Then the residue compound and compound (4-methyl-1-((5-amino-1-*p*-tosyl-1*H*-pyrrolo[2,3-*b*]pyridin-4-yl)amino)piperidin-4-yl)methyl methanesulfonate (600 mg) were dissolved in 30 mL ethanol and stirred at 75°C for 1 hour. After the reaction was completed, the reaction solution was rotary dried and purified to obtain crude (4-(2-hydroxymethyl-6-*p*-tosylimidazo[4,5-*d*]pyrrolo[2,3-*b*]pyridin-1(6*H*)-yl)-1-methylcyclohexyl) methyl methanesulfonate (647 mg, 100% yield).

### Step G:

(4-(2-Hydroxymethyl-6-*p*-tosylimidazo[4,5-*d*]pyrrolo[2,3-*b*]pyridin-1(6*H*)-yl)-1-methylcycloh exyl)methyl methanesulfonate (647 mg) and a 20% aqueous solution of sodium methanethiolate (2.5 mL) were dissolved in 10 mL *N,N*-dimethylformamide and stirred at 100°C for 1 hour. After the reaction was completed, 60 mL water was added to the reaction solution and extracted with dichloromethane (2×60 mL). Organic phases were combined, washed with saturated brine, dried over anhydrous sodium sulfate, filtered, rotary dried, and purified to obtain compound (1-(4-methyl-4-((methylthio)methyl)piperidin-1-yl)-1,6-dihydroimidazo[4,5-*d*]pyrrolo[2,3-*b*]pyridi n-2-yl)methanol (126 mg, 30.8% yield).

### Step H:

(1-(4-Methyl-4-((methylthio)methyl)piperidin-1-yl)-1,6-dihydroimidazo[4,5-*d*]pyrrolo[2,3-*b*]p yridin-2-yl)methanol (65 mg) and thionyl chloride (0.5 mL) were dissolved in 50 mL dichloromethane and stirred at room temperature for 16 hours. After the reaction was completed, the reaction solution was washed with saturated sodium bicarbonate and saturated brine successively, and dried over anhydrous sodium sulfate, filtered, and rotary dried to obtain compound 2-chloromethyl-1-(4-methyl-4-((methylthio)methyl)piperidin-1-yl)-1,6-dihydroimidazo[4,5-*d*]pyrro lo[2,3-*b*]pyridine (68 mg, 99% yield).

### Step I:

2-Chloromethyl-1-(4-methyl-4-((methylthio)methyl)piperidin-1-yl)-1,6-dihydroimidazo[4, 5-*d*] pyrrolo[2,3-*b*]pyridine (68 mg) and silver nitrate (327 mg) were added to 10 mL acetonitrile and stirred at 50°C for 16 hours. After the reaction was completed, the reaction solution was filtered and rotary dried to obtain compound (1-(4-methyl-4-((methylthio)methyl)piperidin-1-yl)-1,6-dihydroimidazo[4,5-*d*]pyrrolo[2,3-*b*]pyridi n-2-yl)methyl nitrate (18 mg, 24.5% yield).
¹H NMR (400 MHz, CDCl₃) *δ* 10.09 (s, 1H), 8.79 (s, 1H), 7.48 (s, 1H), 6.84 - 6.76 (m, 1H), 5.82 (s, 2H), 3.82-3.66 (m, 2H), 3.06 (d, *J =* 10.7 Hz, 2H), 2.59 (s, 1H), 2.28 (s, 1H), 2.21 (s, 3H), 2.01-1.68 (m, 4H), 1.38 (s, 3H).LC-MS: m/z 391.2 [M + H]⁺.

### Example 12: Synthesis of Compound A12

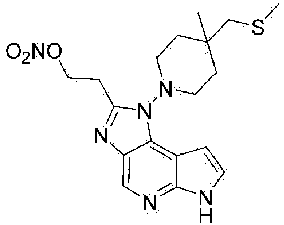

### 2-(1-(4-Methyl-4-((methylthio)methyl)piperidin-1-yl)-1,6-dihydroimidazo[4,5-d]pyrrolo[2,3-b ]pyridin-2-yl)ethyl nitrate

The specific synthesis steps are as follows:

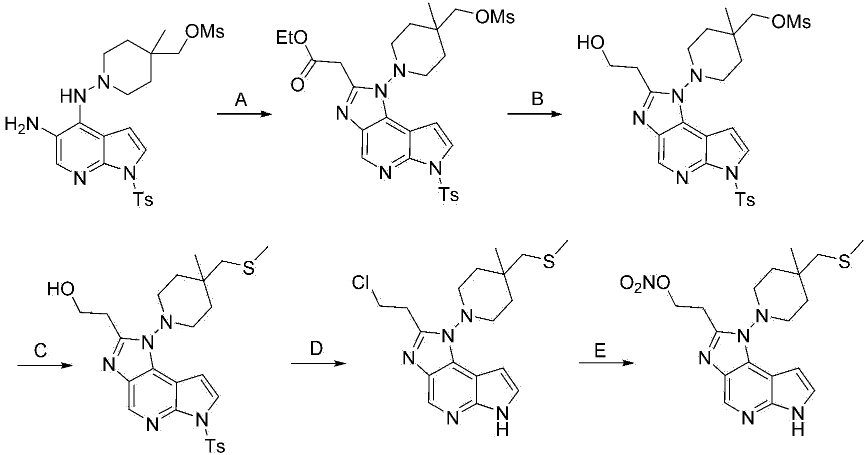

### Step A:

Ethyl succinamate (2.067 g) and triethyloxonium tetrafluoroborate (2.994 g) were dissolved in 40 mL anhydrous tetrahydrofuran, stirred at 35°C for 2 hours, and rotary dried. Then the residue and (4-methyl-1-((5-amino-1-*p*-tosyl-1*H*-pyrrolo[2,3-*b*]pyridin-4-yl)amino)piperidin-4-yl)methyl methanesulfonate (800 mg) were dissolved in 80 mL ethanol and stirred at 75°C for 0.5 hour. After the reaction was completed, the reaction solution was rotary dried and purified to obtain crude compound ethyl 2-(1-(4-methyl-4-(((methanesulfonyl)oxy)methyl)cyclohexyl)-6-*p*-tosyl-1,6-dihydroimidazo[4,5-*d*] pyrrolo[2,3-*b*]pyridin-2-yl)acetate (3.6 g, 100% yield).

### Step B:

Compound ethyl 2-(1-(4-methyl-4-(((methanesulfonyl)oxy)methyl)cyclohexyl)-6-*p*-tosyl-1,6-dihydroimidazo[4,5-*d*] pyrrolo[2,3-*b*]pyridin-2-yl)acetate (1.0 g) was dissolved in 30 mL ethanol, and sodium borohydride (1.253 g) was added slowly. Then the reaction solution was stirred at 50°C for 6 hours. After the reaction was completed, the resultant was filtered, rotary dried, and purified by column chromatography to obtain compound 2-(1-(4-methyl-4-((methylthio)methyl)piperidin-1-yl)-6-*p*-tosyl-1,6-dihydroimidazo[4,5-*d*]pyrrolo[2 ,3-*b*]pyridin-2-yl)-1-ethanol (352 mg, 37.8% yield).

### Step C:

2-(1-(4-Methyl-4-((methylthio)methyl)piperidin-1-yl)-6-*p*-tosyl-1,6-dihydroimidazo[4,5-*d*]pyrr olo[2,3-*b*]pyridin-2-yl)-1-ethanol (351 mg) and sodium methanethiolate (3 mL) were dissolved in 10 mL *N,N-*dimethylformamide, and the reaction solution was stirred at 80°C for 2 hours. After the reaction was completed, 60 mL water was added to the reaction solution and extracted with dichloromethane (2×60 mL). Organic phases were combined, washed with saturated brine, dried over anhydrous sodium sulfate, filtered, rotary dried, and purified to obtain compound 2-(1-(4-methyl-4-((methylthio)methyl)piperidin-1-yl)-6-*p*-tosyl-1,6-dihydroimidazo[4,5-*d*]pyrrolo[2 ,3-*b*]pyridin-2-yl)-1-ethanol (79 mg, 35.2% yield).

### Step D:

2-(1-(4-Methyl-4-((methylthio)methyl)piperidin-1-yl)-6-*p*-tosyl-1,6-dihydroimidazo[4,5-*d*]pyrr olo[2,3-*b*]pyridin-2-yl)-1-ethanol (79 mg) and thionyl chloride (0.5 mL) were dissolved in 50 mL dichloromethane and stirred at room temperature for 2 hours. After the reaction was completed, the reaction solution was washed with saturated sodium bicarbonate and saturated brine successively, and dried over anhydrous sodium sulfate, filtered, and rotary dried to obtain compound 2-(2-chloroethyl)-1-(4-methyl-4-((methylthio)methyl)piperidin-1-yl)-1,6-dihydroimidazo[4,5-*d*]pyrr olo[2,3-*b*]pyridine (45 mg, 54.2% yield).

### Step E:

2-(2-Chloroethyl)-1-(4-methyl-4-((methylthio)methyl)piperidin-1-yl)-1,6-dihydroimidazo[4,5-*d*]pyrrolo[2,3-*b*]pyridine (45 mg) and silver nitrate (405 mg) were added to 30 mL acetonitrile and stirred at 50°C for 16 hours. After the reaction was completed, the reaction solution was filtered and rotary dried to obtain compound 2-(1-(4-methyl-4-((methylthio)methyl)piperidin-1-yl)-1,6-dihydroimidazo[4,5-*d*]pyrrolo[2,3-*b*]pyri din-2-yl)ethyl nitrate (10 mg, 10% yield).
¹H NMR (400 MHz, CDCl₃) *δ* 10.00 (s, 1H), 8.72 (s, 1H), 7.44 (s, 1H), 6.79 - 6.71 (m, 1H), 5.05-4.93 (m, 2H), 3.82 - 3.68 (m, 2H), 3.46 (t, *J =* 6.8 Hz, 2H), 3.01 (d, *J =* 11.5 Hz, 2H), 2.60 (s, 1H), 2.29 (s, 1H), 2.21 (s, 2H), 1.95-1.66 (m, 4H), 1.38 (s, 2H), 1.22 (d, *J =* 28.5 Hz, 2H). LC-MS: m/z 405.2 [M + H]⁺.

### Example 13: Synthesis of Compound B1

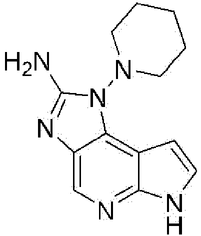

### 1-(Piperidin-1-yl)-1,6-dihydroimidazo[4,5-d]pyrrolo[2,3-b]pyridin-2-amine

The specific synthesis steps are as follows:

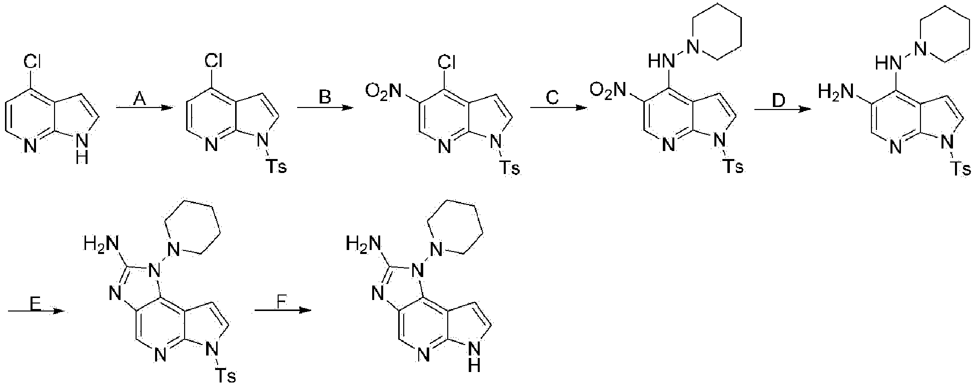

### Step A:

4-Chloro-7-azaindole (50.01 g) was dissolved in 1 L dichloromethane, and triethylamine (66.34 g) was added. Then, p-tosyl chloride (64.36 g) and 4-dimethylaminopyridine (0.4 g) were respectively added at 0°C, and the solution was heated to room temperature and stirred for 16 hours. After the reaction was completed, the solvent was removed under reduced pressure. Crude compound 4-chloro-1-p-tosyl-1*H*-pyrrolo[2,3-*b*]pyridine (99.8 g, 99% yield of crude) was obtained.

### Step B:

Compound 4-chloro-1-p-tosyl-1H-pyrrolo[2,3-b]pyridine (50.0 g) was dissolved in 600 mL dichloromethane. Tetrabutylammonium nitrate (74.44 g) and trifluoroacetic anhydride (53.05 g) were respectively added at 0°C and stirred under nitrogen protection at room temperature for 20 hours. After the reaction was completed, the resultant was quenched with 500 mL saturated sodium bicarbonate aqueous solution. Organic phase was separated, and aqueous phase was extracted with 1.2 L dichloromethane in two fractions. Organic phases were combined, washed with an appropriate amount of water and saturated sodium chloride aqueous solution, respectively, dried over anhydrous sodium sulfate, filtered, and rotary evaporated. The residue slurry was pulped with 80 mL ethyl acetate under ultrasound, filtered, and washed with 100 mL a mixed solvent of ethyl acetate and petroleum ether (volume ratio = 3:7). The filter cake was dried to obtain compound 4-chloro-5-nitro-1-*p*-tosyl-1*H*-pyrrolo[2,3-b]pyridine (30.1 g, 52% yield).
¹H NMR (400 MHz, DMSO-*d₆*) *δ* 9.08 (s, 1H), 8.26 (d, *J* = 4.1 Hz, 1H), 8.05 (d, *J* = 8.4 Hz, 2H), 7.47 (d, *J* = 8.2 Hz, 2H), 7.09 (d, *J* = 4.1 Hz, 1H), 2.37 (s, 3H).

### Step C:

Compound 4-chloro-5-nitro-1-p-tosyl-1*H*-pyrrolo[2,3-*b*]pyridine (6.78 g), N,N-diisopropylethylamine (5.40 g), and 1-aminopiperidine (2.28 g) were added to 250 mL isopropanol (suspension). The mixture was stirred and reacted at 95°C for 16 hours. After the reaction was completed, the resultant was cooled to room temperature, 500 mL water was added, and extracted with 750 mL ethyl acetate in three fractions. The organic phases were combined, dried over anhydrous magnesium sulfate, filtered, rotary evaporated and purified (dichloromethane and methanol in a volume ratio of 40:1) to obtain compound 5-nitro-*N*-(piperidin-1-yl)-1-*p*-tosyl-1*H*-pyrrolo[2,3-*b*]pyridin-4-amine (5.02 g, 64% yield). LCMS ESI(+)m/z: 416.1(M+1).

### Step D:

Compound 5-nitro-*N*-(piperidin-1-yl)-1-*p*-tosyl-1*H*-pyrrolo[2,3-*b*]pyridin-4-amine (4.88 g) was added to a mixed solution of 30 mL ethanol and 10 mL water (suspension), and then solid ammonium chloride (1.88 g) and iron powder (1.96 g) were added successively. The mixture was heated to 80°C and stirred for 3 hours. After the reaction was completed, the reaction solution was filtered, and the filter residue was washed with 50 mL ethyl acetate. 50 mL water was added to the filtrate, and extracted with 240 mL ethyl acetate in three fractions. Organic phases were combined, washed with 100 mL saturated brine, dried over anhydrous sodium sulfate, and suction filtered. The filtrate was concentrated under reduced pressure to obtain crude compound *N⁴*-(piperidin-1-yl)-1-*p*-tosyl-1*H*-pyrrolo[2,3-*b*]pyridin-4,5-diamine (4.01 g, crude 88% yield). LCMS ESI(+)m/z: 386.1(M+1).

### Step E:

Compound *N⁴*-(piperidin-1-yl)-1-*p*-tosyl-1*H*-pyrrolo[2,3-*b*]pyridin-4,5-diamine (150 mg) was dissolved in 3 mL methanol, and a solution of cyanogen bromide in dichloromethane (3.0 M, 0.19 mL) was added and stirred at room temperature for 16 hours. Then 1 N sodium hydroxide solution (10 mL) was added and stirred for 30 minutes, and extracted with 45 mL ethyl acetate in three fractions. Organic phases were combined, washed with 30 mL saturated brine, dried over anhydrous sodium sulfate, and suction filtered. The filtrate was concentrated under reduced pressure and purified by silica gel column chromatography (dichloromethane:methanol volume ratio = 20:1) to obtain compound 1-(piperidin-1-yl)-6-*p*-tosyl-1,6-dihydroimidazo[4,5-*d*]pyrrolo[2,3-*b*]pyridin-2-amine (102 mg, 64% yield). LCMS ESI(+)m/z: 411.1(M+1).

### Step F:

1-(Piperidin-1-yl)-6-*p*-tosyl-1,6-dihydroimidazo[4,5-*d*]pyrrolo[2,3-*b*]pyridin-2-amine (102 mg) was dissolved in 3 mL acetonitrile, and sodium hydroxide (25 mg) was added and stirred and reacted at 60°C for 5 hours. 20 mL water was added to the reaction solution, and extracted with 45 mL ethyl acetate fractions. Organic phases were combined, washed with 50 mL saturated brine, dried over anhydrous sodium sulfate, concentrated under reduced pressure, and purified by silica gel column chromatography (dichloromethane:methanol volume ratio = 15:1) and high performance liquid chromatography to obtain compound 1-(piperidin-1-yl)-1,6-dihydroimidazo[4,5-*d*]pyrrolo[2,3-*b*]pyridin-2-amine (17 mg, 26% yield).
¹H NMR (400 MHz, DMSO-*d₆*) *δ* 12.16 (s, 1H), 8.61 (s, 2H), 8.35 (s, 1H), 7.68 (t, 1H), 6.71 (dd, *J* = 3.3, 1.7 Hz, 1H), 3.47-3.36 (m, 2H), 3.24-3.13 (m, 2H), 1.91-1.74 (m, 5H), 1.57-1.46 (m, 1H). LCMS ESI(+)m/z: 257.1(M+1)_{∘}

### Example 14: Synthesis of Compound B2

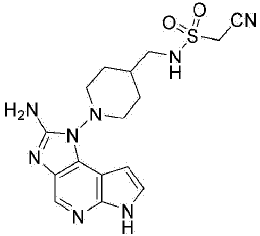

### N-((1-(2-aminoimidazo[4,5-d]pyrrolo[2,3-b]pyridin-1(6H)-yl)-piperidin-4-yl)methyl)-1-cyano methanesulfonamide

The specific synthesis steps are as follows:

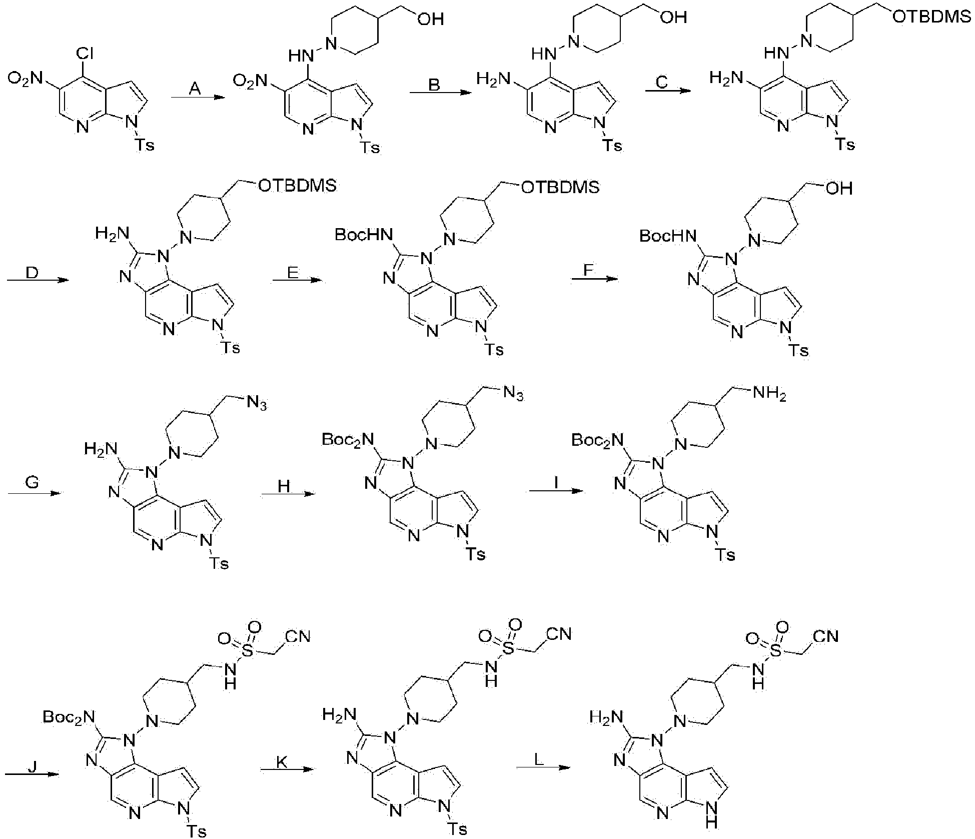

### Step A:

4-Chloro-5-nitro-1-tosyl-1*H*-pyrrolo[2,3-*b*]pyridine (22 g) was dissolved in 200 mL isopropanol, and then DIEA (48.5 g) and (1-aminopiperidin-4-yl)methanol (6.35 g) were added. The reaction solution was stirred at 88°C for 16 hours. After the reaction was completed, the reaction solution was rotary dried and purified by column chromatography to obtain (1-((5-nitro-1-*p*-tosyl-1*H*-pyrrolo[2,3-*b*]pyridin-4-yl)amino)piperidin-4-yl)methanol (27.8 g, 100% yield). LC-MS: m/z 446.30 [M + H]⁺.

### Step B:

(1-((5-Nitro-1-*p*-tosyl-1*H*-pyrrolo[2,3-*b*]pyridin-4-yl)amino)piperidin-4-yl)methanol (12 g) was dissolved in 400 mL ethanol and 80 mL water, and then iron powder (12.1 g) and ammonium chloride (2890 mg) were added. The reaction solution was stirred at 75°C for half an hour. After the reaction was completed, the resultant was filtered, and the filtrate was rotary dried and purified by column chromatography to obtain compound *N⁴*-(piperidin-1-yl)-1-*p*-tosyl-1*H*-pyrrolo[2,3-*b*]pyridin-4,5-diamine (10 g, 89% yield). LC-MS: m/z 415.3 [M + H]⁺.

### Step C:

Compound *N⁴*-(piperidin-l-yl)-1-*p*-tosyl-1*H*-pyrrolo[2,3-*b*]pyridin-4,5-diamine (3.00 g) was dissolved in 10 mL DMF, and then imidazole (1.47 g) and tert-butyldimethylchlorosilane (1.63 g) were added successively under nitrogen protection and an ice bath. After the addition, the mixture was stirred at 0°C for 2 hours. The reaction solution was poured into 100 mL water, extracted three times with 50 mL ethyl acetate. Organic phases were combined, washed with 10 mL saturated brine, dried over anhydrous sodium sulfate, concentrated, and purified by silica gel column chromatography (ethyl acetate: petroleum ether = 1:2) to obtain compound *N⁴*-(4-(((tert-butyldimethylsilyl)oxy)methyl)piperidin-1-yl)-1-*p*-tosyl-1*H*-pyrrolo[2,3-*b*]pyridin-4,5-diamine (3.70 g, 97% yield).
¹H NMR (400 MHz, DMSO-*d*₆) *δ* 8.00 (d, *J =* 8.4 Hz, 2H), 7.81 (s, 1H), 7.43 (d, *J* = 4.0 Hz, 1H), 7.22 (d, *J* = 8.1 Hz, 2H), 6.94 (d, *J* = 4.1 Hz, 1H), 5.20 (s, 1H), 3.48 (d, *J* = 6.1 Hz, 2H), 3.24 - 3.16 (m, 2H), 2.35 (s, 3H), 2.33 - 2.24 (m, 2H), 1.83 - 1.75 (m, 2H), 1.47 - 1.35 (m, 2H), 0.90 (s, 9H), 0.05 (s, 6H). LCMS ESI(+)m/z: 530.0 (M+1) .

### Step D:

*N⁴*-(4-(((tert-butyldimethylsilyl)oxy)methyl)piperidin-1-yl)-1-*p*-tosyl-1*H*-pyrrolo[2,3-*b*]pyridin -4,5-diamine (3.70 g) was suspended in 200 mL methanol, and cyanogen bromide (814 mg) was added under nitrogen protection at room temperature. The mixture was stirred at room temperature for 16 hours. The solvent was evaporated under reduced pressure, and saturated sodium bicarbonate solution (15 mL), saturated sodium thiosulfate (15 mL) and a mixed solvent of methanol and ethyl acetate (volume ratio = 1:10, 100 mL) were added and stirred at room temperature for 15 minutes. Organic phase was separated, and the aqueous phase was extracted three times with a mixed solvent of methanol and ethyl acetate (50 mL). Organic phases were combined, washed with saturated brine (20 mL), and dried over anhydrous sodium sulfate. The solvent was evaporated under reduced pressure to obtain crude product compound 1-(4-(((tert-butyldimethylsilyl)oxy)methyl)piperidin-1-yl)-6-*p*-tosyl-1,6-dihydroimidazo[4,5-*d*]pyrr olo[2,3-*b*]pyridin-2-amine (3.87 g, yield 100%). LCMS ESI(+)m/z: 555.2(M+1).

### Step E:

1-(4-(((tert-Butyldimethylsilyl)oxy)methyl)piperidin-1-yl)-6-*p*-tosyl-1,6-dihydroimidazo[4,5-*d* ]pyrrolo[2,3-*b*]pyridin-2-amine (3.87 g) was dissolved in 200 mL methanol, and triethylamine (3.90 mL) and di-tert-butyl dicarbonate (1.83 g) were added at room temperature. The mixture was stirred for 16 hours at room temperature under nitrogen protection, and then concentrated under reduced pressure and purified by silica gel column chromatography (ethyl acetate:petroleum ether = 1:7) to obtain 1-(4-(((tert-butyldimethylsilyl)oxy)methyl)piperidin-1-yl)-6-*p*-tosyl-1,6-dihydroimidazo[4,5-*d*]pyrr olo[2,3-*b*]pyridin-2-amine (3.03 g, 66% yield).
¹H NMR (400 MHz, CDCl₃) *δ* 8.53 (s, 1H), 8.04 (d, *J* = 8.4 Hz, 2H), 7.78 (s, 1H), 7.62 (d, *J =* 4.0 Hz, 1H), 7.26 (d, *J =* 8.0 Hz, 2H), 6.96 (d, *J* = 4.0 Hz, 1H), 4.08 - 3.95 (m, 2H), 3.51 (d, *J* = 5.9 Hz, 2H), 3.11 - 3.02 (m, 2H), 2.36 (s, 3H), 1.89 - 1.79 (m, 2H), 1.69 (s, 9H), 1.54 - 1.37 (m, 3H), 0.91 (s, 9H), 0.06 (s, 6H).

### Step F:

1-(4-(((tert-Butyldimethylsilyl)oxy)methyl)piperidin-1-yl)-6-*p*-tosyl-1,6-dihydroimidazo[4,5-*d* ]pyrrolo[2,3-*b*]pyridin-2-amine (3.03 g) was dissolved in 120 mL tetrahydrofuran, and tetrabutylammonium fluoride trihydrate (5.84 g) was added at room temperature and stirred at room temperature for 2 hours. 250 mL ethyl acetate was added, and the mixture was washed three times with 100 mL water. Organic phases were combined, washed with saturated brine (30 mL), and dried over anhydrous sodium sulfate. The solvent was evaporated under reduced pressure, and the residue was purified by silica gel column chromatography (ethyl acetate:petroleum ether = 3:1) to obtain compound tert-butyl (1-(hydroxymethyl)piperidin-1-yl)-6-*p*-tosyl-1,6-dihydroimidazo[4,5-*d*]pyrrolo[2,3-*b*]pyridin-2-yl)c arbamate (1.14 g, 46% yield).
¹H NMR (400 MHz,CDCl₃) *δ* 8.54 (s,1H), 8.08-8.01 (m,2H), 7.79 (s,1H), 7.62 (d, *J =* 4.0 Hz, 1H), 7.29-7.22 (m, 2H), 6.95 (d, *J =* 4.0 Hz, 1H), 4.09-3.99 (m, 2H), 3.63-3.54 (m, 2H), 3.13-3.04 (m, 2H), 2.36 (s, 3H), 1.95-1.86 (m, 2H), 1.69 (s, 9H), 1.63-1.54 (m, 1H), 1.54-1.41 (m, 2H), 1.41-1.32 (m, 1H). LCMS ESI(+)m/z: 555.2 (M+1) .

### Step G:

Compound tert-butyl (1-(hydroxymethyl)piperidin-1-yl)-6-*p*-tosyl-1,6-dihydroimidazo[4,5-*d*]pyrrolo[2,3-*b*]pyridin-2-yl)c arbamate (1.10 g), DPPA (2.24 g) and DBU (2.48 g) were added to 50 mL toluene under ice-bath cooling and nitrogen protection. The mixture was heated to 100°C and stirred for 2 hours. The reaction solution was concentrated under reduced pressure, and the residue was purified by silica gel column chromatography (ethyl acetate) to obtain compound 1-(4-(methylazide)piperidin-1-yl)-6-*p*-tosyl-1,6-dihydroimidazo[4,5-*d*]pyrrolo[2,3-*b*]pyridin-2-amin e (647 mg, 68% yield).
¹H NMR (400 MHz, CDCl₃) *δ* 8.53 (s, 1H), 8.10 (d, *J* = 8.4 Hz, 2H), 7.76 (d, *J* = 4.1 Hz, 1H), 7.25 *(d, J =* 8.4 Hz, 2H), 6.64 (d, *J* = 4.1 Hz, 1H), 5.17 (s, 2H), 3.52 - 3.42 (m, 2H), 3.34 (d, *J =* 6.4 Hz, 2H), 3.20 - 3.12 (m, 2H), 2.35 (s, 3H), 2.04 - 1.97 (m, 2H), 1.67 - 1.54 (m, 3H). LCMS ESI(+)m/z: 466.2 (M+1) .

### Step H:

1-(4-(Methylazide)piperidin-1-yl)-6-*p*-tosyl-1,6-dihydroimidazo[4,5-*d*]pyrrolo[2,3-*b*]pyridin-2 -amine (525 mg) was dissolved in 30 mL dichloroethane, and di-tert-butyl dicarbonate (1.23 g), triethylamine (571 mg) and 4,4-dimethylaminopyridine (138 mg) were added. The mixture was stirred at room temperature for 1 hour. The reaction solution was concentrated under reduced pressure, and the residue was purified by silica gel column chromatography (petroleum ether:ethyl acetate = 4:1) to obtain compound tert-butyl (1-(4-(methylazide)piperidin-1-yl)-6-*p*-tosyl-1,6-dihydroimidazo[4,5-*d*]pyrrolo[2,3-*b*]pyridin-2-yl)( tert-butoxycarbonyl)carbamate (647 mg, 68% yield). LCMS ESI(+)m/z: 666.2(M+1).

### Step I:

Compound tert-butyl (1-(4-(methylazide)piperidin-1-yl)-6-*p*-tosyl-1,6-dihydroimidazo[4,5-*d*]pyrrolo[2,3-*b*]pyridin-2-yl)( tert-butoxycarbonyl)carbamate (235 mg) was dissolved in 25 mL methanol, and 10% palladium/carbon (40 mg) was added under nitrogen protection. The reaction system was purged with hydrogen and stirred under hydrogen atmosphere at room temperature for 16 hours. Then the solution was suction filtered and washed with 5 mL methanol. The filtrate was concentrated under reduced pressure to obtain crude product compound tert-butyl (1-(4-aminomethylpiperidin-1-yl)-6-*p*-tosyl-1,6-dihydroimidazo[4,5-*d*]pyrrolo[2,3-*b*]pyridin-2-yl)(t ert-butoxycarbonyl)carbamate (145 mg, 64% yield). LCMS ESI(+)m/z: 640.3(M+1).

### Step J:

Compound tert-butyl (1-(4-aminomethylpiperidin-1-yl)-6-*p*-tosyl-1,6-dihydroimidazo[4,5-*d*]pyrrolo[2,3-*b*]pyridin-2-yl)(t ert-butoxycarbonyl)carbamate (70 mg) was dissolved in 5 mL dichloromethane, and triethylamine (33 mg) and cyanomethylsulfonyl chloride (23 mg) were added successively under nitrogen protection at 0°C and stirred at 0°C for 2 hours. The reaction solution was purified by silica gel column chromatography (petroleum ether:ethyl acetate = 1:1) to obtain compound tert-butyl (tert-butoxycarbonyl)(1-(4-(((cyanomethyl)sulfonylamino)methyl)piperidin-1-yl)-6-*p*-tosyl-1,6-dih ydroimidazo[4,5-*d*]pyrrolo[2,3-*b*]pyridin-2-yl)carbamate (70 mg, 89% yield). LCMS ESI(+)m/z: 718.2(M+1).

### Step K:

Compound tert-butyl (tert-butoxycarbonyl)(1-(4-(((cyanomethyl)sulfonylamino)methyl)piperidin-1-yl)-6-*p*-tosyl-1,6-dih ydroimidazo[4,5-*d*]pyrrolo[2,3-*b*]pyridin-2-yl)carbamate (70 mg) was dissolved in 3 mL dichloromethane. 4 N hydrochloric acid in dioxane (2.5 mL) was added under nitrogen protection at 0°C and stirred at room temperature for 3 hours. The reaction solution was concentrated under reduced pressure to obtain crude product compound *N*-((1-(2-amino-6-silylimidazo[4,5-*d*]pyrrolo[2,3-*b*]pyridin-1(6*H*)-yl)-piperidin-4-yl)methyl)-1-cyan omethanesulfonamide, which is directly used in the next step.

### Step L:

Crude product compound *N*-((1-(2-amino-6-silylimidazo[4,5-*d*]pyrrolo[2,3-*b*]pyridin-1(6*H*)-yl)-piperidin-4-yl)methyl)-1-cyan omethanesulfonamide was suspended in 3 mL methanol, and 2 N sodium hydroxide solution (1 mL) was added and stirred at 35°C for 16 hours. 1 N HCl was used to adjust to pH = 7. Methanol was removed by evaporating under reduced pressure. The residue was purified by preparative thin-layer chromatography (methanol:dichloromethane = 1:10) to obtain *N*-((1-(2-aminoimidazo[4,5-*d*]pyrrolo[2,3-*b*]pyridin-1(6*H*)-yl)-piperidin-4-yl)methyl)-1-cyanometh anesulfonamide (2 mg, 5% yield).
¹H NMR (400 MHz, DMSO-*d*₆) *δ* 11.51 (s, 1H), 8.29-8.01 (m, 2H), 7.46-7.34 (m, 1H), 6.47 (dd, *J =* 3.3, 1.8 Hz, 1H), 6.24 (s, 2H), 4.81 (s, 2H), 3.53-3.42 (m, 2H), 3.11-2.97 (m, 4H), 2.04-1.95 (m, 1H), 1.94-1.87 (m, 2H), 1.60-1.49 (m, 2H). LCMS ESI(+)m/z: 389.0 ( M+1 ) .

### Example 15: Synthesis of Compound B3

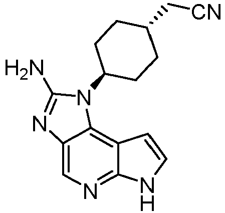

### 2-((1R,4R)-4-(2-aminoimidazo[4,5-d]pyrrolo[2,3-b]pyridin-1(6H)-ethyl)cyclohexyl)acetonitrile

The specific synthesis steps are as follows:

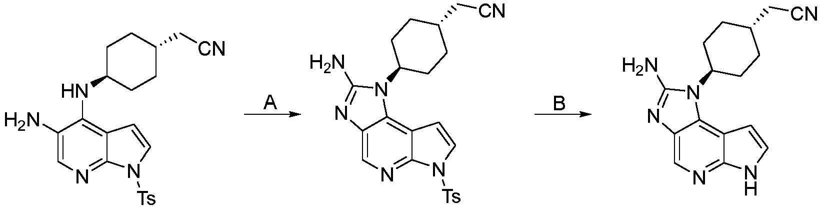

### Step A:

Compound *N⁴*-(piperidin-1-yl)-1-p-toluenesulfonyl-1*H*-pyrrolo[2,3-*b*]pyridin-4,5-diamine (700 mg) was suspended in methanol (100 mL), and cyanogen bromide (199 mg) was added to the above solution at room temperature under nitrogen protection. The reaction system was then stirred at room temperature for 16 hours. The solvent was evaporated under reduced pressure, and saturated sodium bicarbonate solution (10 mL), saturated sodium thiosulfate solution (10 mL) and a mixed solvent of methanol and ethyl acetate (1:10 of volume ratio, 40 mL) were added. Then the reaction system was stirred at room temperature for 15 minutes. The organic phase was separated and the aqueous phase was extracted 3 times with a mixed solvent of methanol and ethyl acetate (20 mL). The organic phases were combined, washed with saturated brine (10 mL), dried with anhydrous sodium sulfate, and then the solvent was evaporated under reduced pressure to obtain a crude product of compound 2-((1*R*,4*R*)-4-(2-amino-6-(benzenesulfonyl)imidazo[4,5-*d*]pyrrolo[4,5-*b*]pyidin-1-(6*H*)-ethyl)cyclo hexyl)acetonitrile (740 mg, 100% of yield). LCMS ESI(+)m/z: 435.2 (M+1).

### Step B:

2-((1*R*,4*R*)-4-(2-Amino-6-(benzenesulfonyl)imidazo[4,5-*d*]pyrrolo[4,5-*b*]pyridin-1-(6*H*-ethyl) cyclohexyl)acetonitrile (100 mg) was suspended in 6 mL methanol, and 2 N sodium hydroxide solution (1.5 mL) was added to the above solution. The reaction system was then stirred at room temperature for 16 hours, and adjusted to pH=7 with 1 N HCl. Methanol was evaporated under reduced pressure and reversed phase HPLC was performed to obtain the compound 2-((1*R*,4*R*)-4-(2-aminoimidazo[4,5-*d*]pyrrolo[2,3-*b*]pyridin-1(6*H*)-ethyl)cyclohexyl)acetonitrile (35 mg, 52% of yield). ¹H NMR (400 MHz, DMSO-*d*₆) *δ* 11.47 (s, 1H), 8.13 (s, 1H), 7.44-7.26 (m, 1H), 6.55 (dd, *J* = 3.3*,* 1.6 Hz, 1H), 6.21 (s, 2H), 4.49-4.26 (m, 1H), 2.59 (d, *J =* 5.8 Hz, 2H), 2.40-2.25 (m, 2H), 2.06-1.92 (m, 3H), 1.89-1.79 (m, 2H), 1.47-1.31 (m, 2H). LCMS ESI(+)m/z:295.2(M+1).

### Example 16: Synthesis of Compound B4

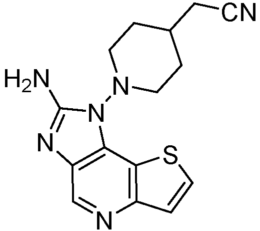

### 2-(1-(2-amino-1H-imidazo[4,5-d]thieno[3,2-b]pyridin-1-yl)piperidin-4-yl)acetonitrile

The specific synthesis method is as follows:

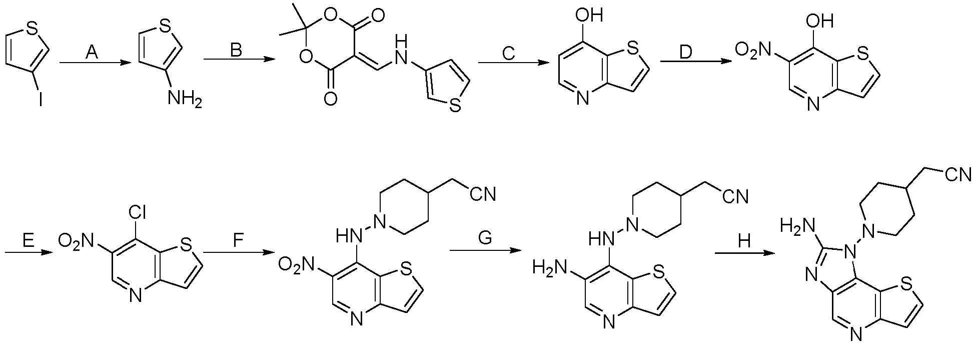

### Step A:

CuI (3.6 g), L-proline (4.4 g), potassium carbonate (39.6 g), 3-iodothiophene (20 g), and concentrated ammonia (8.7 g) were added successively to a 250 mL three-necked flask. The reaction system was stirred at room temperature under nitrogen for 24 hours. The reaction solution was filtered, and the filtrate was extracted with dichloromethane. Then the organic phases were combined, dried with anhydrous magnesium sulfate, and spin-dried. The resulting residue was purified by silica gel column chromatography (ethyl acetate and petroleum ether = 15%) to obtain a compound 3-aminothiophene (9 g, 95%).

### Step B:

2,2-Dimethyl-1,3-dioxan-4,6-dione (14.4g) was dissolved in 200 mL of isopropanol, then triethyl orthoformate (45 mL, 400 mmol) was added to the above solution. The reaction system was stirred at 100°C for 1 hour, and then cooled to room temperature to precipitate a white solid, which was directly put into the next step. The compound 3-aminothiophene (10 g) and isopropanol were added to the reaction system. Then the reaction system was refluxed for half an hour, cooled to room temperature to precipitate a plurality of white solid, and filtered to obtain a compound 2,2-dimethyl-5-((thiophen-3-ylamino)methylene)-1,3-dioxan-4,6-dione (3 g, 12% of yield). ¹H NMR (400 MHz, DMSO-*d*₆) *δ* 11.38 (d, *J =* 14.7 Hz, 1H), 8.49 (d, *J =* 14.7 Hz, 1H), 7.69 (dd, *J =* 3.2, 1.4 Hz, 1H), 7.62 (dd, *J =* 5.2, 3.2 Hz, 1H), 7.44 (dd, J = 5.2, 1.5 Hz, 1H), 1.66 (s, 6H). LCMS ESI(+)m/z:196.0 (M+1-58).

### Step C:

Diphenyl ether was added to a 50 mL round-bottom flask, the reaction solvent was heated to 250°C, and the compound 2,2-dimethyl-5-((thiophen-3-ylamino)methylene)-1,3-dioxan-4,6-dione (3.00 g) was added to the system. The system was reacted at 250°C for 30 minutes, cooled to room temperature, and filtered to obtain a white solid compound thieno[3,2-b]pyridin-7-ol (1.2 g, 68% of yield). LCMS ESI(+)m/z:152.0(M+1).

### Step D:

The compound thieno[3,2-b]pyridin-7-ol (900 mg) was dissolved in propionic acid (20 mL). The reaction system was heated to 110°C under nitrogen atmosphere, and then 4.8 mL of fuming nitric acid was added. The reaction system was reacted at 150°C for 1 hour, and then cooled to room temperature. Then diethyl ether was added to the reaction system, and the reaction system was filtered to obtain a solid. The obtained solid was washed with water and dried under reduced pressure to obtain a compound 6-nitrothieno[3,2-b]pyridin-7-ol (700 mg, 60% of yield). LCMS ESI(+)m/z: 197.0(M+1).

### Step E:

The compound 6-nitrothieno[3,2-*b*]pyridin-7-ol (600 mg) was added to phosphorus oxychloride (25 mL). The reaction system was heated to 110°C under nitrogen atmosphere and reacted for 1 hour, and the solvent was evaporated under reduced pressure. Then dichloromethane and saturated sodium bicarbonate solution were added, and the organic phases were separated, combined, washed three times with water, dried with anhydrous sodium sulfate, and filtered. The filtered solvent was evaporated under reduced pressure to obtain a compound 7-chloro-6-nitrothieno[3,2-*b*]pyridine (483 mg, 75% yield). LCMS ESI(+)m/z: 215.0(M+1).

### Step F:

The compound 7-chloro-6-nitrothieno[3,2-*b*]pyridine (480 mg) was dissolved in isopropanol (15 mL), and 2-(1-aminopiperidin-4-yl)acetonitrile (471 mg) and diisopropylethylamine (1 mL) were added. The reaction system was reacted at 90°C for 2 hours, and then cooled to room temperature to precipitate a plurality of solid, which was filtered to obtain a yellow solid and then dried under reduced pressure to obtain a compound 2-(1-((6-nitrothieno[3,2-*b*]pyridin-7-yl)amino)piperidin-4-yl)acetonitrile (600 mg, 85% of yield). ¹H NMR (400 MHz, CDCl₃) *δ* 9.30 (s, 1H), 9.24 (d, *J* = 8.5 Hz, 1H), 7.86 (d, *J =* 5.5 Hz, 1H), 7.51 (d, *J* = 5.5 Hz, 1H), 4.21 (m, *J =* 14.9, 7.3 Hz, 1H), 2.39 (d, *J =* 6.2 Hz, 2H), 2.05 (d, *J =* 12.9 Hz, 2H), 1.91-1.73 (m, 1H), 1.52 (ddd, *J =* 41.0, 19.4, 8.4 Hz,5H).

### Step G:

The compound 2-(1-((6-nitrothieno[3,2-*b*]pyridin-7-yl)amino)piperidin-4-yl)acetonitrile (200 mg) was dissolved in ethanol (100 mL), and saturated ammonium chloride solution and Fe (353 mg) were added to the above solution. The reaction system was reacted at 75°C for 30 minutes under nitrogen condition. After the reaction was completed, the system was filtered while hot, and the filtrate was concentrated. The resulting residue was purified by silica gel column chromatography (methanol: dichloromethane = 1:10) to obtain a compound 2-(1-((6-aminothieno[3,2-*b*]pyridin-7-yl)amino)piperidin-4-yl)acetonitrile (180 mg, 98% of yield). LCMS ESI(+)m/z: 288.2(M+1).

### Step H:

The compound 2-(1-((6-aminothieno[3,2-*b*]pyridin-7-yl)amino)piperidin-4-yl)acetonitrile (72 mg) was dissolved in methanol (5 mL) and cyanogen bromide (53 mg) was added to the above solution. The reaction system was stirred at room temperature under nitrogen atmosphere for 16 hours. The solvent was evaporated under reduced pressure, and saturated sodium bicarbonate solution (5 mL), saturated sodium thiosulfate solution (5 mL) and a mixed solvent of methanol and ethyl acetate (1:10 of volume ratio, 10 mL) were added and stirred at room temperature for 15 minutes. The organic phase was separated and the aqueous phase was extracted 3 times with a mixed solvent of methanol and ethyl acetate (10 mL). The organic phases were combined, washed with saturated brine (10 mL), and dried with anhydrous sodium sulfate, and then the solvent was evaporated under reduced pressure. The resulting residue was purified by silica gel column chromatography (methanol: dichloromethane = 1:10) to obtain the product 2-(1-(2-amino-1*H*-imidazo[4,5-*d*]thieno[3,2-b]pyridin-1-yl)piperidin-4-yl)acetonitrile (11 mg,15% of yield). ¹H NMR (400 MHz, DMSO-*d*₆) *δ* 8.52 (s, 1H), 7.78 (d, *J =* 5.5 Hz, 1H), 7.51 (d, *J =* 5.5 Hz, 1H), 6.70 (s, 2H), 4.59-4.33 (m, 1H), 2.61 (d, *J =* 5.8 Hz, 2H), 2.44-2.32 (m, 2H), 2.00 (d, *J =* 11.8 Hz, 2H), 1.92 (d, *J =* 10.9 Hz, 2H), 1.48-1.32 (m, 2H). LCMS ESI(+)m/z: 313.2 (M+1).

### Example 17: Synthesis of Compound B5

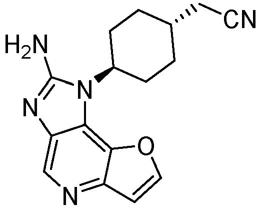

### 2-((1R,4R)-4-(2-amino-1H-furo[3,2-b]imidazo[4,5-d]pyridin-1-yl)cyclohexyl)acetonitrile

The specific synthesis steps are as follows:

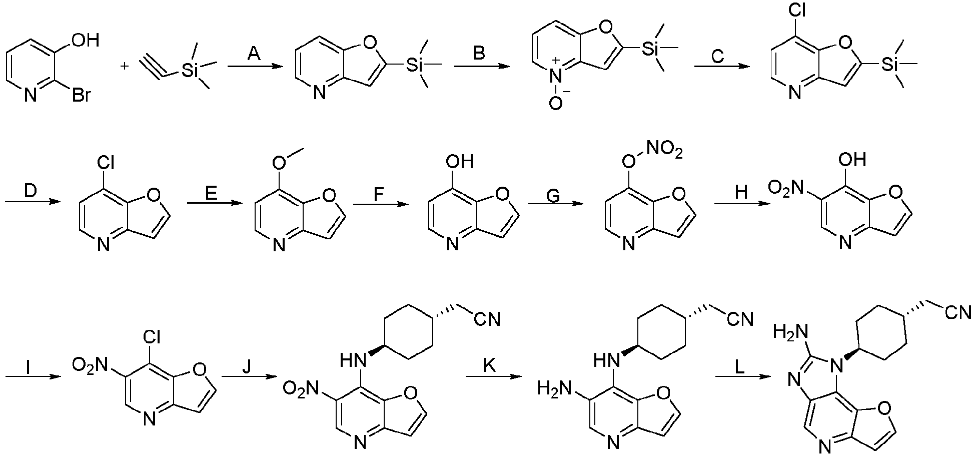

### Step A:

2-Bromo-3-hydroxypyridine (25 g), trimethylethynylsilicon (17 g), cuprous iodide (2.74 g, 14.4 mmol), bis(triphenylphosphine)dichloropalladium (2.0 g) and 1,4-dioxane (200 mL) were added to a dry and clean 500 mL single-necked flask. The system was purged with nitrogen 3 times and stirred at room temperature for 30 minutes. Then triethylamine (43.6 g) was added to the system, and the reaction system was reacted at 65°C under nitrogen protection for 6 hours, and then cooled to room temperature and suction filtered under reduced pressure. The filter cake was washed with 30 mL of methyl tert-butyl ether, and the filtrate was washed with 400 mL of water. Then the aqueous phase was extracted twice with 400 mL methyl tert-butyl ether, the organic phases were combined and then washed with 1 L of saturated brine, dried with anhydrous sodium sulfate, and filtered. The filtrate was spin-dried to obtain a crude product, and 200 mL of petroleum ether was added to the obtained crude product, pulped for 30 minutes, and filtered. The filter cake was washed twice with 30 mL of petroleum ether, and the filtrate was spin-dried to obtain a compound 2-(trimethylsilyl)furo[3,2-b]pyridine (23.3 g, 85% of yield). ¹H NMR (400 MHz, CDCl₃) *δ* 8.53 (dd, *J =* 4.7, 1.1 Hz, 1H), 7.75 (dt, *J* = 8.3, 1.1 Hz, 1H), 7.19 (dd, *J =* 8.3, 4.7 Hz, 1H), 7.15 *(d, J* = 1.0 Hz, 1H), 0.38 (s, 9H).

### Step B:

The compound 2-(trimethylsilyl)furo[3,2-b]pyridine (36.6 g) and dichloromethane (400 mL) were added to a dry and clean 1 L single-necked flask. At room temperature, m-chlorperoxybenzoic acid (70%) (76.9 g) was added to the above solution, the reaction system was reacted overnight at room temperature. After the reaction was completed, the system was filtered. The filter cake was washed twice with 20 mL of dichloromethane. The filtrate was combined, and then washed with saturated sodium bicarbonate solution and saturated brine respectively, dried with anhydrous sodium sulfate, and filtered. The filtrate was spin-dried to obtain a compound 2-(trimethylsilyl)furo[3,2-b]pyridin4-oxide (41.4 g, crude product). ¹H NMR (400 MHz, CDCl₃) *δ* 8.18 (d, *J =* 6.3 Hz, 1H), 7.45 (d, *J =* 8.4 Hz, 1H), 7.40 (d, *J* = 0.7 Hz, 1H), 7.15 (dd, *J =* 8.4, 6.3 Hz, 1H), 0.38 (s, 9H).

### Step C:

The crude product 2-(trimethylsilyl)furo[3,2-b]pyridine-4-oxide (41.4 g) and toluene (150 mL) were added to a dry and clean 500 mL single-necked flask, and 150 mL of phosphorus oxychloride was slowly added dropwise to the above solution at 0°C. After dropping, the system was heated to 95°C and reacted for 3 hours. After the reaction was completed, the system was cooled to room temperature, spin-dried, and the resulting residue was diluted with 300 mL of water, and the above solution was adjusted to pH=8 with saturated sodium bicarbonate aqueous solution. The system was extracted 3 times with 500 mL of methyl tert-butyl ether. The filtrate was combined, and then washed with saturated brine, dried with anhydrous sodium sulfate, and filtered. The filtrate was spin-dried to obtain a crude product. The crude product was purified by a silica gel column (eluent: ethyl acetate: petroleum ether =1:10) to obtain a compound 2-(trimethylsilyl)-7-chlorofuro[3,2-b]pyridine (20.7 g, 46% of yield). ¹H NMR (400 MHz, CDCl₃) *δ* 8.40 (d, *J =* 5.2 Hz, 1H), 7.21 (d, *J =* 5.2 Hz, 1H), 7.17 (s, 1H), 0.40 (s, 9H).

### Step D:

The compound 2-(trimethylsilyl)-7-chlorofuro[3,2-b]pyridine (20.7 g) and tetrahydrofuran (150 mL) were added to a dry and clean 500 mL single-necked flask, 150 mL of sodium hydroxide (18 g) aqueous solution was added to the above solution at room temperature. After addition, the system was heated to 50°C and reacted for 1 hour. After the reaction was completed, the system was cooled to room temperature, spin-dried to remove solvent, and the resulting aqueous solution was extracted 3 times with 200 mL of methyl tert-butyl ether. The filtrate was combined, and then washed with saturated brine, dried with anhydrous sodium sulfate, and filtered. The filtrate was spin-dried to obtain a compound 7-chlorofuro[3,2-*b*]pyridine (11.8 g, 84% of yield). ¹H NMR (400 MHz, CDCl₃) *δ* 8.46 (d, *J* = 5.2 Hz, 1H), 7.92 (d, *J* = 2.3 Hz, 1H), 7.28 (d, *J* = 5.2 Hz, 1H), 7.05 (d, *J* = 2.3 Hz, 1H). Step E:
A compound 5 (10.8 g) and methanol (200 mL) were added to a dry and clean 500 mL single-necked flask, and 100 mL of sodium hydroxide (42.6 g) aqueous solution was added to the above solution at room temperature. After addition, the system was heated to 75°C and reacted overnight. After the reaction was completed, the system was cooled to room temperature, spin-dried to remove solvent, and the resulting solution was diluted with 100 mL of water, and extracted 2 times with 200 mL of dichloromethane. The filtrate was combined, and then washed with saturated brine, dried with anhydrous sodium sulfate, and filtered. The filtrate was spin-dried to obtain a compound 7-methoxyfuro[3,2-b]pyridine (5.6 g, 53.3% of yield). ¹H NMR (400 MHz, CDCl₃) *δ* 8.43 (s, 1H), 7.80 (d, *J* = 2.2 Hz, 1H), 6.97 (d, *J* = 2.2 Hz, 1H), 6.76 (d, *J* = 5.5 Hz, 1H), 4.08 (s, 3H).

### Step F:

The compound 7-methoxyfuro[3,2-*b*]pyridine (5.0 g), hydrobromic acid in acetic acid solution (10 mL) and water (4 mL) were added to a dry and clean 25 mL single-necked flask, then the reaction system was reacted at 125°C overnight. After the reaction was completed, the system was cooled to room temperature, spin-dried to remove solvent, and the resulting residue was dissolved with 50 mL of toluene, and then spin-dried again. These steps were repeated for 3 times to obtain 7.2 g of a crude product. The crude product was purified by C18 reverse phase column chromatography with an eluent of methanol: water (0.1% ammonia water) system, and eluted with 100% water to obtain a compound 7-hydroxyfuro[3,2-b]pyridine (3.1 g, 46.5% of yield). ¹H NMR (400 MHz, DMSO-*d*₆) *δ* 8.60 (d, *J =* 2.2 Hz, 1H), 8.54 (d, *J =* 6.9 Hz, 1H), 7.31 (d, *J* = 2.2 Hz, 1H), 7.14 (d, *J =* 6.8 Hz, 1H). Step G:
The compound 7-hydroxyfuro[3,2-*b*]pyridine (1.5 g) and dichloromethane (15 mL) were added to a dry and clean 50 mL single-necked flask, and tetrabutylammonium nitrate (10 g) and trifluoroacetic anhydride (11.6 g) were added to the above solution at 0°C. The reaction system was reacted overnight at room temperature. After the reaction was completed, the system was diluted with 50 mL of water, and extracted twice with 50 mL of ethyl acetate. The filtrate was combined, and then washed with saturated sodium bicarbonate aqueous solution and saturated brine respectively, dried with anhydrous sodium sulfate, and filtered. The filtrate was spin-dried to obtain a crude product, which was separated with a normal phase column chromatography (ethyl acetate: petroleum ether = 50%) to obtain a compound furo[3,2-b]pyridin-7-yl nitrate (540 mg, 27% of yield). ¹H NMR (400 MHz, CDCl₃) *δ* 8.59 (d, *J =* 8.7 Hz, 1H), 7.83 (d, *J =* 2.1 Hz, 1H), 7.37 (d, *J* = 2.1 Hz, 1H), 6.43 (d, *J* = 8.7 Hz, 1H).

### Step H:

The compound furo[3,2-b]pyridin-7-yl nitrate (430 mg) and propionic acid (15 mL) were added to a dry and clean 50 mL single-necked flask, and fuming nitric acid (551 mg) was added to the above solution at room temperature. Then the reaction system was reacted at 125°C for 30 minutes. After the reaction was completed, the system was cooled to room temperature, diluted by 50 mL of methyl tert-butyl ether, and then filtered. The resulting solid was washed twice with 3 mL of methyl tert-butyl ether to obtain a compound 6-nitrofuro[3,2-*b*]pyridin-7-ol (220 mg, 37.5% of yield). ¹H NMR (400 MHz, DMSO-*d*₆) *δ* 13.12 (s, 1H), 9.01 (s, 1H), 8.22 (d, *J =* 2.1 Hz, 1H), 6.95 (d, *J* = 2.1 Hz, 1H).

### Step I:

The compound 6-nitrofuro[3,2-*b*]pyridin-7-ol (356 mg) and DCE (12 mL) were added to a dry and clean 25 mL single-necked flask, and phosphorus oxychloride (4.2 mL) was added to the above solution at room temperature. Then the reaction system was reacted at 95°C for 2 hours. After the reaction was completed, the system was cooled to room temperature, spin-dried to remove solvent, diluted with an addition of 10 mL of water, and adjusted to pH=7 to 8 with saturated sodium bicarbonate aqueous solution. The resulting aqueous phase was extracted 3 times with 50 mL of methyl tert-butyl ether. The filtrate was combined, and then washed with saturated brine, dried with anhydrous sodium sulfate, and filtered. The filtrate was spin-dried to obtain a yellow solid compound 7-chloro-6-nitrofuro[3,2-*b*]pyridine (287 mg, 73.2% of yield). ¹H NMR (400 MHz, CDCl₃) *δ* 9.22 (s, 1H), 8.16 (d, *J* = 2.2 Hz, 1H), 7.17 (d, *J =* 2.2 Hz, 1H).

### Step J:

The compound 7-chloro-6-nitrofuro[3,2-*b*]pyridine (70 mg), 2-((1R,4R)-4-aminocyclohexyl)acetonitrile (74.4 mg) and tert-butanol (5 mL) were added to a dry and clean 10 mL single-necked flask, and DIPEA (100 mg) was added to the above solution at room temperature, and then the reaction system was reacted overnight at 135°C. After the reaction was completed, the reaction solution was spin-dried to obtain a crude product, and the crude product was separated with a normal phase column chromatography (ethyl acetate: petroleum ether = 50%) to obtain a yellow solid compound 2-((1*R*,4*R*)-4-((6-nitrofuro[3,2-*b*]pyridin-7-yl)amino)cyclohexyl)acetonitrile (55 mg, 51.8% of yield). ¹H NMR (400 MHz, DMSO-*d*₆) *δ* 9.05 (s, 1H), 8.49-8.39 (m, 2H), 7.14 (d, *J =* 2.2 Hz, 1H), 4.33-4.21 (m, 1H), 2.53 (d, *J =* 6.4 Hz, 2H), 2.11 (d, *J =* 9.7 Hz, 2H), 1.87 (d, *J =* 11.9 Hz, 2H), 1.74-1.64 (m, 1H), 1.61-1.49 (m, 2H), 1.33-1.25 (m, 2H).

### Step K:

The compound 2-((1*R*,4*R*)-4-((6-nitrofuro[3,2-*b*]pyridin-7-yl)amino)cyclohexyl)acetonitrile (55 mg) and methanol (10 mL) were added to a dry and clean 50 mL single-necked flask, palladium carbon (25 mg, 60% content) was added to the above solution at room temperature, and then the reaction system was purged with hydrogen for 3 times and reacted under one hydrogen balloon pressure for 30 minutes. After the reaction was completed, the reaction solution was filtered through diatomite. Then the filter cake was washed with 3 mL of methanol. The filtrate was combined and spin-dried to obtain a compound 2-((1*R*,4*R*)-4-((6-aminofuro[3,2-*b*]pyridin-7-yl)amino)cyclohexyl)acetonitrile (43 mg, 86.5% of yield). ¹H NMR (400 MHz, DMSO-*d*₆) *δ* 7.87 (d, *J =* 2.3 Hz, 1H), 7.75 (s, 1H), 6.73 (d, *J =* 2.3 Hz, 1H), 4.91 (d, *J =* 9.1 Hz, 1H), 4.59 (s, 2H), 3.97-3.85 (m, 1H), 2.00 (d, *J =* 9.4 Hz, 2H), 1.83 (d, *J =* 12.1 Hz, 2H), 1.67-1.59 (m, 1H), 1.37-1.16 (m, 6H).

### Step L:

The compound 2-((1*R*,4*R*)-4-((6-aminofuro[3,2-*b*]pyridin-7-yl)amino)cyclohexyl)acetonitrile (33 mg) and methanol (5 mL) were added to a dry and clean 25 mL bottle, cyanogen bromide (14.3 mg) was added to the above solution at room temperature, and then the reaction system was reacted overnight at room temperature. After the reaction was completed, 20 mL of water was added to the reaction solution. The aqueous phase was extracted with 30 mL of dichloromethane for 3 times respectively, and then the filtrate was combined, washed with saturated brine, dried with anhydrous sodium sulfate, and filtered. The filtrate was spin-dried to obtain 40 mg of crude product, and the crude product was prepared by high-pressure liquid phase chromatography (0.05% ammonia water system) to obtain a white solid compound 2-((1R,4R)-4-(2-amino-1H-furo[3,2-b]imidazo[4,5-d]pyridin-1-yl)cyclohexyl)acetonitrile (3.8 mg, 10.5% of yield). ¹H NMR (400 MHz, CDCl₃) *δ* 8.72 (s, 1H), 7.80 (d, *J =* 2.2 Hz, 1H), 7.09 (d, *J =* 2.2 Hz, 1H), 4.84 (s, 2H), 4.17-4.03 (m, 1H), 2.54-2.44 (m, 2H), 2.43 (d, *J* = 6.1 Hz, 2H), 2.16-2.02 (m, 5H), 1.47 (d, *J =* 12.8 Hz, 2H). ESI(+)m/z:296.7 (M+1).

### Example 18: Synthesis of Compound B6

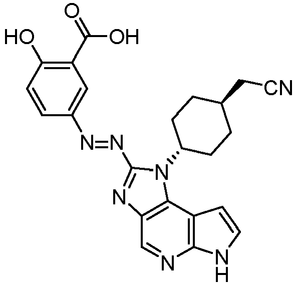

### 5-((E)-(1-((1R,4R)-4-(cyanomethyl)cyclohexyl)-1,6-dihydroimidazo[4,5-d]pyrrolo[2,3-b]pyridin-2-yl)diazenyl)-2-hydroxybenzoic acid

The specific synthesis steps are as follows:

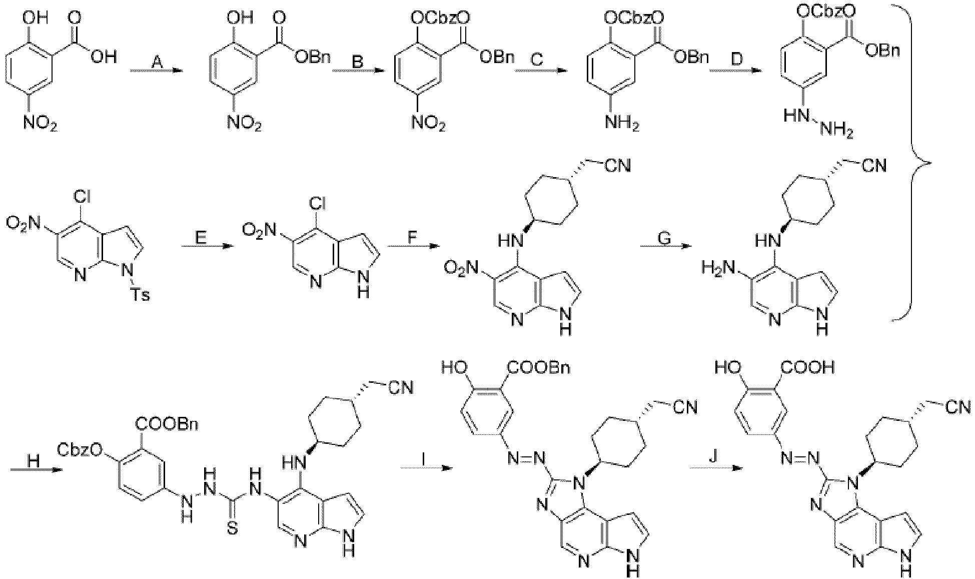

### Step A:

5-Nitrosalicylic acid (5.00 g) was dissolved in 60 mL of DMF, and KHCO₃ (3.01 g) and benzyl bromide (5.14 g) were added to the above solution at room temperature. The reaction system was then stirred at room temperature for 16 hours, and concentrated under reduced pressure. The above solution was poured into water (50 mL), and ethyl acetate (100 mL) was added to separate the organic phase. The resultant was extracted with ethyl acetate (30 mL) for 2 times. The organic phases were combined, washed with saturated brine (10 mL), dried with anhydrous sodium sulfate, suction filtered, concentrated under reduced pressure, and purified by silica gel column chromatography (1: 9 of ethyl acetate : petroleum ether) to obtain a compound benzyl 2-hydroxy-5-nitrobenzoate (5.84 g, 78% of yield). ¹H NMR (400 MHz, DMSO-*d*₆) *δ* 11.60 (s,1H), 8.54 (d, *J* = 2.9 Hz,1H), 8.33 (dd,*J* = 9.2, 3.0 Hz,1H), 7.51 (dd,*J* = 7.7, 1.1 Hz,2H), 7.47-7.34 (m,3H), 7.18 (d,*J* = 9.2 Hz, 1H),5.40 (s,2H).

### Step B:

Benzyl 2-hydroxy-5-nitrobenzoate (5.84 g) was dissolved in dichloromethane (100 mL), and triethylamine (3.24 g) and benzyl chloroformate (4.38 g) were added successively under an ice bath and nitrogen protection. The reaction system was stirred at room temperature under nitrogen protection for 16 hours. The reaction solution was concentrated under reduced pressure, and then purified by silica gel column chromatography (1: 10 of ethyl acetate : petroleum ether) to obtain a compound benzyl 2-((benzyloxycarbonyl)oxy)-5-nitrobenzoate (6.56 g, 75% of yield). ¹H NMR (400 MHz, CDCl₃) *δ* 8.89 (d, *J =* 2.8 Hz,1H), 8.41 (dd, *J =* 8.9, 2.8 Hz, 1H), 7.46-7.31 (m, 11H), 5.30 (s,2H), 5.15 (s,2H).

### Step C:

The compound benzyl 2-((benzyloxycarbonyl)oxy)-5-nitrobenzoate (1.20 g) was dissolved in ethanol (10 mL) and acetonitrile (10 mL), and water (0.5 mL) and stannous chloride dihydrate (3.32g) were added to the above solution at room temperature. The reaction system was stirred at 80°C for 2 hours. The reaction solution was cooled to room temperature, adjusted to alkaline with saturated sodium bicarbonate aqueous solution, and stirred for 15 minutes. Ethyl acetate (50 mL) was added to the reaction solution, and suction filtered with diatomite, and the filter cake was washed with ethyl acetate (50 mL). The organic phases were separated, and extracted twice with ethyl acetate (20 mL). Then the organic phases were combined, washed with saturated brine (10 mL), dried with anhydrous sodium sulfate, suction filtered, concentrated under reduced pressure, and purified by silica gel column chromatography (1: 4 of ethyl acetate : petroleum ether) to obtain a compound benzyl 2-((benzyloxycarbonyl)oxy)-5-aminobenzoate (662 mg, 60% of yield). ¹H NMR (400 MHz, DMSO-*d*₆) *δ* 7.43-7.33 (m, 11H), 7.08-7.02 (m, 2H), 6.99 (dd, *J =* 8.8, 2.8 Hz, 1H), 5.22 (s, 2H), 5.10 (s, 2H), 4.11 (s, 2H). LCMS ESI(+)m/z:378.2(M+1).

### Step D:

The compound benzyl 2-((benzyloxycarbonyl)oxy)-5-aminobenzoate (2.00 g) was dissolved in acetonitrile (20 mL), the reaction solution temperature was cooled to -10°C, and then concentrated hydrochloric acid (3 mL) and sodium nitrite (366 mg) in water (0.6 mL) were added to the reaction solution. The reaction system was stirred for 15 minutes. Then stannous chloride dihydrate (3.59 g) was added to the reaction system and stirred for 1 hour. The reaction system was adjusted to pH=8 with 2 N sodium hydroxide aqueous solution. Ethyl acetate (100 mL) was added to the above solution, and suction filtered with diatomite, and the filter cake was washed with ethyl acetate (100 mL). The organic phases were separated, and extracted twice with ethyl acetate (20 mL). Then the organic phases were combined, washed with saturated brine (10 mL), dried with anhydrous sodium sulfate, suction filtered, concentrated under reduced pressure, and purified by silica gel column chromatography (1:1 of ethyl acetate : petroleum ether) to obtain a compound benzyl 2-((benzyloxycarbonyl)oxy)-5-hydrazinylbenzoate (1.3 g, 63% of yield). ¹H NMR (400 MHz, DMSO-*d*₆) *δ* 7.43-7.33 (m, 11H), 7.08-7.02 (m, 2H), 6.99 (dd, *J =* 8.8, 2.8 Hz, 1H), 5.22 (s, 2H), 5.10 (s, 2H), 4.11 (s, 2H). LCMS ESI(+)m/z:393.2(M+1).

### Step E:

4-Chloro-5-nitro-1-(p-toluenesulfonyl)-1*H*-pyrrolo[2,3-*b*]pyridine (5.15 g) was suspended in tert-butanol (80 mL), and 1 N sodium hydroxide aqueous solution (38.1 mL) was added to the above solution. The reaction system was stirred at room temperature for 16 hours. Water (200 mL) and ethyl acetate (300 mL) were added to the above solution, and the organic phases were separated, and extracted twice with ethyl acetate (200 mL). Then the organic phases were combined, washed with saturated brine (50 mL), dried with anhydrous sodium sulfate, suction filtered, and concentrated under reduced pressure to obtain a compound 4-chloro-5-nitro-1H-pyrrolo[2,3-*b*]pyridine (3.01 g, 100% of yield). ¹H NMR (400 MHz, CDCl₃) *δ* 9.02 (s, 1H), 7.72 (d, *J =* 4.1 Hz, 1H), 7.11-7.02 (m, 2H), 6.77-6.66 (m, 2H), 6.61 (d, *J =* 4.1 Hz, 1H), 6.42-6.34 (m, 1H), 3.71-3.51 (m, 3H), 3.45-3.36 (m, 1H), 3.16-3.09 (m, 1H), 3.00-2.91 (m, 1H), 2.36 (d, *J* = 6.7 Hz, 2H), 2.07-1.99 (m, 1H), 1.98-1.82 (m, 2H), 1.79 (d,*J* = 6.7 Hz,3H), 1.75-1.63 (m, 11H), 1.36-1.23 (m, 1H).

### Step F:

The compound 4-chloro-5-nitro-1*H*-pyrrolo[2,3-*b*]pyridine (3.22 g) was suspended in 100 mL isopropyl alcohol, and *N*,*N*-diisopropylethylamine (6.32 mL) and 2-((1*R*,4*R*)-4-aminocyclohexyl)acetonitrile hydrochloride (3.70 g) were added successively to the above solution. The reaction system was heated to 90°C under nitrogen protection and stirred for 16 hours. Then the reaction system was cooled to room temperature, suction filtered under reduced pressure, and washed with 20 mL of isopropanol. The filter cake was dried under reduced pressure to obtain a compound 2-((1*R*,4*R*)-4-((5-nitro-1*H*-pyrrolo[2,3-*b*]pyridin-4-yl)amino)cyclohexyl)acetonitrile (4.88 g, 100% of yield). ¹H NMR (400 MHz, DMSO-*d*₆) *δ* 12.18 (s, 1H), 9.01 (d, *J =* 8.0 Hz, 1H), 8.86 (s, 1H), 7.98 (s, 1H), 7.33 (d, *J =* 3.2 Hz, 1H), 6.71 (d, *J =* 3.5 Hz, 1H), 4.13-4.01 (m, 1H), 2.54-2.51 (m, 2H), 2.21-2.12 (m, 2H), 1.91-1.82 (m, 2H), 1.76-1.65 (m, 1H), 1.56-1.45 (m, 2H), 1.42-1.30 (m, 2H).

### Step G:

2-((1*R*,4*R*)-4-((5-nitro-1*H*-pyrrolo[2,3-*b*]pyridin-4-yl)amino)cyclohexyl)acetonitrile (4.98 g) was added to 200 mL of ethanol, and iron powder (18.6 g) and saturated ammonium chloride (19 mL) were added successively to the above solution. The reaction system was stirred at 75°C for 40 minutes, suction filtered with diatomite while still hot, and washed with 40 mL of ethanol. The filtrate was concentrated and purified by silica gel column chromatography (1: 9 of methanol : dichloromethane) to obtain a compound 2-((1*R*,4*R*)-4-((5-amino-1*H*-pyrrolo[2,3-*b*]pyridin-4-yl)amino)cyclohexyl)acetonitrile (4.33 mg, 97% of yield). ¹H NMR (400 MHz, DMSO-*d*₆) *δ* 11.99 (s, 1H), 8.10 (s, 1H), 7.54 (s, 1H), 7.26 (d, *J=* 3.6 Hz, 1H), 6.70 (d, *J =* 8.0 Hz, 1H), 6.60 (d, *J =* 3.7 Hz, 1H), 4.06-3.91 (m, 1H), 3.34 (s, 2H), 2.50-2.41 (m, 2H), 2.14-2.02 (m, 2H), 1.86 (d, *J* = 11.6 Hz, 2H), 1.75-1.61 (m, 1H), 1.59-1.46 (m, 2H), 1.40-1.28 (m, 2H). LCMS ESI(+)m/z:270.2(M+1).

### Step H:

Thiocarbonyldiimidazole (744 mg) was dissolved in THF (50 mL), and compound 5 (1.64 g) in THF (10 mL) was added to the above solution at room temperature. The reaction system was stirred at room temperature for 15 minutes under nitrogen protection. The compound 2-((1*R*,4*R*)-4-((5-amino-1*H*-pyrrolo[2,3-*b*]pyridin-4-yl)amino)cyclohexyl)acetonitrile (865 mg) in DMF (30 mL) and triethylamine (422 mg) were added to the above solution. The reaction system was reacted at 60°C for 2.5 hours under nitrogen protection, concentrated under reduced pressure, and purified by silica gel column chromatography (1: 19 of methanol : dichloromethane) to obtain a compound benzyl 2-((benzyloxycarbonyl)oxy)-5-(2-((4-(((1*R*,4*R*)-4-(cyanomethyl)cyclohexyl)amino)-1*H*-pyrrolo[2,3 -*b*]pyridin-5-yl)aminomethylthio)hydrazinyl)benzoate (1.43 mg, 63% of yield). LCMS ESI(+)m/z:704.2(M+1).

### Step I:

The compound benzyl 2-((benzyloxycarbonyl)oxy)-5-(2-((4-((1*R*,4*R*)-4-(cyanomethyl)cyclohexyl)amino)-1*H*-pyrrolo[2,3-*b*]pyridin-5-yl)aminomethylthio)hydrazinyl)benzoate (1.43 g) was dissolved in 50 mL of dry THF, and EDCI (584 mg) and triethylamine (616 mg) were added successively to the above solution. The reaction system was stirred at room temperature 16 hours, concentrated under reduced pressure, and purified by C18 reverse silica gel column chromatography (methanol : 0.1% formic acid aqueous solution = 75%) to obtain a compound benzyl 5-((*E*)-(1-((1*R*,4*R*)-4-(cyanomethyl)cyclohexyl)-1,6-dihydroimidazo[4,5-*d*]pyrrolo[2,3-*b*]pyridin-2-yl)diazenyl)-2-hydroxybenzoate (318 mg, 29% of yield). LCMS ESI(+)m/z: 534.2(M+1).

### Step J:

The compound benzyl 5-((*E*)-(1-((1*R*,4*R*)-4-(cyanomethyl)cyclohexyl)-1,6-dihydroimidazo[4,5-*d*]pyrrolo[2,3-*b*]pyridin-2-yl)diazenyl)-2-hydroxybenzoate (661 mg) was suspended in 60 mL of isopropanol, and 3.5 N sodium hydroxide solution (12 mL) was added to the above solution. The reaction system was stirred at room temperature for 16 hours, and then was adjusted to pH=5 with 1 N HCl. The reaction solution was evaporated to remove isopropanol under reduced pressure, suction filtered under reduced pressure, washed with water (30 mL), then washed with acetonitrile (30 mL), and lyophilized to obtain the compound 5-((*E*)-(1-((1*R*,4*R*)-4-(cyanomethyl)cyclohexyl)-1,6-dihydroimidazo[4,5-*d*]pyrrolo[2,3-*b*]pyridin-2-yl)diazenyl)-2-hydroxybenzoic acid (415 mg, 76% of yield). ¹H NMR (400 MHz, DMSO-*d*₆) *δ* 12.16 (s, 1H), 8.70 (s, 1H), 8.46 (d, *J =* 2.4 Hz, 1H), 8.14 (dd, *J =* 8.9, 2.2 Hz, 1H), 7.58 (s, 1H), 7.10 (d, *J =* 9.0 Hz, 1H), 6.95 (s, 1H), 5.19 (s, 1H), 2.60 (d, *J =* 5.9 Hz, 2H), 2.44 (d, *J =* 12.3 Hz, 2H), 2.15 (d, *J* = 11.1 Hz, 2H), 2.04 (d, *J* = 11.5 Hz, 3H), 1.61-1.45 (m, 2H). LCMS ESI(+)m/z:444.2(M+1).

### Example 19: Synthesis of Compound B7

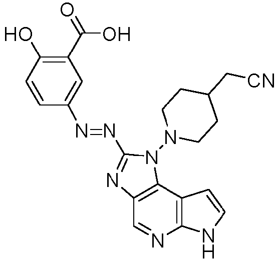

### (E)-5-((1-(4-cyanomethylpiperidin-1-yl)-1,6-dihydroimidazo[4,5-d]pyrrolo[2,3-b]pyridin-2-yl)diaze nyl)-2-hydroxybenzoic acid

The specific synthesis steps are as follows:

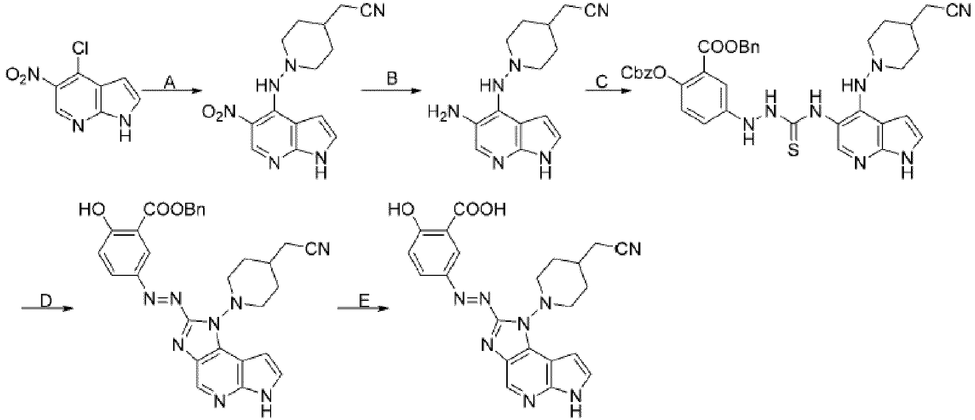

### Step A:

The compound 4-chloro-5-nitro-1*H*-pyrrolo[2,3-*b*]pyridine (3.22 g) was suspended in 100 mL isopropyl alcohol, and *N*,*N*-diisopropylethylamine (6.32 mL) and 2-((1*R*,4*R*)-4-aminocyclohexyl)acetonitrile hydrochloride (3.70 g) were added successively to the above solution. The reaction system was heated to 90°C under nitrogen protection, and stirred for 16 hours. Then the reaction system was cooled to room temperature, suction filtered under reduced pressure, and washed with 20 mL isopropanol. The filter cake was dried under reduced pressure to obtain a compound 2-(4-((5-nitro-1*H*-pyrrolo[2,3-b]pyridin-4-yl)amino)cyclohexyl)acetonitrile (4.88 g, 100% of yield). ¹H NMR (400 MHz, DMSO-*d*₆) *δ* 12.18 (s,1H), 9.01 (d, *J =* 8.0 Hz, 1H), 8.86 (s, 1H), 7.98 (s, 1H), 7.33 (d, *J =* 3.2 Hz, 1H), 6.71 (d, *J =* 3.5 Hz,1H), 4.13-4.01 (m,1H), 2.54-2.51 (m, 2H), 2.21-2.12 (m, 2H), 1.91-1.82 (m, 2H), 1.76-1.65 (m, 1H), 1.56-1.45 (m,2H), 1.42-1.30 (m, 2H).

### Step B:

The compound 2-(4-((5-nitro-1*H*-pyrrolo[2,3-b]pyridin-4-yl)amino)cyclohexyl)acetonitrile (3.20 g) was added to 100 mL of ethanol, and stannous chloride dihydrate (12.0 g) was added to the above solution at room temperature. The reaction system was stirred at 45°C for 2 hours, cooled to room temperature, and adjusted to pH=8-9 with an addition of 2 N NaOH solution under an ice bath, and then stirred at room temperature for 15 minutes. 300 mL ethyl acetate and diatomite were added to the reaction solution, which was then suction filtered with diatomite, and the filter cake was washed with 30 mL of ethyl acetate. The organic phase was separated and extracted twice with 50 mL of ethyl acetate. Then the organic phases were combined, washed with 50 mL saturated brine, dried with anhydrous sodium sulfate, and then suction filtered. The filtrate was concentrated and purified by silica gel column chromatography (methanol : dichloromethane = 1: 20) to obtain a compound 2-(4-((5-amino-1*H*-pyrrolo[2,3-*b*]pyridin-4-yl)amino)cyclohexyl)acetonitrile (1.23 g, 43% of yield). ¹H NMR (400 MHz, DMSO-*d*₆) *δ* 10.79 (s, 1H), 7.54 (s, 1H), 7.00 (d, *J =* 1.9 Hz, 1H), 6.70 (d, *J* = 2.1 Hz, 1H), 6.31 (s, 1H), 4.40 (s, 2H), 3.08 (d, *J* = 11.0 Hz, 2H), 2.55 (d, *J =* 6.5 Hz, 2H), 2.48-2.40 (m, 2H), 1.82-1.74 (m, 2H), 1.72-1.62 (m, 1H), 1.53-1.39 (m, 2H). LCMS ESI(+)m/z: 271.2(M+1).

### Step C:

Thiocarbonyldiimidazole (923 mg) was dissolved in THF (100 mL), and compound 2-(4-((5-amino-1*H*-pyrrolo[2,3-*b*]pyridin-4-yl)amino)cyclohexyl)acetonitrile (2.03 g) in THF (15 mL) was added to the above solution at room temperature. The reaction system was stirred at room temperature for 15 minutes under nitrogen protection. Compound 9 (1.00 g) in DMF (30 mL) and triethylamine (524 mg) were added to the reaction system and stirred at 60°C for 2 hours under nitrogen protection. The reaction solution was concentrated under reduced pressure and purified by silica gel column chromatography (methanol : dichloromethane = 1: 30) to obtain a compound 2-((1-((5-amino-1*H*-pyrrolo[2,3-*b*]pyridin-4-yl)amino)piperidin-4-yl)acetonitrile (2.13 g). LCMS ESI(+)m/z: 705.2 (M+1).

### Step D:

2-((1-((5-Amino-1*H*-pyrrolo[2,3-*b*]pyridin-4-yl)amino)piperidin-4-yl)acetonitrile (2.13 g) was dissolved in 150 mL dry THF under nitrogen, and EDCI (1.16 g) and triethylamine (917 mg) were added successively to the above solution and stirred at room temperature for 48 hours. The reaction solution was concentrated under reduced pressure and purified by C18 reverse silica gel column chromatography (methanol : 0.1% formic acid aqueous solution = 70%) to obtain a compound benzyl (*E*)-5-((1-(4-(cyanomethyl)piperidin-1-yl)-1,6-dihydroimidazo[4,5-*d*]pyrrolo[2,3-*b*]pyridin-2-yl)dia zenyl)-2-hydroxybenzoate (102 mg, 6% of yield). ¹H NMR (400 MHz, DMSO-*d*₆) *δ* 11.97 (s, 1H), 8.61 (s, 1H), 8.45 (m, 2H), 8.19-8.12 (m, 2H), 7.57-7.49 (m, 4H), 7.50-7.34 (m, 1H), 7.16 *(d, J=* 9.0 Hz, 1H), 6.93-6.88 (m, 1H), 5.42 (s, 2H), 3.64 (m, 2H), 3.41 (d, *J=* 10.7 Hz, 2H), 2.63 *(d, J=* 5.9 Hz, 2H), 1.95-1.87 (m, 2H), 1.86-1.65 (m, 3H). LCMS ESI(+)m/z: 534.2 (M+1) .

### Step E:

The compound benzyl (*E*)-5-((1-(4-(cyanomethyl)piperidin-1-yl)-1,6-dihydroimidazo[4,5-*d*]pyrrolo[2,3-*b*]pyridin-2-yl)dia zenyl)-2-hydroxybenzoate (100 mg) was suspended in 9 mL of isopropanol and 3 N sodium hydroxide solution (1.5 mL) was added to the above solution. The reaction system was stirred at 25°C for 16 hours, and adjusted to pH=3 with 1 N HCl. The reaction solution was evaporated to remove isopropanol under reduced pressure, suction filtered under reduced pressure, washed with water (30 mL), then washed with acetonitrile (30 mL), and lyophilized to obtain the compound (*E*)-5-((1-(4-cyanomethylpiperidin-1-yl)-1,6-dihydroimidazo[4,5-*d*]pyrrolo[2,3-*b*]pyridin-2-yl)diaze nyl)-2-hydroxybenzoic acid (82 mg, 99% of yield). ¹H NMR (400 MHz, DMSO-*d*₆) *δ* 12.19 (s, 1H), 8.73 (s, 1H), 8.51 (d, *J* = 2.6 Hz, 1H), 8.22 (dd, *J* = 9.0, 2.6 Hz, 1H), 7.61-7.54 (m, 1H), 7.21 (d, *J =* 9.0 Hz, 1H), 7.00-6.96 (m, 1H), 3.73-3.64 (m, 2H), 3.51-3.44 (m, 2H), 2.69 (d, *J =* 5.9 Hz, 2H), 2.05-1.85 (m, 3H), 1.82-1.66 (m, 2H). LCMS ESI(+)m/z:445.2(M+1).

### Example 20: Synthesis of Compound B8

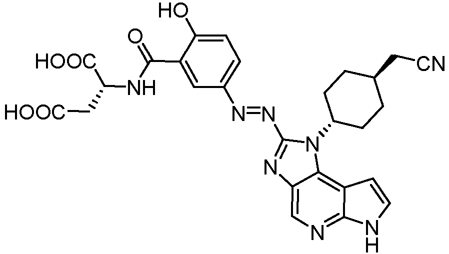

### (5-((E)-(1-((1R,4R)-4-(cyanomethyl)cyclohexyl)-1,6-dihydroimidazo[4,5-d]pyrrolo[2,3-b]pyridin-2 -yl)diazenyl)-2-hydroxybenzoyl)-D-aspartic acid

The specific synthesis steps are as follows:

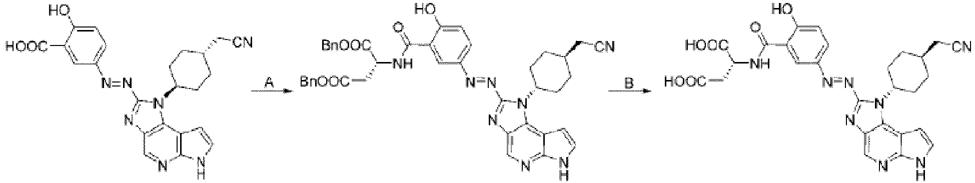

### Step A:

5-((E)-(1-((1R,4R)-4-(cyanomethyl)cyclohexyl)-1,6-dihydroimidazo[4,5-*d*]pyrrolo[2,3-*b*]pyrid in-2-yl)diazenyl)-2-hydroxybenzoic acid (147 mg) was dissolved in DMF (8 mL), and dibenzyl D-aspartate (260 mg), DCC (171 mg) and pyridine (157 mg) were added successively to the above solution under nitrogen protection at room temperature. The reaction system was stirred at room temperature under nitrogen protection for 48 hours, and purified by C18 reverse silica gel column chromatography (acetonitrile : 0.1% formic acid aqueous solution = 60%) to obtain dibenzyl (5-((E)-(1-((1R,4R)-4-(cyanomethyl)cyclohexyl)-1,6-dihydroimidazo[4,5-*d*]pyrrolo[2,3-b]pyridin-2 -yl)diazenyl)-2-hydroxybenzoyl)-D-aspartate (73 mg, 30% of yield). ¹H NMR (400 MHz, CDCl₃) *δ* 12.13 (s, 1H), 8.77-8.62 (m, 2H), 8.10 (d, *J =* 8.2 Hz, 1H), 7.57 (s, 1H), 7.39-7.25 (m, 10H), 7.17 (s, 1H), 6.93 (s, 1H), 5.17 (s, 2H), 5.12 (s, 2H), 5.11-5.04 (m, 1H), 3.17-3.02 (m, 2H), 2.59 (d, *J=* 6.1 Hz, 2H), 2.48-2.38 (m, 2H), 2.19-2.08 (m, 2H), 2.08-1.94 (m, 3H), 1.60-1.45 (m, 2H). LCMS ESI(+)m/z:444.2(M+1). LCMS ESI(+)m/z:739.2(M+1).

### Step B:

The compound dibenzyl (5-((*E*)-(1-((1*R*,4*R*)-4-(cyanomethyl)cyclohexyl)-1,6-dihydroimidazo[4,5-*d*]pyrrolo[2,3-*b*]pyridin-2 -yl)diazenyl)-2-hydroxybenzoyl)-*D*-aspartate (69 mg) was suspended in 12 mL of isopropanol and 3.5 N sodium hydroxide solution (3 mL) was added to the above solution. The reaction system was stirred at room temperature for 16 hours, and adjusted to pH=5 with 1 N HCl. The reaction solution was evaporated under reduced pressure to remove isopropanol, suction filtered under reduced pressure, and lyophilized. Water (12 mL) was added to the reaction system, and the reaction system was centrifuged. Then water (12 mL) was added to the reaction system, and the reaction system was centrifuged and lyophilized to obtain the compound (5-((*E*)-(1-((1*R*,4*R*)-4-(cyanomethyl)cyclohexyl)-1,6-dihydroimidazo[4,5-*d*]pyrrolo[2,3-*b*]pyridin-2 -yl)diazenyl)-2-hydroxybenzoyl)-D-aspartic acid (45 mg, 86% of yield). ¹H NMR (400 MHz, DMSO-*d*₆) *δ* 12.98 (s, 1H), 12.57 (s, 1H), 12.17 (s, 1H), 9.46 (s, 1H), 8.83-8.63 (m, 2H), 8.11 (d, *J* = 8.1 Hz, 1H), 7.58 (s, 1H), 7.20 (s, 1H), 6.95 (s, 1H), 5.20 (s, 1H), 4.86 (dd, *J =* 13.4, 5.9 Hz, 1H), 2.89 (d, *J =* 5.7 Hz, 2H), 2.69-2.56 (m, 2H), 2.48-2.37 (m, 2H), 2.20- 2.09 (m, 2H), 2.09-1.99 (m, 3H), 1.59-1.46 (m, 2H). LCMS ESI(+)m/z:559.2(M+1).

### Example 21: Synthesis of Compound B9

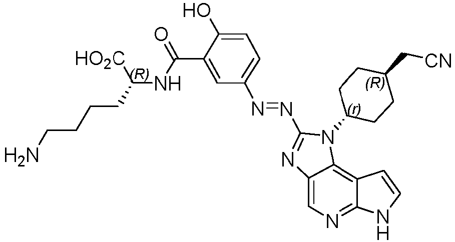

### (5-((E)-(1-((1R,4R)-4-(cyanomethyl)cyclohexyl)-1,6-dihydroimidazo[4,5-d]pyrrolo[2,3-b]pyridin-2 -yl)diazenyl)-2-hydroxybenzoyl)-D-lysine

The specific synthesis steps are as follows:

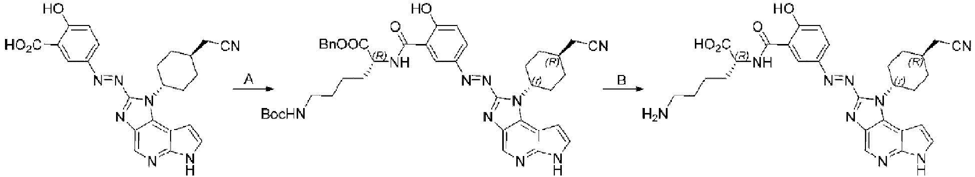

### Step A:

5-((*E*)-(1-((1*R*,4*R*)-4-(cyanomethyl)cyclohexyl)-1,6-dihydroimidazo[4,5-*d*]pyrrolo[2,3-*b*]pyrid in-2-yl)diazenyl)-2-hydroxybenzoic acid (285 mg) was dissolved in DMF (15 mL), and benzyl *N⁶*-Boc-*D*-lysinate (540 mg), DCC (332 mg) and pyridine (305 mg) were added successively to the above solution at room temperature under nitrogen protection. The reaction system was stirred at room temperature under nitrogen protection for 18 hours, and purified by C18 reverse silica gel column chromatography (acetonitrile : 0.1% formic acid aqueous solution = 50%) to obtain a compound benzyl *N⁶*-Boc-*N²*-(5-((*E*)-(1-((1*R*,4*R*)-4-(cyanomethyl)cyclohexyl)-1,6-dihydroimidazo[4,5-*d*]pyrrolo[2,3-*b*]pyridin-2-yl)diazenyl)-2-hydroxybenzoyl)-*D*-lysinate (120 mg, 25% of yield). ¹H NMR (400 MHz, CDCl₃) *δ* 13.61 (s, 1H), 12.05 (s, 1H), 8.77 - 8.62 (m, 2H), 8.13 (s, 1H), 7.95 (s, 1H), 7.54(t, *J* = 2.8 Hz, 1H), 7.38-7.32 (m, 5H), 6.98 - 6.77 (m, 3H), 5.19-5.13 (m, 3H), 4.57-4.55 (m, 1H),3.17 (s, 1H), 2.59 (d, *J* = 6.1 Hz, 2H), 2.48 - 2.38 (m, 2H), 2.19-1.94 (m, 5H), 1.87-1.75 (m, 2H), 1.52-1.48 (m, 2H), 1.45-1.41 (m, 10H). LCMS ESI(+)m/z:762.2(M+1).

### Step B:

The compound benzyl *N⁶*-Boc-*N²*-(5-((*E*)-(1-((1*R*,4*R*)-4-(cyanomethyl)cyclohexyl)-1,6-dihydroimidazo[4,5-*d*]pyrrolo[2,3-*b*]pyridin-2-yl)diazenyl)-2-hydroxybenzoyl)-*D*-lysinate (110 mg) was suspended in 10 mL of isopropanol, and 3.5 N sodium hydroxide solution (2.4 mL) was added to the above solution. The reaction system was stirred at room temperature for 16 hours, and adjusted to pH=5 with 1 N HCl. The reaction solution was evaporated to remove isopropanol under reduced pressure, and suction filtered under reduced pressure. Then water (12 mL) was added to the reaction system, centrifuged, and lyophilized to obtain the compound (5-((*E*)-(1-((1*R*,4*R*)-4-(cyanomethyl)cyclohexyl)-1,6-dihydroimidazo[4,5-*d*]pyrrolo[2,3-*b*]pyridin-2 -yl)diazenyl)-2-hydroxybenzoyl)-*D*-lysine (73 mg, 89% of yield). ¹H NMR (400 MHz, DMSO-*d*₆) *δ* 12.26 (s, 1H), 9.33 (t, *J =* 1.6 Hz, 1H), 8.85 (d, *J =* 2.0 Hz, 1H), 8.73 (s, 1H), 8.12-8.09 (m, 1H), 7.95 - 7.89 (m, 3H), 7.60 (t, *J =* 3.2 Hz, 1H), 7.23 (d, *J =* 8.4 Hz, 1H), 6.98 (s, 1H), 5.21 - 5.12 (m, 1H), 4.51 - 4.47 (m, 1H), 2.78-2.71 (m, 2H), 2.67-2.56 (m, 2H), 2.48-2.37 (m, 1H), 2.20-2.09 (m, 2H), 2.09-1.99 (m, 4H), 1.74-1.38 (m, 7H). LCMS ESI(+)m/z:572.2(M+1).

### Example 22: Synthesis of Compound B10

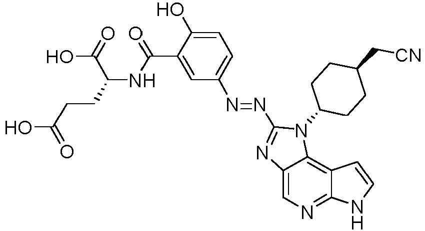

### (5-((E)-(1-((1R,4R)-4-(cyanomethyl)cyclohexyl)-1,6-dihydroimidazo[4,5-d]pyrrolo[2,3-b]pyridin-2 -yl)diazenyl)-2-hydroxybenzoyl)-D-glutamic acid

The specific synthesis steps are as follows:

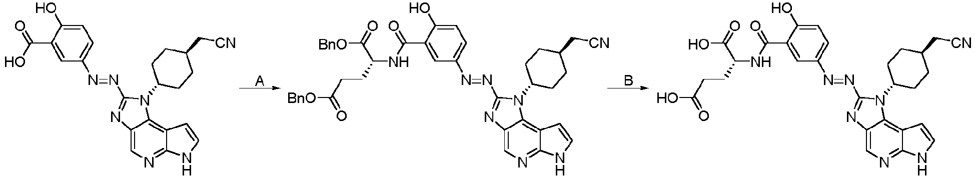

### Step A:

5-((*E*)-(1-((1*R*,4*R*)-4-(cyanomethyl)cyclohexyl)-1,6-dihydroimidazo[4,5-*d*]pyrrolo[2,3-*b*]pyri din-2-yl)diazenyl)-2-hydroxybenzoic acid (140 mg) and dibenzyl *D*-glutamate (246 mg) were dissolved in 5 mL of *N*,*N*-dimethylformamide, and then dicyclohexylcarbodiimide (163 mg) and pyridine (55 mg) were added to the above solution. The reaction solution was stirred at room temperature for 16 hours under nitrogen protection. After the reaction was completed, the reaction system was spin-dried and purified by column chromatography to obtain dibenzyl (5-((*E*)-(1-((1*R*, 4*R*)-4-(cyanomethyl)cyclohexyl)-1,6-dihydroimidazo[4,5-*d*]pyrrolo[2,3-*b*]pyridin-2 -yl)diazenyl)-2-hydroxybenzoyl)-*D*-glutamate (135 mg, 56.8% of yield).

### Step B:

The compound dibenzyl (5-((*E*)-(1-((1*R*,4*R*)-4-(cyanomethyl)cyclohexyl)-1,6-dihydroimidazo[4,5-*d*]pyrrolo[2,3-*b*]pyridin-2 -yl)diazenyl)-2-hydroxybenzoyl)-*D*-glutamate (135 mg) was dissolved in 24 mL of isopropanol and 3.5 N sodium hydroxide solution (6.0 mL) was added to the above solution. The reaction solution was stirred at room temperature for 16 hours. After the reaction was completed, the reaction system was adjusted to pH = 7 with an addition of 1 N hydrochloric acid, evaporated to remove isopropanol under reduced pressure, then adjusted to pH = 3 with 1 N hydrochloric acid, and lyophilized to obtain a solid. The solid was added with water and centrifuged twice, and the supernatant was removed. The residue was lyophilized to obtain the compound (5-((*E*)-(1-((1*R*, 4*R*)-4-(cyanomethyl)cyclohexyl)-1,6-dihydroimidazo[4,5-*d*]pyrrolo[2,3-*b*]pyridin-2 -yl)diazenyl)-2-hydroxybenzoyl)-*D*-glutamic acid (95 mg, 92.5% of yield). ¹H NMR (400 MHz, DMSO-*d*₆) *δ* 13.38-12.51 (s, 2H), 12.23-12.09 (m, 1H), 9.32 (s, 1H), 8.82 (d, *J =* 2.4 Hz, 1H), 8.70 (d, *J =* 6.2 Hz, 1H), 8.12 (dd, *J =* 9.1, 2.4 Hz, 1H), 7.60 -7.55 (m, 1H), 7.18 (d, *J =* 8.9 Hz, 1H), 6.95 (s, 1H), 5.33-5.08 (m, 1H), 4.56 -4.49 (m, 1H), 2.59 (d, *J =* 6.1 Hz, 2H), 2.47-2.44 (m, 1H), 2.41 (t, *J=* 7.5 Hz, 2H), 2.22-1.98 (m, 8H), 1.58 - 1.48 (m, 2H). LC-MS: m/z 573.2 [M + H]⁺.

### Example 23: Synthesis of Compound B11

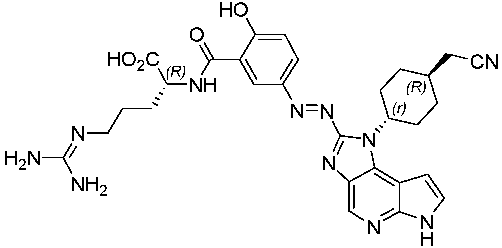

### (R)-2-(5-((E)-(1-((1R,4R)-4-(cyanomethyl)cyclohexyl)-1,6-dihydroimidazo[4,5-d]pyrrolo[2,3-b]pyr idin-2-yl)diazenyl)-2-hydroxybenzamido)-5-((diaminomethylene)amino)valeric acid

The specific synthesis steps are as follows:

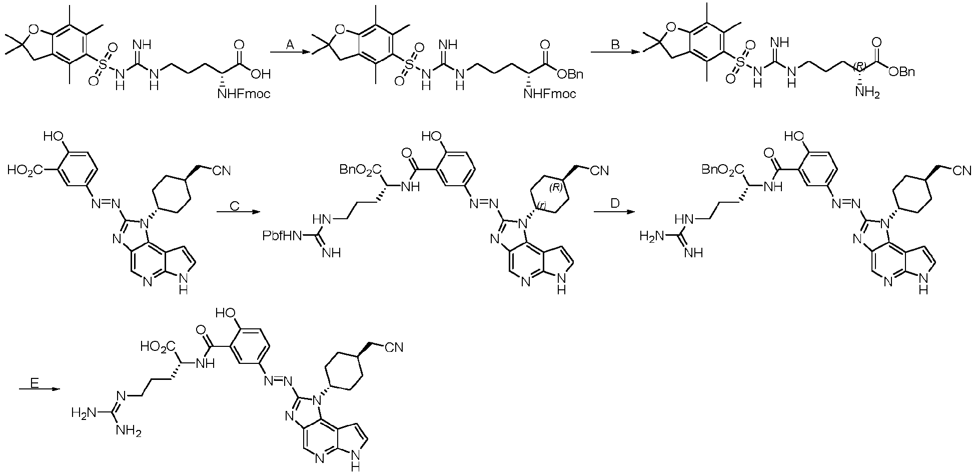

### Step A:

*N²*-(((9H-fluoren-9-yl)methoxy)carbonyl)-*N^{ω}*-((2,2,4,6,7-pentamethyl-2,3-dihydrobenzofuran-5-yl)sulfonyl)-*D*-arginine (1.60 g) was dissolved in *N*,*N*-dimethylformamide (100 mL), and potassium carbonate (0.408 g) and benzyl bromide (0.506 g) were added to the above solution at room temperature. The reaction system was stirred at room temperature under nitrogen protection for 16 hours. After the reaction was completed, the reaction system was suction filtered, concentrated under reduced pressure and purified by column chromatography to obtain benzyl *N²*-(((9*H*-fluoren-9-yl)methoxy)carbonyl)-*N^{ω}*-((2,2,4,6,7-pentamethyl-2,3-dihydrobenzofuran-5-yl)s ulfonyl)-*D*-argininate (1.76 g, 96% of yield). LCMS ESI(+)m/z:739.2(M+1).

### Step B:

Piperidine (2 mL dissolved in 8 mL of *N*,*N*-dimethylformamide) was added to benzyl *N²*-(((9H-fluoren-9-yl)methoxy)carbonyl)-*N^{ω}*-((2,2,4,6,7-pentamethyl-2,3-dihydrobenzofuran-5-yl)s ulfonyl)-*D*-argininate (1.66 g). The reaction system was stirred at room temperature under nitrogen protection for 1 hour. At the reaction was completed, solvent was spin-dried, and the residue was purified by column chromatography to obtain a compound *N^{ω}*-((2,2,4,6,7-pentamethyl-2,3-dihydrobenzofuran-5-yl)sulfonyl)-D-arginine (928 mg, 80% of yield). LCMS ESI(+)m/z:517.2(M+1).

### Step C:

5-((*E*)-(1-((1*R*,4*R*)-4-(cyanomethyl)cyclohexyl)-1,6-dihydroimidazo[4,5-*d*]pyrrolo[2,3-*b*]pyrid in-2-yl)diazenyl)-2-hydroxybenzoic acid (260 mg) was dissolved in DMF (15 mL), and *N^{ω}*-((2,2,4,6,7-pentamethyl-2,3-dihydrobenzofuran-5-yl)sulfonyl)-*D*-arginine (757 mg), HOBT (119 mg), DCC (181 mg) and 4-methylmorpholine (356 mg) were added successively to the above solution at room temperature under nitrogen protection. The reaction system was stirred at 40°C for 18 hours, and was purified by C18 reverse silica gel column chromatography to obtain benzyl *N²*-(5-((*E*)-(1-((1*R*,4*R*)-4-(cyanomethyl)cyclohexyl)-1,6-dihydroimidazo[4,5-*d*]pyrrolo[2,3-*b*]pyridi n-2-yl)diazenyl)-2-hydroxybenzoyl)-*N^{ω}*-((2,2,4,6,7-pentamethyl-2,3-dihydrobenzofuran-5-yl)sulfon yl)-*D*-argininate (280 mg, 50% of yield). ¹H NMR (400 MHz, DMSO-*d*₆) *δ* 13.03 (s, 1H), 12.11 (s, 1H), 8.77 (s, 1H), 8.65 (s, 1H), 8.14 (s, 1H), 7.56 (s, 1H), 7.37-7.30 (m, 5H), 7.11 - 6.77 (m, 3H), 6.42 (s, 1H), 5.21-5.13 (m, 3H), 4.58-4.56 (m, 1H), 3.11 - 3.08 (m, 2H), 2.89 (s, 2H), 2.59 - 2.51 (m,2H), 2.46-2.40 (m, 8H), 2.12-1.88 (m, 10H), 1.52-1.23 (m, 10H). LCMS ESI(+)m/z:943.2(M+1).

### Step D:

The compound benzyl *N²*-(5-((*E*)-(1-((1*R*,4*R*)-4-(cyanomethyl)cyclohexyl)-1,6-dihydroimidazo[4,5-*d*]pyrrolo[2,3-*b*]pyridi *n*-2-yl)diazenyl)-2-hydroxybenzoyl)-*N^{ω}*-((2,2,4,6,7-pentamethyl-2,3-dihydrobenzofuran-5-yl)sulfon yl)-D-argininate (0.27 g) was dissolved in 10 mL of dichloromethane, and 10 mL of trifluoroacetic acid was added dropwise under an ice bath, and then reacted at room temperature for 2 hours. After the reaction was completed, solvent was spin-dried and the reaction system was purified by column chromatography to obtain a compound benzyl (5-((*E*)-(1-((1*R*,4*R*)-4-(cyanomethyl)cyclohexyl)-1,6-dihydroimidazo[4,5-*d*]pyrrolo[2,3-*b*]pyridin-2 -yl)diazenyl)-2-hydroxybenzoyl)-*D*-argininate (180 mg, 91% of yield). LCMS ESI(+)m/z:590.2(M+1).

### Step E:

The compound benzyl *(5-((E)-(1-((1R,4R)-4-(cyanomethyl)cyclohexyl)-1,6-dihydroimidazo[4,5-d]pyrrolo[2,3-b]pyridin-2* -yl)diazenyl)-2-hydroxybenzoyl)-*D*-argininate (160 mg) was suspended in 30 mL of isopropanol, and 4 N sodium hydroxide solution (5 mL) was added to the above solution. The reaction system was stirred at room temperature for 16 hours, then adjusted to pH=5 with 1 N HCl, evaporated under reduced pressure to remove isopropanol, suction filtered under reduced pressure, and lyophilized. Water (12 mL) was added to the reaction system, and the reaction system was centrifuged, lyophilized and finally purified by reverse column chromatography to obtain (R)-2-(5-((*E*)-(1-((1*R*,4*R*)-4-(cyanomethyl)cyclohexyl)-1,6-dihydroimidazo[4,5-*d*]pyrrolo[2,3-*b*]pyr idin-2-yl)diazenyl)-2-hydroxybenzamido)-5-((diaminomethylene)amino)valeric acid (69.1 mg, 50% of yield).
¹H NMR (400 MHz, DMSO-*d*₆) *δ* 11.91 (s, 1H), 11.34 (s, 1H), 8.56 (s, 1H), 8.45 (d, *J =* 2.8 Hz, 1H), 7.82 - 6.77 (m, 8H), 6.54 (d, *J =* 9.2 Hz, 1H), 5.12 (q, *J =* 2.4 Hz, 1H), 4.50 (d, *J =* 3.2 Hz, 1H), 3.18-3.17 (m, 2H), 2.61-2.60 (m, 2H), 2.48-2.44 (m, 1H), 2.05-1.73 (m, 8H), 1.58-1.47 (m, 4H). LCMS ESI(+)m/z:600.2(M+1).

### Example 24: Synthesis of Compound B12

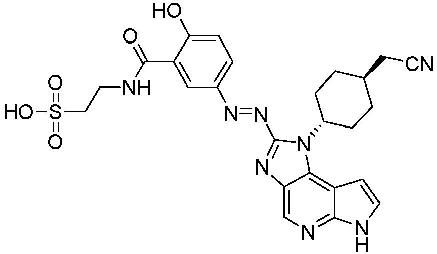

### 2-(5-((E)-(1-((1R,4R)-4-(cyanomethyl)cyclohexyl)-1,6-dihydroimidazo[4,5-d]pyrrolo[2,3-b]pyridin -2-yl)diazenyl)-2-hydroxybenzamido)ethan-1-sulfonic acid

The specific synthesis steps are as follows:

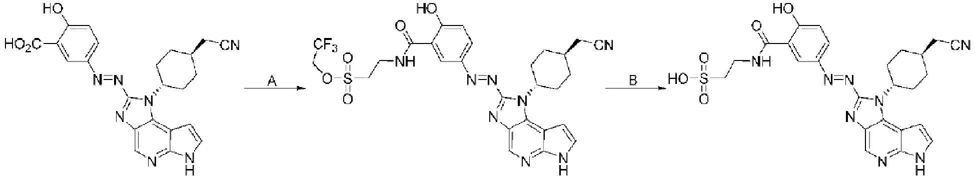

### Step A:

5-((*E*)-(1-((1*R*,4*R*)-4-(cyanomethyl)cyclohexyl)-1,6-dihydroimidazo[4,5-*d*]pyrrolo[2,3-*b*]pyrid in-2-yl)diazenyl)-2-hydroxybenzoic acid (236 mg) was dissolved in DMF (10 mL), and 2,2,2-trifluoroethyl 2-aminoethan-1-sulfonate (276 mg), DCC (275 mg) and pyridine (253 mg) were added successively to the above solution at room temperature under nitrogen protection. The reaction system was stirred at 35°C under nitrogen protection for 16 hours, and then purified by column chromatography (acetonitrile : 0.1% formic acid aqueous solution = 50%) to obtain a compound 2,2,2-trifluoroethyl 2-(5-((*E*)-(1-((1*R*,4*R*)-4-(cyanomethyl)cyclohexyl)-1,6-dihydroimidazo[4,5-*d*]pyrrolo[2,3-*b*]pyridin -2-yl)diazenyl)-2-hydroxybenzamido)ethan-1-sulfonate (141 mg, 42% of yield). ¹H NMR (400 MHz, DMSO-*d*₆) *δ* 13.04 (s, 1H), 12.08 (s, 1H), 9.71 (s, 1H), 8.66 (s, 2H), 8.04 (d, *J =* 7.2 Hz, 1H), 7.61 - 7.51 (m, 1H), 7.05 (s, 1H), 6.92 (s, 1H), 5.18 (s, 1H), 5.00 (q, *J* = 8.6 Hz, 2H), 3.83 (m, 4H), 2.60 (d, *J* = 6.1 Hz, 2H), 2.45 (m, 2H), 2.08 (m, 5H), 1.54 (m, 2H). LCMS ESI(+)m/z:663.4(M+1). Step B:
The compound 2,2,2-trifluoroethyl 2-(5-((E)-(1-((1R,4R)-4-(cyanomethyl)cyclohexyl)-1,6-dihydroimidazo[4,5-d]pyrrolo[2,3-*b*]pyridin -2-yl)diazenyl)-2-hydroxybenzamido)ethan-1-sulfonate (110 mg) was suspended in 12 mL of isopropanol, and 4 N sodium hydroxide solution (6 mL) was added to the above solution and stirred at 80°C for 16 hours. The reaction solution was adjusted to pH=3 with 1 N HCl, evaporated to remove isopropanol under reduced pressure, suction filtered under reduced pressure, and lyophilized. Then water (12 mL) was added to the reaction system and centrifuged. Then the residue was purified by C18 reversed silica gel column chromatography (methanol : 0.1% formic acid aqueous solution = 20%) to obtain the compound 2-(5-((*E*)-(1-((1*R*,4*R*)-4-(cyanomethyl)cyclohexyl)-1,6-dihydroimidazo[4,5-*d*]pyrrolo[2,3-*b*]pyridin -2-yl)diazenyl)-2-hydroxybenzamido)ethan-1-sulfonic acid (25 mg, 26% of yield). ¹H NMR (400 MHz, DMSO-*d*₆) *δ* 11.93 (s, 1H), 11.00 (s, 1H), 8.58 (s, 1H), 8.48 (d, *J =* 2.8 Hz, 1H), 7.78 (dd, *J =* 9.1, 2.8 Hz, 1H), 7.49 (s, 1H), 6.85 (d, *J =* 3.0 Hz, 1H), 6.53 (d, *J =* 9.1 Hz, 1H), 5.25-4.98 (m, 1H), 3.62 (dd, *J =* 12.1, 6.2 Hz, 2H), 2.75 (t, *J =* 6.4 Hz, 2H), 2.61 (d, *J =* 6.1 Hz, 2H), 2.48-2.42 (m, 2H), 2.07-1.93 (m, 5H), 1.57-1.42 (m, 2H). LCMS ESI(+)m/z:551.4(M+1).

The compounds in Table 1 were prepared by similar methods to those aforementioned examples (e.g., Examples 18 and 19) via different reaction starting materials and suitable reagents.

**Table 1**

| Examples | Compound No. | Chemical structure | Chemical name | LCMS ESI(+)m /z |
|---|---|---|---|---|
| 25 | B13 | 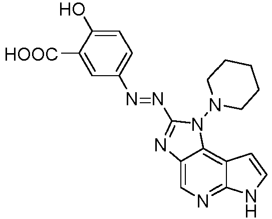 | (E)-2-hydroxyl-5-((1-(piperidin-1-yl)-1,6-dihydroimidazo[4,5-*d*]pyrr olo[2,3-*b*]pyridin-2-yl)diazenyl)be nzoic acid | 406.2 |
| 26 | B14 | 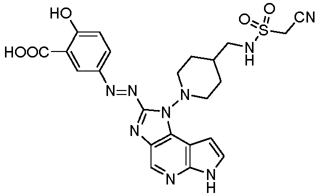 | (E)-5-((1-(4-((cyanomethyl))sulfon amido)methyl)piperidin-1-yl)-1,6-dihydroimidazo[4,5-*d*]pyrrolo[2,3-*b*]pyridin-2-yl)diazenyl)-2-hydrox ybenzoic acid | 538.2 |
| 27 | B15 | 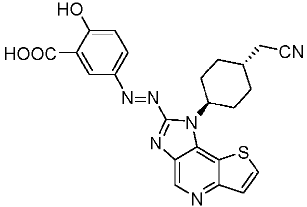 | 5-((E)-(1-((1R,4R)-4-(cyanomethy l)cyclohexyl)-1*H*-imidazo[4,5-*d*]th ieno[3,2-*b*]pyridin-2-yl)diazenyl)-2-hydroxybenzoic acid | 461.1 |
| 28 | B16 | 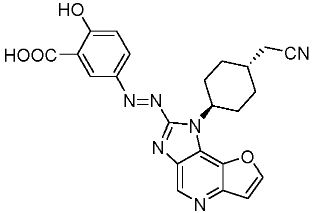 | 5-((E)-(1-((1R,4R)-4-(cyanomethy l)cyclohexyl)-1*H*-furo[3,2-*b*]imida zo[4,5-*d*]pyridin-2-yl)diazenyl)-2-hydroxybenzoic acid | 445.2 |

### Example A: Activity detection of JAK kinase small molecule inhibitors

### Experimental scheme

### 1. Reagent preparation

### (1) Kinase reaction buffer

Kinase reaction buffer was prepared with the following components: 50 mM HEPES, pH 7.5, 1 mM EGTA, 10 mM MgCl₂, 2 mM DTT, 0.01% Tween20.

### (2) 1X Detection buffer

Detection buffer was prepared by diluting 10X detection buffer to 1X with deionized water in a ratio of 9:1.

### (3) 4X Kinase solution

JAK kinase was diluted with kinase reaction buffer to 4X final concentration (JAK1: 40 nM, JAK2: 0.5 nM).

### (4) 4X Substrate solution

ULight^{™}-JAK-1 (Tyr1023) substrate was diluted with kinase reaction buffer to 200 nM (final concentration: 50 nM).

### (5) 4X ATP solution

ATP was diluted with kinase reaction buffer to 4X final concentration (JAK1: 160 µM, JAK2: 40 µM).

### (6) 4X Test compound solution

Compound to be tested was dissolved with DMSO to obtain a 10 mM storage solution, which was diluted to desired concentrations by a 3-fold gradient; for each compound 10 concentration points were set, and the final concentration of the compound to be tested ranges from 10 µM to 0.5 nM.

### (7) 4X Enzyme reaction termination solution

EDTA was dissolved with 1X detection buffer to 40 mM (EDTA final concentration: 10 mM).

### (8) 4X Detection antibody solution

Eu-labeled detection antibody (anti-phosphotyrosine (PT66)) was diluted with 1X detection buffer to 8 nM (final antibody concentration: 2 nM).

### 2. Experimental process

(1) To a 384-well plate, 2.5 µL 4X kinase solution and 2.5 µL 4X test compound solution with different concentrations that have been diluted were added successively, each concentration was set in duplicate, and an enzyme solution blank control and a negative control (DMSO group) were also set.
(2) The 384-well plate was shaken to mix enzyme and compound well, and then centrifuged at 1000 rpm for 1 minute, and incubated at room temperature for 60 minutes.
(3) 2.5 µL 4X substrate solution was added to the 384-well plate and centrifuged at 1000 rpm for 1 minute.
(4) 2.5 µL 4X ATP solution was added to the 384-well plate and centrifuged at 1000 rpm for 1 minute, and then enzyme reaction was initiated.
(5) For JAK1 the reaction was carried out at room temperature for 2 hours, and for JAK2 the reaction was carried out at room temperature for 1 hour.
(6) For the JAK1 reaction, the final concentrations of components are as follows: JAK1: 10 nM, substrate: 50 nM, ATP: 40 µM; and the final concentration of the tested compounds ranges from 10 µM to 0.5 nM.
   For the JAK2 reaction, the final concentrations of component are as follows: JAK2: 0.125 nM, substrate: 50 nM, ATP: 10 µM; and the final concentration of the tested compound ranges from 10 µM to 0.5 nM.
(7) After the enzyme reaction was completed, 5 µL 4X enzyme reaction termination solution was added to each well of the 384-well plate, and then centrifuged at 1000 rpm for 1 minute and incubated at room temperature for 5 minutes.
(8) 5 µL 4X detection antibody solution (the final concentration of detection antibody is 2 nM) was added to each well of the 384-well plate, and then centrifuged at 1000 rpm for 1 minute and incubated at room temperature for 1 hour.
(9) At the antibody incubation was completed, signal value of each well was measured on a Envision plate reader.

### 3. Data analysis

(1) The percentage inhibition rate corresponding to each concentration of the tested compound was calculated on the basis that the blank control of enzyme solution corresponds to 100% and the negative control (DMSO group) corresponds to 0%.
(2) A nonlinear regression analysis of the logarithm of the concentration of the detected compound and the corresponding percentage inhibition rate was performed in GraphPad Prism software to obtain the half-inhibiting concentration (IC₅₀) of the tested compound. The obtained experimental results are listed in Table 2.

**Table 2 Half-inhibiting concentration IC₅₀ of compounds**

| Example | JAK1 (IC₅₀, nM) | JAK2 (IC₅₀, nM) | | Example | JAK1 (IC₅₀, nM) | JAK2 (IC₅₀, nM) |
|---|---|---|---|---|---|---|
| A1 | 133 | 536.1 | | B1 | 2.15 | 38.7 |
| A2 | 5.94 | 27.0 | | B2 | 0.045 | 0.1 |
| A3 | 7.04 | 101.9 | | B3 | 0.04 | 0.37 |
| A4 | 105 | 100.1 | | B4 | 0.47 | 1.74 |
| A5 | 1.83 | 4.04 | | B5 | 1.33 | 7.15 |
| A6 | 31.3 | 40.3 | | B6 | 0.45 | 0.81 |
| A7 | 3.85 | 57.8 | | B7 | 1.0 | 0.27 |
| A8 | 5.86 | 72.0 | | B8 | 1.75 | 4.26 |
| A9 | 3.05 | 56.4 | | B9 | 2.52 | 6.62 |
| A10 | 96.7 | 21.1 | | B10 | 1.34 | 0.28 |
| A11 | 5.62 | 4.19 | | B11 | 1.6 | 3.38 |
| A12 | 38.9 | 37.9 | | B12 | 1.02 | 0.47 |

The above results show that the compounds of the present application have good kinase inhibitory activities on JAK1 and JAK2.

### Example B Study on Pharmacokinetic and Fecal Excretion in Rats

### 1. Experimental scheme

Compounds A1, A2, A3, B3, B6 and B3 were administered once to SD rats by gavage. The concentrations of medicaments and metabolites thereof in plasma were collected and measured at different time points, and the pharmacokinetic parameters of each compound were calculated to study the pharmacokinetic characteristics of the compounds in plasma of SD rats. Fecal samples at 8 and 24 hours were collected simultaneously, and the concentrations of prodrugs and degraded products were detected to investigate the excretion recovery rate.

### 2. Experimental process

Rats were grouped in groups of 3, and all SD rats were fasted overnight (12 hours or more) the day before the experiment. On the day of the experiment, after weighing the body weight, the rats were administrated once with compound A1, A2, A3, B3, B6, and B3 by gavage respectively. Dosages of test solution administered by gavage are shown in Table 3. SD rats were able to resume eating 4 hours after administration and were able to drink freely during the experiment. 0.20 mL of blood was collected from the jugular vein before administration and at 0.5, 1, 2, 4, 8, 12, and 24 hours after administration, respectively, and was placed in EDTA-K2 anticoagulation tube. All whole blood samples were centrifuged at 1500 g to 1600 g for 10 minutes within half an hour after collection to separate plasma, which was stored in a refrigerator at a temperature of between -90°C and -60°C for sample analysis. All the feces from the metabolic cage chassis were collected at 24 h after administration and weighed accurately. The required volume of homogenate liquid (aqueous solution containing 20% methanol) was added at a weight/volume ratio of 1:10, and the resultant was homogenized and analyzed.

### 3. Data analysis

**Table 3 Cumulative recovery rate of prodrugs and metabolites in feces at 24 h**

| Administration group | Dose (mg/kg) | Analyte | Cumulative recovery rate at 24 h (%) |
|---|---|---|---|
| Compound A1 | 50 | Compound A1 (prodrug) | 83 |
| | | Compound A13 (metabolite) | 11.6 |
| Compound A2 | 50 | Compound A2 (prodrug) | 7.2 |
| | | Compound A13 (metabolism) | 24.8 |
| Compound A3 | 40 | Compound A3 (prodrug) | 0.78 |
| | | Compound A13 (metabolite) | 34.3 |
| Compound B6 | 50 | Compound B6 (prodrug) | 5.1 |
| | | Compound B3 (metabolite) | 82.1 |
| Compound B3 | 33 (equimolar with B6) | Compound B3 | 71.2 |

**Table 4 PK parameters of prodrugs and metabolites in rats**

| Administration group | Dose (mg/kg) | Analyte | Tₘₐₓ (h) | Cₘₐₓ (ng·mL⁻¹) | AUC₀₋ₜ (ng·h·g⁻¹) |
|---|---|---|---|---|---|
| Compound A1 | 50 | Compound A1 (prodrug) | NA | BLOQ | NA |
| | | Compound A13 (metabolite) | 12 | 9.1 | NA |
| Compound A2 | 50 | Compound A2 (prodrug) | NA | BLOQ | NA |
| | | Compound A13 (metabolism) | 4 | 80.6 | 972 |
| Compound A3 | 40 | Compound A3 (prodrug) | 4 | 2.08 | 8.92 |
| | | Compound A13 (metabolism) | 12 | 69.9 | 836 |
| Compound B6 | 50 | Compound B6 (prodrug) | 4 | 0.7 | NA |
| | | Compound B3 (metabolite) | 12 | 4.8 | 69.1 |
| Compound B3 | 33 (equimolar with B6) | Compound B3 | 1.2 | 126 | 324 |

| | | | | | |
|---|---|---|---|---|---|
| "-": Not applicable BLOQ: Below the quantitative limit NA: Not applicable (Note: The metabolite A13 generated by compound A1 merely showed little absorption at 12 h, and AUC cannot be calculated. Compound B6 merely showed little absorption at 4 h, and AUC cannot be calculated.) | | | | | |

As can be seen from Table 3, the prodrugs can be degraded in the intestine to release active metabolites.

As shown in Table 4, the Tₘₐₓ of the active metabolites are generally higher than 4 h and typically 12 h, indicating that the degradation and absorption of the metabolites occur at the back end of the digestive tract, i.e., in the colon. After direct oral administration of the active compound B3, Tₘₐₓ is 1.2 h. These results indicate that the prodrug can delay the release of active ingredient and deliver the active ingredient to the back end of the digestive tract. In addition, as prodrugs, compounds A1, A2, A3, and B6 were absent or rarely present in plasma, indicating they were hardly absorbed via the blood system. They were degraded into active metabolites in the intestine, and only a small portion of the corresponding active metabolites was absorbed by the blood system. Compared with direct oral administration of equimolar amounts of the active metabolite B3, oral administration of the prodrug B6 resulted in a significant reduction in the blood system absorption (AUC₀₋ₜ) of the active metabolite B3, indicating that the design of the prodrug can significantly reduce the exposure of active metabolite in vivo, thereby reducing systemic side effects.

### Example C Study on Pharmacokinetic in Rats in vivo

### 1. Experimental scheme

Compounds A3 and A13 were administered once to SD rats by gavage in an equal molar amount relative to the body weight of rats. The concentration of the medicaments and metabolites thereof in plasma and colon tissues were collected and measured at different time points, and the pharmacokinetic parameters of the compounds were calculated to compare the pharmacokinetic characteristics of the prodrug and the active metabolite in SD rats in vivo.

### 2. Experimental process

Rats were grouped in groups of 27, and all SD rats were fasted overnight (12 hours or more) the day before the experiment. On the day of the experiment, after weighing the body weight, the compounds A3 (30 mg/kg) and A13 (15 mg/kg) were administered once by gavage respectively. Dosages of test solution administered by gavage are shown in Table 3. SD rats were able to resume eating after 4 hours of administration and were able to drink freely during the experiment. Plasma and colonic tissue were collected at 0.5, 1, 2, 4, 6, 8, 12, 24 and 48 hours after the administration to detect the concentration of A13 in the plasma and colon tissue homogenate liquid.

### 3. Data analysis, see Fig. 1

As can be seen from Fig. 1, after administration of an equal molar amount relative to the body weight of either prodrug A3 or compound A13 to rats, the amount of active metabolite A13 was less in the blood and more in the colonic tissue when the prodrug A3 was administered. This indicates that prodrug A3 can reduce the systemic exposure of compound A13, reduce systemic side effects, and increase its concentration at the target treatment site and enhance efficacy.

Although the present application has been fully described by means of embodiments, it is worth noting that various variations and modifications are readily apparent to a person skilled in the art. Such variations and modifications are intended to be included within the scope of the appended claims of the present invention.

## Claims

1. A compound of formula I, or a stereoisomer, a geometric isomer, a tautomer, a hydrate, a solvate, a metabolite or a pharmaceutically acceptable salt thereof: 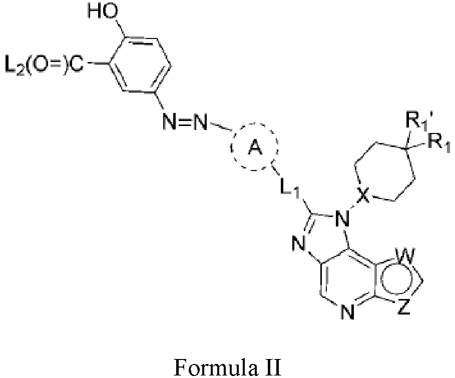 wherein,
X is selected from the group consisting of CRₐ and N;
W and Z are each independently selected from the group consisting of O, N, S, NR_{b}, and CR_{c}; W and Z are not CR_{c} at the same time;
R₁ is selected from the group consisting of H, halogen, CN, C₁₋₆-alkyl, C₁₋₆-alkoxy, and C₁₋₆-haloalkyl; the C₁₋₆-alkyl, C₁₋₆-alkoxy, and C₁₋₆-haloalkyl are optionally substituted by one or more R₁;
R₁' is selected from the group consisting of H, halogen, amino, hydroxyl, C₁₋₆-alkyl, C₁₋₆-haloalkyl, C₁₋₆-alkoxy, and C₁₋₆-haloalkoxy;
L₁ is absent or a C₁₋₁₄ (preferably C₁₋₇) divalent saturated or unsaturated, linear or branched hydrocarbon group, and 1, 2, or 3 methylene groups in the hydrocarbon group are optionally and independently replaced by: -CH(Rₑ)-, -C(Rₑ)₂-, C₃₋₅-cycloalkylene, -N(Rₑ)-, -N(Rₑ)C(=O)-, -C(=O)N(Rₑ)-, -N(Rₑ)S(=O)₂-, -S(=O)₂N(Rₑ)-, -O-, -C(=O)-, -OC(=O)-, -C(=O)O-, -S-, -S(=O)-, or -S(=O)₂-;
ring A is absent or is selected from the group consisting of C₆₋₁₄-aryl, 5-14-membered heteroaryl, 3-14-membered heterocyclyl, and 3-14-membered carbocyclyl; the C₆₋₁₄-aryl, 5-14-membered heteroaryl, 3-14-membered heterocyclyl, and 3-14-membered carbocyclyl are each optionally substituted by one or more R_{f};
L₂ is selected from the group consisting of H, OH, C₁₋₆-alkyl, C₁₋₆-alkoxy, C₂₋₆-alkenyl, -C₀₋₆-alkylene-NH(C₁₋₆-alkyl), and -C₀₋₆-alkylene-N(C₁₋₆-alkyl)₂; the C₁₋₆-alkyl, C₁₋₆-alkoxy, C₂₋₆-alkenyl, -C₀₋₆-alkylene-NH(C₁₋₆-alkyl), and -C₀₋₆-alkylene-N(C₁₋₆-alkyl)₂ are optionally substituted with one or more substituents selected from the group consisting of hydrogen, halogen, -CN, C₁₋₆-alkyl, C₁₋₆-haloalkyl, C₂₋₆-alkenyl, C₂₋₆-alkynyl, C₁₋₆-alkoxy, C₁₋₆-haloalkoxy, -OR_{g}, -NR_{g}Rₕ, -ONO₂, -NO₂, -S(=O)₂OR_{g}, -N=CR_{g}Rₕ, -COOR_{g}, -CONHR_{g}, -C₀₋₆-alkylene-NHC(=O)R_{g}, -C₀₋₆-alkylene-C(=O)NHR_{g}, and -C(=O)NR_{g}Rₕ;
Rₐ, R_{b}, and R_{c} are each independently selected from the group consisting of H, C₁₋₆-alkyl, C₁₋₆-haloalkyl, C₁₋₆-alkoxy, and C₁₋₆-haloalkoxy;
each R_{d} is independently selected from the group consisting of H, halogen, CN, -NO₂, OH, NH₂, -NH(C₁₋₆-alkyl), -N(C₁₋₆-alkyl)₂, oxo, C₁₋₆-alkyl, C₁₋₆-alkoxy, C₂₋₆-alkenyl, C₂₋₆-alkynyl, -S-C₁₋₆-alkyl, -S(=O)-C₁₋₆-alkyl, -S(=O)₂-C₁₋₆-alkyl, -S(=O)NH₂, -S(=O)NHC₁₋₆-alkyl, -S(=O)N(C₁₋₆-alkyl)₂, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl; the -OH, NH₂, -NH(C₁₋₆-alkyl), -N(C₁₋₆-alkyl)₂, C₁₋₆-alkyl, C₁₋₆-alkoxy, C₂₋₆-alkenyl, C₂₋₆-alkynyl, -S-C₁₋₆-alkyl, -S(=O)-C₁₋₆-alkyl, -S(=O)₂-C₁₋₆-alkyl, -S(=O)NH₂, -S(=O)NHC₁₋₆-alkyl, -S(=O)N(C₁₋₆-alkyl)₂, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl are optionally substituted with one or more substituents selected from the group consisting of H, halogen, CN, -NO₂, OH, NH₂, -COOH, oxo, C₁₋₆-alkyl, C₁₋₆-haloalkyl, C₂₋₆-alkenyl, C₂₋₆-alkynyl, C₁₋₆-alkoxy, C₁₋₆-haloalkoxy, -NH(C₁₋₆-alkyl), -N(C₁₋₆-alkyl)₂, -COO(C₁₋₆-alkyl), -CONH(C₁₋₆-alkyl), -C₀₋₆-alkylene-NHC(=O)C₁₋₆-alkyl, -C₀₋₆-alkylene-NHC(=O)C₁₋₆-haloalkyl, -C₀₋₆-alkylene-NHC(=O)C₂₋₆-alkenyl, and -CON(C₁₋₆-alkyl)₂; and
Rₑ, R_{f}, R_{g}, and Rₕ are each independently selected from the group consisting of H, C₁₋₆-alkyl, C₁₋₆-alkoxy, C₂₋₆-alkenyl, C₂₋₆-alkynyl, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl; wherein the C₁₋₆-alkyl, C₁₋₆-alkoxy, C₂₋₆-alkenyl, C₂₋₆-alkynyl, -C₀₋₆-alkylene-NHS(=O)₂-C₁₋₆-alkyl, -C(=O)-C₁₋₆-alkyl, -C(=O)-C₂₋₆-alkenyl, -C₀₋₆-alkylene-C₆₋₁₄-aryl, -C₀₋₆-alkylene-5-14-membered heteroaryl, -C₀₋₆-alkylene-C₃₋₁₄-carbocyclyl, and -C₀₋₆-alkylene-3-14-membered heterocyclyl are optionally substituted with one or more substituents selected from the group consisting of H, halogen, CN, -NO₂, OH, NH₂, -COOH, oxo, C₁₋₆-alkyl, C₁₋₆-haloalkyl, C₂₋₆-alkenyl, C₂₋₆-alkynyl, C₁₋₆-alkoxy, C₁₋₆-haloalkoxy, -NH(C₁₋₆-alkyl), -N(C₁₋₆-alkyl)₂, -COO(C₁₋₆-alkyl), -CONH(C₁₋₆-alkyl), -C₀₋₆-alkylene-NHC(=O)C₁₋₆-alkyl, -C₀₋₆-alkylene-NHC(=O)C₁₋₆-haloalkyl, -C₀₋₆-alkylene-NHC(=O)C₂₋₆-alkenyl, and -CON(C₁₋₆-alkyl)₂.

2. The compound or the stereoisomer, geometric isomer, tautomer, hydrate, solvate, metabolite or pharmaceutically acceptable salt thereof according to claim 1, wherein X is selected from the group consisting of CH and N.

3. The compound or the stereoisomer, geometric isomer, tautomer, hydrate, solvate, metabolite or pharmaceutically acceptable salt thereof according to claim 1 or 2, wherein W is selected from the group consisting of O, N, S, NH, and CH; preferably, W is CH.

4. The compound or the stereoisomer, geometric isomer, tautomer, hydrate, solvate, metabolite or pharmaceutically acceptable salt thereof according to any one of claims 1-3, wherein Z is selected from the group consisting of N, NR_{b}, and CR_{c}.

5. The compound or the stereoisomer, geometric isomer, tautomer, hydrate, solvate, metabolite or pharmaceutically acceptable salt thereof according to any one of claims 1-4, wherein Z is selected from the group consisting of N, NH, and CH; preferably, Z is NH.

6. The compound or the stereoisomer, geometric isomer, tautomer, hydrate, solvate, metabolite or pharmaceutically acceptable salt thereof according to any one of claims 1-5, wherein R_{d} is selected from the group consisting of H, OH, halogen, amino, CN, C₁₋₆-alkyl, C₁₋₆-haloalkyl, C₁₋₆-alkoxy, and -NHS(=O)₂-C₁₋₆-alkyl; wherein the C₁₋₆-alkyl, C₁₋₆-haloalkyl, C₁₋₆-alkoxy, -NHS(=O)₂-C₁₋₆-alkyl are optionally substituted with one or more substituents selected from the group consisting of halogen, CN, OH, NH₂, -NH(C₁₋₆-alkyl), and -N(C₁₋₆-alkyl)₂.

7. The compound or the stereoisomer, geometric isomer, tautomer, hydrate, solvate, metabolite or pharmaceutically acceptable salt thereof according to any one of claims 1-6, wherein R₁ is selected from the group consisting of H and C₁₋₄-alkyl, and the C₁₋₄-alkyl is optionally substituted with one or more substituents selected from the group consisting of CN, C₁₋₆-alkoxy, -S-C₁₋₆-alkyl, and -NHS(=O)₂C₁₋₆-alkyl-CN.

8. The compound or the stereoisomer, geometric isomer, tautomer, hydrate, solvate, metabolite or pharmaceutically acceptable salt thereof according to any one of claims 1-7, wherein R₁ is selected from the group consisting of H, -CH₂-CN, -CH₂-S-CH₃, and

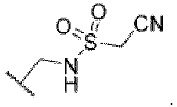

9. The compound or the stereoisomer, geometric isomer, tautomer, hydrate, solvate, metabolite or pharmaceutically acceptable salt thereof according to any one of claims 1-8, wherein R₁' is selected from the group consisting of H and C₁₋₃-alkyl.

10. The compound or the stereoisomer, geometric isomer, tautomer, hydrate, solvate, metabolite or pharmaceutically acceptable salt thereof according to any one of claims 1-9, wherein R₁' is selected from the group consisting of H and CH₃.

11. The compound or the stereoisomer, geometric isomer, tautomer, hydrate, solvate, metabolite or pharmaceutically acceptable salt thereof according to any one of claims 1-10, wherein Rₑ is selected from the group consisting of H, amino, C₁₋₄-alkyl, C₁₋₄-haloalkyl, and C₁₋₄-alkoxy.

12. The compound or the stereoisomer, geometric isomer, tautomer, hydrate, solvate, metabolite or pharmaceutically acceptable salt thereof according to any one of claims 1-11, wherein Rₑ is selected from the group consisting of H, methyl, ethyl, n-propyl, and isopropyl.

13. The compound or the stereoisomer, geometric isomer, tautomer, hydrate, solvate, metabolite or pharmaceutically acceptable salt thereof according to any one of claims 1-12, wherein L₁ is absent or a C₁₋₁₄ (preferably C₁₋₄) divalent saturated or unsaturated, liner or branched hydrocarbon group; 1, 2, or 3 methylene groups of the hydrocarbon group are optionally and independently replaced by: -CH(Rₑ)-, -C(Rₑ)₂-, C₃₋₅-cycloalkylene, -N(Rₑ)-, -N(Rₑ)C(=O)-, -C(=O)N(Rₑ)-, -O-, -C(=O)-, -OC(=O)-, or -C(=O)O-.

14. The compound or the stereoisomer, geometric isomer, tautomer, hydrate, solvate, metabolite or pharmaceutically acceptable salt thereof according to any one of claims 1-13, wherein L₁ is absent or is selected from the group consisting of -C₁₋₆-alkylene-, -C₁₋₆-alkylene-O-C₁₋₆-alkylene-, -C₁₋₆-alkylene-OC(=O)C₁₋₆-alkylene-, -C₁₋₆-alkylene-OC(=O)OC₁₋₆-alkylene-, -C₁₋₆-alkylene-OC(=O)C₁₋₆-alkylene-O-, -C₁₋₆-alkylene-OC(=O)-NRₐC₁₋₆-alkylene-, and -C₁₋₆-alkylene-OC(=O)-NRₐ-; wherein each C₁₋₆ alkylene is optionally substituted with 1-6 Rₑ.

15. The compound or the stereoisomer, geometric isomer, tautomer, hydrate, solvate, metabolite or pharmaceutically acceptable salt thereof according to any one of claims 1-14, wherein L₁ is absent or is selected from the group consisting of, -CH₂-, -CH₂CH₂-,

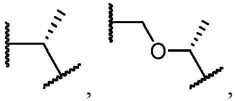

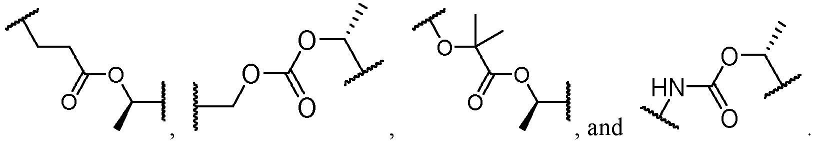

16. The compound or the stereoisomer, geometric isomer, tautomer, hydrate, solvate, metabolite or pharmaceutically acceptable salt thereof according to any one of claims 1-15, wherein R_{g} and Rₕ are each independently selected from the group consisting of H, C₁₋₆-alkyl, amino, and -COOH.

17. The compound or the stereoisomer, geometric isomer, tautomer, hydrate, solvate, metabolite or pharmaceutically acceptable salt thereof according to any one of claims 1-16, wherein L₂ is selected from the group consisting of hydrogen, OH, C₁₋₆-alkyl, C₂₋₆-alkenyl, -C₀₋₆-alkylene-NH(C₁₋₆-alkyl), and -C₀₋₆-alkylene-N(C₁₋₆-alkyl)₂; the C₁₋₆-alkyl, C₂₋₆-alkenyl, -C₀₋₆-alkylene-NH(C₁₋₆-alkyl), and -C₀₋₆-alkylene-N(C₁₋₆-alkyl)₂ are optionally substituted with one or more substituents selected from the group consisting of hydrogen, halogen, CN, OH, NH₂, -ONO₂, -COOH, -S(=O)₂OH, -N=C(NH₂)₂, C₁₋₆-alkyl, and -COO(C₁₋₆-alkyl).

18. The compound or the stereoisomer, geometric isomer, tautomer, hydrate, solvate, metabolite or pharmaceutically acceptable salt thereof according to any one of claims 1-17, wherein L₂ is selected from the group consisting of OH, CH₃,

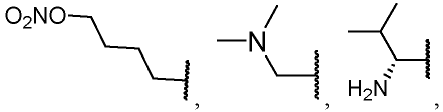

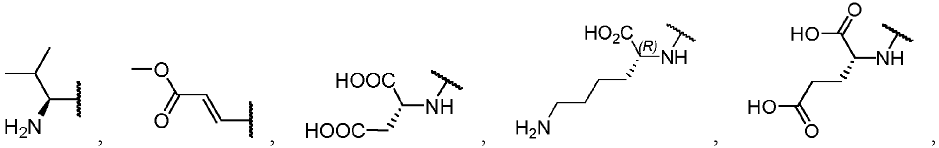

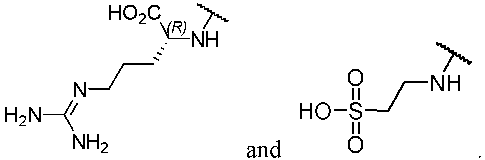

19. The compound or the stereoisomer, geometric isomer, tautomer, hydrate, solvate, metabolite or pharmaceutically acceptable salt thereof according to any one of claims 1-18, wherein L₂ is OH.

20. The compound or the stereoisomer, geometric isomer, tautomer, hydrate, solvate, metabolite or pharmaceutically acceptable salt thereof according to any one of claims 1-18, wherein L₂ is selected from the group consisting of CH₃, 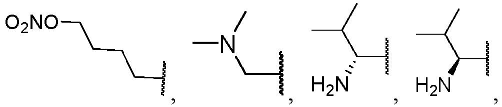 and

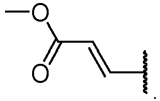

21. The compound or the stereoisomer, geometric isomer, tautomer, hydrate, solvate, metabolite or pharmaceutically acceptable salt thereof according to any one of claims 1-18, wherein L₂ is selected from the group consisting of

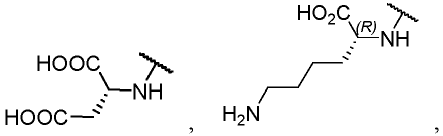

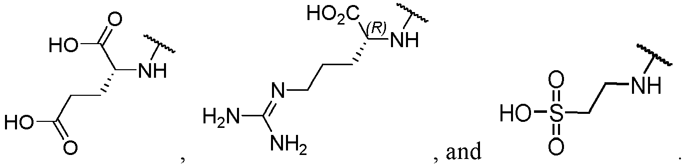

22. The compound or the stereoisomer, geometric isomer, tautomer, hydrate, solvate, metabolite or pharmaceutically acceptable salt thereof according to any one of claims 1-21, wherein R_{f} is selected from the group consisting of H and C₁₋₃-alkyl.

23. The compound or the stereoisomer, geometric isomer, tautomer, hydrate, solvate, metabolite or pharmaceutically acceptable salt thereof according to any one of claims 1-22, wherein ring A is absent or C₆₋₁₄-aryl; the C₆₋₁₄-aryl is optionally substituted by one or more R_{f}.

24. The compound or the stereoisomer, geometric isomer, tautomer, hydrate, solvate, metabolite or pharmaceutically acceptable salt thereof according to any one of claims 1-23, wherein R_{f} is selected from the group consisting of H and C₁₋₃-alkyl.

25. The compound or the stereoisomer, geometric isomer, tautomer, hydrate, solvate, metabolite or pharmaceutically acceptable salt thereof according to any one of claims 1-24, wherein ring A is absent or C₆₋₁₀-aryl.

26. The compound or the stereoisomer, geometric isomer, tautomer, hydrate, solvate, metabolite or pharmaceutically acceptable salt thereof according to any one of claims 1-25, wherein ring A is absent or phenyl.

27. The compound or the stereoisomer, geometric isomer, tautomer, hydrate, solvate, metabolite or pharmaceutically acceptable salt thereof according to any one of claims 1-26, wherein ring A is absent or is selected from the group consisting of

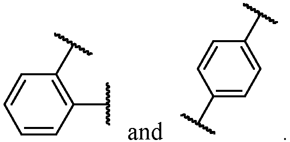

28. The compound or the stereoisomer, geometric isomer, tautomer, hydrate, solvate, metabolite or pharmaceutically acceptable salt thereof according to any one of claims 1-27, wherein the compound of formula I is a compound of formula III: 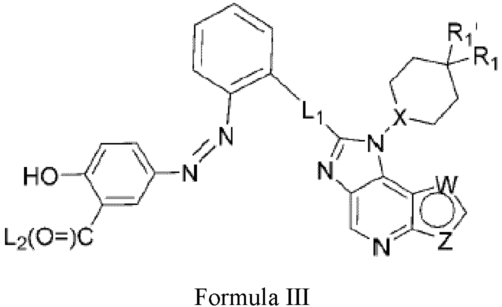 wherein the X, W, Z, R₁, R₁', L₁, and L₂ in the compound of formula III are as defined in any one of claims 1-27.

29. The compound or the stereoisomer, geometric isomer, tautomer, hydrate, solvate, metabolite or pharmaceutically acceptable salt thereof according to any one of claims 1-28, wherein the compound of formula I is a compound of formula IV: 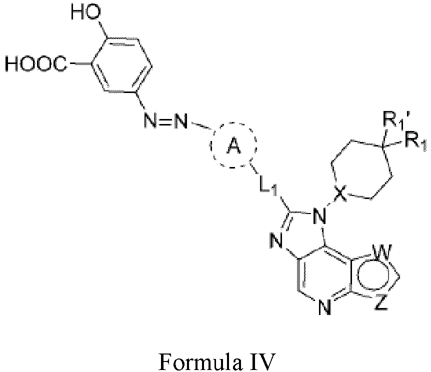 wherein the X, W, Z, R₁, R₁', L₁, and ring A in the compound of formula IV are as defined in any one of claims 1-28.

30. The compound or the stereoisomer, geometric isomer, tautomer, hydrate, solvate, metabolite or pharmaceutically acceptable salt thereof according to any one of claims 1-29, wherein the compound of formula I is a compound of formula V: 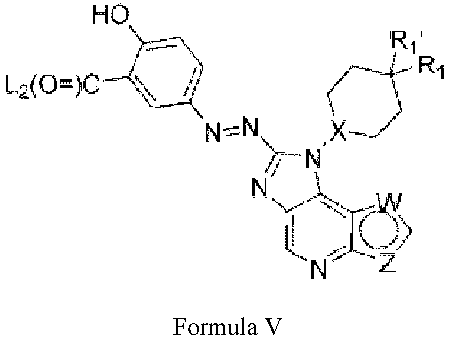 wherein the X, W, Z, R₁, R₁', and L₂ in the compound of formula V are as defined in any one of claims 1-29.

31. The compound or the stereoisomer, geometric isomer, tautomer, hydrate, solvate, metabolite or pharmaceutically acceptable salt thereof according to any one of claims 1-30, wherein the compound of formula I is a compound of formula VI: 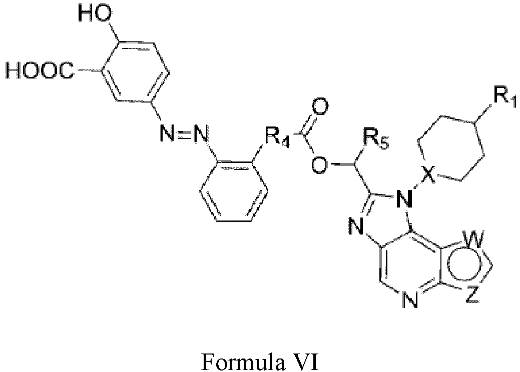
wherein R₄ is selected from the group consisting of C₁₋₃-alkylene and C₁₋₃-alkyleneoxy;
R₅ is selected from the group consisting of hydrogen and C₁₋₃ alkyl;
wherein R₄ and R₅ are each optionally substituted by one or more Rₑ, and Rₑ is as defined in any one of claims 1-30; and
the X, W, Z, and R₁ in the compound of formula VI are as defined in any one of claims 1-30.

32. The compound or the stereoisomer, geometric isomer, tautomer, hydrate, solvate, metabolite or pharmaceutically acceptable salt thereof according to any one of claims 1-27, wherein the compound of formula I is a compound of formula IX: 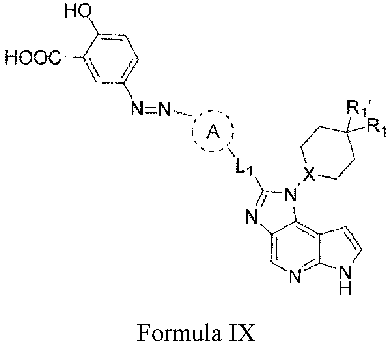 wherein the X, R₁, R₁', L₁, and ring A in the compound of formula IX are as defined in the formula I of any one of claims 1-27.

33. The compound or the stereoisomer, geometric isomer, tautomer, hydrate, solvate, metabolite or pharmaceutically acceptable salt thereof according to any one of claims 1-27, wherein the compound of formula I is a compound of formula X: 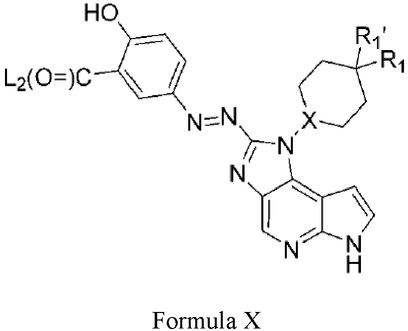 wherein the X, R₁, R₁', and L₂ in the compound of formula X are as defined in formula I of any one of claims 1-27.

34. The compound or the stereoisomer, geometric isomer, tautomer, hydrate, solvate, metabolite or pharmaceutically acceptable salt thereof according to any one of claims 1-27, wherein the compound of formula I is a compound of formula XI: 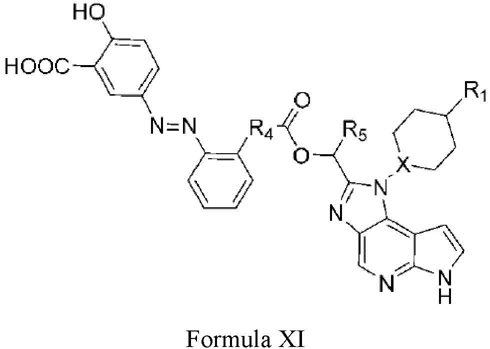
wherein R₄ is selected from the group consisting of C₁₋₃-alkylene and C₁₋₃-alkyleneoxy;
R₅ is selected from the group consisting of hydrogen and C₁₋₃-alkyl;
wherein R₄ and R₅ are each optionally substituted by one or more Rₑ, and Rₑ is defined in the aforementioned formula I; and
the X and R₁ in the compound of formula XI are as defined in formula I of any one of claims 1-27.

35. The compound or the stereoisomer, geometric isomer, tautomer, hydrate, solvate, metabolite or pharmaceutically acceptable salt thereof according to claim 1, wherein the compound is selected from the group consisting of:
(*R,E*)-5-((2-(3-(1-(1-(4-(cyanomethyl)piperidin-1-yl)-1,6-dihydroimidazo[4,5-*d*]pyrrolo[2,3-*b*] pyridin-2-yl)ethoxy)-3-oxopropyl)phenyl)diazenyl)-2-hydroxybenzoic acid;
(*R,E*)-5-((4-((((1-(1-(4-cyanomethylpiperidin-1-yl)-1,6-dihydroimidazo[4,5-*d*]pyrrolo[2,3-*b*]py ridin-2-yl)ethoxy)carbonyl)oxy)methyl)phenyl)diazenyl)-2-hydroxybenzoic acid;
(*R,E*)-5-((2-((1-(1-(1-(4-cyanomethylpiperidin-1-yl)-1,6-dihydroimidazo[4,5-*d*]pyrrolo[2,3-*b*]p yridin-2-yl)ethoxy)-2-methyl-1-oxopropan-2-yl)oxy)phenyl)diazenyl)-2-hydroxybenzoic acid;
(*R,E*)-5-((2-((1-(1-(4-(cyanomethyl)piperidin-1-yl)-1,6-dihydroimidazo[4,5-*d*]pyrrolo[2,3-*b*]py ridin-2-yl)ethoxy)carbonyl)amino)phenyl)diazenyl)-2-hydroxybenzoic acid;
(*R*)-1-(1-(4-(cyanomethyl)piperidin-1-yl)-1,6-dihydroimidazo[4,5-*d*]pyrrolo[2,3-*b*]pyridin-2-yl )ethyl dimethylglycinate;
(*R*)-1-(1-(4-(cyanomethyl)piperidin-1-yl)-1,6-dihydroimidazo[4,5-*d*]pyrrolo[2,3-*b*]pyridin-2-yl )ethyl D-valinate;
(*R*)-1-(1-(4-cyanomethylpiperidin-1-yl)-1,6-dihydroimidazo[4,5-*d*]pyrrolo[2,3-*b*]pyridin-2-yl)e thyl L-valinate;
5-((*E*)-(1-((1*R*,4*R*)-4-(cyanomethyl)cyclohexyl)-1,6-dihydroimidazo[4,5-*d*]pyrrolo[2,3-*b*]pyrid in-2-yl)diazenyl)-2-hydroxybenzoic acid;
(*E*)-5-((1-(4-cyanomethylpiperidin-1-yl)-1,6-dihydroimidazo[4,5-*d*]pyrrolo[2,3-*b*]pyridin-2-yl) diazenyl)-2-hydroxybenzoic acid;
(5-((*E*)-(1-((1*R*,4*R*)-4-(cyanomethyl)cyclohexyl)-1,6-dihydroimidazo[4,5-*d*]pyrrolo[2,3-*b*]pyri din-2-yl)diazenyl)-2-hydroxybenzoyl)-D-aspartic acid;
(5-((*E*)-(1-((1*R*,4*R*)-4-(cyanomethyl)cyclohexyl)-1,6-dihydroimidazo[4,5-*d*]pyrrolo[2,3-*b*]pyri din-2-yl)diazenyl)-2-hydroxybenzoyl)-D-lysine;
(5-((*E*)-(1-((1*R*,4*R*)-4-(cyanomethyl)cyclohexyl)-1,6-dihydroimidazo[4,5-*d*]pyrrolo[2,3-*b*]pyri din-2-yl)diazenyl)-2-hydroxybenzoyl)-D-glutamic acid;
(*R*)-2-(5-((*E*)-(1-((1*R*,4*R*)-4-(cyanomethyl)cyclohexyl)-1,6-dihydroimidazo[4,5-*d*]pyrrolo[2,3-*b*]pyridin-2-yl)diazenyl)-2-hydroxybenzamido)-5-((diaminomethylene)amino)valeric acid;
2-(5-((*E*)-(1-((1*R*,4*R*)-4-(cyanomethyl)cyclohexyl)-1,6-dihydroimidazo[4,5-*d*]pyrrolo[2,3-*b*]py ridin-2-yl)diazenyl)-2-hydroxybenzamido)ethan-1-sulfonic acid;
(*E*)-2-hydroxy-5-((1-(piperidin-1-yl)-1,6-dihydroimidazo[4,5-*d*]pyrrolo[2,3-*b*]pyridin-2-yl)dia zenyl)benzoic acid;
(*E*)-5-((1-(4-(((cyanomethyl)sulfonamido)methyl)piperidin-1-yl)-1,6-dihydroimidazo[4,5-*d*]py rrolo[2,3-*b*]pyridin-2-yl)diazenyl)-2-hydroxybenzoic acid;
5-((*E*)-(1-((1*R*,4*R*)-4-(cyanomethyl)cyclohexyl)-1H-imidazo[4,5-*d*]thieno[3,2-*b*]pyridin-2-yl)d iazenyl)-2-hydroxybenzoic acid; and
5-((*E*)-(1-((1*R*,4*R*)-4-(cyanomethyl)cyclohexyl)-1*H*-furo[3,2-*b*]imidazo[4,5-*d*]pyridin-2-yl)dia zenyl)-2-hydroxybenzoic acid.

36. A pharmaceutical composition comprising a therapeutically effective amount of the compound or the stereoisomer, geometric isomer, tautomer, hydrate, solvate, metabolite or pharmaceutically acceptable salt thereof according to any one of claims 1-35, and one or more pharmaceutically acceptable carriers, diluents, or excipients.

37. Use of the compound or the stereoisomer, geometric isomer, tautomer, hydrate, solvate, metabolite or pharmaceutically acceptable salt thereof according to any one of claims 1-35, or the pharmaceutical composition according to claim 36, in the manufacture of a medicament of a prodrug of a JAK inhibitor.

38. Use of the compound or the stereoisomer, geometric isomer, tautomer, hydrate, solvate, metabolite or pharmaceutically acceptable salt thereof according to any one of claims 1-35, or the pharmaceutical composition according to claim 36, in the manufacture of a medicament of a JAK inhibitor.

39. Use of the compound or the stereoisomer, geometric isomer, tautomer, hydrate, solvate, metabolite or pharmaceutically acceptable salt thereof according to any one of claims 1-35, or the pharmaceutical composition according to claim 36, in the manufacture of a medicament for the treatment of an inflammatory disease and/or a cancerous disease.

40. The use according to claim 39, wherein the inflammatory disease and/or cancerous disease are mediated by JAK.

41. The use according to claim 39 or 40, wherein the inflammatory disease is selected from the group consisting of rheumatoid arthritis, dermatitis, psoriasis, and inflammatory bowel disease; the cancerous disease is selected from the group consisting of myelofibrosis, polycythemia vera, essential thrombocythemia, myeloid leukemia, acute lymphocytic leukemia, ductal carcinoma of breast, and non-small cell lung carcinoma.

42. The use according to claim 41, wherein the inflammatory bowel disease is a chronic intestinal inflammatory disease, more preferably ulcerative colitis or Crohn's disease.
